(19) Europäisches Patentamt
European Patent Office
Office européen des brevets

(11) EP 2 428 572 A2

(12) EUROPEAN PATENT APPLICATION

(43) Date of publication:
**14.03.2012 Bulletin 2012/11**

(21) Application number: **11192225.8**

(22) Date of filing: **04.03.2008**

(51) Int Cl.:
***C12N 15/56*** (2006.01) ***C12N 9/28*** (2006.01)
***C12P 19/14*** (2006.01) ***A23L 1/305*** (2006.01)
***C11D 3/386*** (2006.01) ***D06M 16/00*** (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.03.2007 US 905811 P**

(62) Document number(s) of the earlier application(s) in accordance with Art. 76 EPC:
**08731341.7 / 2 126 089**

(71) Applicant: **Danisco US, Inc., Genencor Division**
**Palo Alto, CA 94304 (US)**

(72) Inventors:
- **Chang, Claudine**
**Palo Alto, CA 94304 (US)**
- **De Nobel, Hans**
**Palo Alto, CA 94304 (US)**
- **Jones, Brian E.**
**Palo Alto, CA 94304 (US)**
- **Naab, Corey**
**Palo Alto, CA 94304 (US)**
- **Kolkman, Marc**
**Palo Alto, CA 94304 (US)**
- **Vroemen, Casper**
**Palo Alto, CA 94304 (US)**
- **Weyler, Walter**
**Palo Alto, CA 94304 (US)**

(74) Representative: **Forrest, Graham Robert**
**Mewburn Ellis LLP**
**33 Gutter Lane**
**London**
**EC2V 8AS (GB)**

Remarks:
This application was filed on 06-12-2011 as a divisional application to the application mentioned under INID code 62.

# (54) Alkaliphilic Bacillus species alpha-amylase variants, compositions comprising alpha-amylase variants, and methods of use

(57) Disclosed are variants of the α-amylase derived from Bacillus sp. no. 707, compositions comprising said variants, compositions comprising the variants, and methods of using the variants. The methods of use include methods of cleaning surfaces, laundering textiles, desizing, hydrolyzing biofilms of various substrates, and treating starch (e.g., liquefaction and saccharification).

EP 2 428 572 A2

SEQUENCE LISTING

<110> Danisco US Inc., Genencor Division

<120> Alkaphilic Bacillus Species Alpha-Amylase Variants, Compositions Comprising Alpha-Amylase Variants, and Methods of Use

<130> GRF/FP6733299

<140> Divisional of EPA08731341.7
<141> 2008-03-04

<150> PCT/US2008/055780
<151> 2008-03-04

<150> US 60/905,811
<151> 2007-03-09

<160> 3

<170> PatentIn version 3.5

<210> 1
<211> 87
<212> DNA
<213> Bacillus licheniformis

<400> 1
```
atgaaacaac aaaaacggct ttacgcccga ttgctgacgc tgttatttgc gctcatcttc      60
ttgctgcctc attctgcagc ttcagca                                          87
```

<210> 2
<211> 1455
<212> DNA
<213> Bacillus sp. 707

<400> 2
```
catcataacg gtacgaacgg gacaatgatg caatactttg aatggtatct acctaatgac      60
ggaaatcatt ggaatcgatt aaactctgat gcgagtaacc ttaaaagcaa agggattaca     120
gcggtgtgga ttcctccagc atggaagggc gcttctcaaa atgacgtagg atacggagcc     180
tatgacctgt atgatctggg agaatttaat caaaaaggta ccgtccgtac aaaatatgga     240
acacgtagtc agttacaagc tgcggtaacc tccttaaaaa ataatggaat tcaagtatat     300
ggtgacgttg ttatgaatca caaaggtggc gcagacgcta ctgaaatggt aagggccgtt     360
gaagtgaatc ccaataaccg taaccaagaa gtgactggtg aatataccat tgaagcttgg     420
actagatttg attttccagg gcgaggaaat actcattcta gctttaaatg gagatggtat     480
cattttgatg gtgtggattg ggatcagtca cgtagactga acaatcgcat ctataaattt     540
agaggtcatg gcaaagcttg ggattgggaa gttgatacgg aaaatggtaa ttatgattat     600
ttaatgtacg ctgatattga tatggatcac ccagaagtag taaatgaatt aagaaattgg     660
ggtgtttggt acacaaacac attaggactc gatggattta gaatagatgc ggttaaacat     720
ataaagtata gctttacgcg cgattggatt aatcacgtta gaagtgcaac aggtaaaaat     780
atgtttgcgg ttgctgagtt ttggaagaat gatttaggtg caattgaaaa ctatctgcag     840
aaaacaaact ggaaccattc agtctttgat gtgccgttac attataatct ttataatgca     900
tcaaaaagcg gagggaacta tgatatgcga aacatattta atggaacggt tgttcaacga     960
catccaagtc atgctgtaac atttgttgat aatcatgatt cgcagcctga agaagcatta    1020
gaatcttttg ttgaagaatg gtttaaacca ttagcgtatg cgcttacatt aacgcgtgaa    1080
caaggatacc cttctgtatt ttacggagat tattatggga ttccaacaca tggagtgcca    1140
gcaatgagat caaaaatcga tccgatttta gaagcacgtc aaaagtatgc atacggaaaa    1200
caaaatgatt acttagacca tcataatatc attggttgga cgcgtgaagg gaatacagca    1260
caccccaatt caggtctagc taccatcatg tctgatggag cgggtggaag taagtggatg    1320
tttgttgggc gtaataaggc tggtcaagta tggagtgata ttacaggaaa ccgtacaggt    1380
acggttacaa tcaatgcaga cggttggggc aatttctctg tgaatggagg gtcagtttct    1440
atttgggtca acaaa                                                     1455
```

<210> 3

<211> 485
<212> PRT
<213> Bacillus sp. 707

<400> 3

```
His His Asn Gly Thr Asn Gly Thr Met Met Gln Tyr Phe Glu Trp Tyr
1               5                   10                  15
Leu Pro Asn Asp Gly Asn His Trp Asn Arg Leu Asn Ser Asp Ala Ser
            20                  25                  30
Asn Leu Lys Ser Lys Gly Ile Thr Ala Val Trp Ile Pro Pro Ala Trp
        35                  40                  45
Lys Gly Ala Ser Gln Asn Asp Val Gly Tyr Gly Ala Tyr Asp Leu Tyr
    50                  55                  60
Asp Leu Gly Glu Phe Asn Gln Lys Gly Thr Val Arg Thr Lys Tyr Gly
65                  70                  75                  80
Thr Arg Ser Gln Leu Gln Ala Ala Val Thr Ser Leu Lys Asn Asn Gly
                85                  90                  95
Ile Gln Val Tyr Gly Asp Val Val Met Asn His Lys Gly Gly Ala Asp
            100                 105                 110
Ala Thr Glu Met Val Arg Ala Val Glu Val Asn Pro Asn Asn Arg Asn
        115                 120                 125
Gln Glu Val Thr Gly Glu Tyr Thr Ile Glu Ala Trp Thr Arg Phe Asp
    130                 135                 140
Phe Pro Gly Arg Gly Asn Thr His Ser Ser Phe Lys Trp Arg Trp Tyr
145                 150                 155                 160
His Phe Asp Gly Val Asp Trp Asp Gln Ser Arg Arg Leu Asn Asn Arg
                165                 170                 175
Ile Tyr Lys Phe Arg Gly His Gly Lys Ala Trp Asp Trp Glu Val Asp
            180                 185                 190
Thr Glu Asn Gly Asn Tyr Asp Tyr Leu Met Tyr Ala Asp Ile Asp Met
        195                 200                 205
Asp His Pro Glu Val Val Asn Glu Leu Arg Asn Trp Gly Val Trp Tyr
    210                 215                 220
Thr Asn Thr Leu Gly Leu Asp Gly Phe Arg Ile Asp Ala Val Lys His
225                 230                 235                 240
Ile Lys Tyr Ser Phe Thr Arg Asp Trp Ile Asn His Val Arg Ser Ala
                245                 250                 255
Thr Gly Lys Asn Met Phe Ala Val Ala Glu Phe Trp Lys Asn Asp Leu
            260                 265                 270
Gly Ala Ile Glu Asn Tyr Leu Gln Lys Thr Asn Trp Asn His Ser Val
        275                 280                 285
Phe Asp Val Pro Leu His Tyr Asn Leu Tyr Asn Ala Ser Lys Ser Gly
    290                 295                 300
Gly Asn Tyr Asp Met Arg Asn Ile Phe Asn Gly Thr Val Val Gln Arg
305                 310                 315                 320
His Pro Ser His Ala Val Thr Phe Val Asp Asn His Asp Ser Gln Pro
                325                 330                 335
Glu Glu Ala Leu Glu Ser Phe Val Glu Glu Trp Phe Lys Pro Leu Ala
            340                 345                 350
Tyr Ala Leu Thr Leu Thr Arg Glu Gln Gly Tyr Pro Ser Val Phe Tyr
        355                 360                 365
Gly Asp Tyr Tyr Gly Ile Pro Thr His Gly Val Pro Ala Met Arg Ser
    370                 375                 380
Lys Ile Asp Pro Ile Leu Glu Ala Arg Gln Lys Tyr Ala Tyr Gly Lys
385                 390                 395                 400
Gln Asn Asp Tyr Leu Asp His His Asn Ile Ile Gly Trp Thr Arg Glu
                405                 410                 415
Gly Asn Thr Ala His Pro Asn Ser Gly Leu Ala Thr Ile Met Ser Asp
            420                 425                 430
Gly Ala Gly Gly Ser Lys Trp Met Phe Val Gly Arg Asn Lys Ala Gly
        435                 440                 445
Gln Val Trp Ser Asp Ile Thr Gly Asn Arg Thr Gly Thr Val Thr Ile
    450                 455                 460
Asn Ala Asp Gly Trp Gly Asn Phe Ser Val Asn Gly Gly Ser Val Ser
465                 470                 475                 480
```

```
Ile Trp Val Asn Lys
                485
```

## Description

### RELATED APPLICATIONS

The present application claims priority to U.S. Provisional Patent Application Serial No. 60/905,811, entitled "Alkaliphilic Bacillus Species α-Amylase Variants, Compositions Comprising α-Amylase Variants, And Methods of Use", filed March 9, 2007.

### SEQUENCE LISTING

Attached is a sequence listing comprising SEQ ID NOS: 1-3, which are herein incorporated by reference in their entirety for all purposes.

### FIELD OF THE INVENTION

Disclosed are a *Bacillus* sp. No. 707 α-amylase and variants thereof as well as compositions and uses for same.

### BACKGROUND

Starch consists of a mixture of amylose (15-30% w/w) and amylopectin (70-85% w/w). Amylose consists of linear chains of α-1,4-linked glucose units having a molecular weight (MW) from about 60,000 to about 800,000. Amylopectin is a branched polymer containing α-1,6 branch points every 24-30 glucose units; its MW may be as high as 100 million.

Sugars from starch, in the form of concentrated dextrose syrups, are currently produced by an enzyme catalyzed process involving: (1) liquefaction (or thinning) of solid starch with an α-amylase into dextrins having an average degree of polymerization of about 7-10; and (2) saccharification of the resulting liquefied starch (i.e. starch hydrolysate) with amyloglucosidase (also called glucoamylase or GA). The resulting syrup has a high glucose content. Much of the glucose syrup, which is commercially produced, is subsequently enzymatically isomerized to a dextrose/fructose mixture known as isosyrup.

α-amylases (EC 3.2.1.1) hydrolyze starch, glycogen, and related polysaccharides by cleaving internal α-1,4-glucosidic bonds at random. This enzyme class has a number of important commercial applications in, for example, starch liquefaction, textile desizing, starch modification in the paper and pulp industry, and for brewing. These enzymes can also be used to remove starchy stains during dishwashing and laundry washing and in the sugar, brewing, alcohol and textile industries. α-amylases are isolated from a wide variety of bacterial, fungal, plant and animal sources. Industrially, many important α-amylases are those isolated from Bacilli.

One characterized α-amylase is that of an alkaliphilic *Bacillus* sp. no. 707. *Bacillus* sp. no. 707 produces mainly five kinds of enzymes exhibiting starch hydrolyzing activity. Their molecular weights are estimated to be approximately 110, 95, 85, 75, and 60 kDa. A. Tsukamoto et al., "Nucleotide sequence of the maltohexaose-producing amylase gene from an alkalophilic Bacillus sp. #707 and structural similarity to liquefying type α-amylase," Biochem. & Biophys. Res. Comm. 151(1): 25-31 (1988). The α-amylase identified is 518 amino acids in length with an estimated molecular weight of 59,007.5 Daltons. Tsukamoto et al. (1988). The first 33 amino acids act as the signal peptide that is involved in the secretion of the exported protein. The extracellular form would therefore have 485 amino acids with an estimated molecular weight of 55,372 Daltons. The nucleic acid encoding the enzyme was cloned and characterized as discussed in K. Kimura et al., "Cloning of a gene for maltohexaose producing amylase of an alkalophilic Bacillus and hyper-production of the enzyme in Bacillus subtilis cells," Appl. Microbiol. Biotechnol. 27: 372-377 (1988). The α-amylase is a G6 amylase or maltohexaose-producing amylase (E.C. 3.2.1.98) and belongs to the glycoside hydrolase family 13. The amino acid sequence of the α-amylase from *Bacillus* sp. no. 707 is 65.5%, 65.9% and 66.3% identical to those of liquefying α-amylases from *Bacillus amyloliquefaciens* (BAA), *Bacillus licheniformis* (BLA), and *Bacillus stearothermophilus,* respectively. R. Kanai et al., "Biochemical and crystallographic analyses of maltohexaose-producing amylase from alkalophilic Bacillus sp. 707," Biochemistry 43: 14047-14056 (2004). Therefore, the G6-amylase of *Bacillus* sp. no. 707 is presumed to structurally differ from that of G4-amylases. R. Kanai et al. (2004).

Thus, there is a need for α-amylase variants of *Bacillus* sp. no. 707, wherein there are enhanced characteristics and/or rates of production. Increased production will lead to, among other things, reduced costs, improved cost margins, plant capacity savings, and higher activity products. Additionally, increased specific activity can likewise improve cost margins by requiring for example, less enzyme.

## SUMMARY

Thus, provided herein a compositions and methods of using said compositions using an α-amylase with improved characteristics for use in said compositions and methods.

For example, one aspect contemplates a manual or automatic dishwashing composition comprising a *Bacillus* sp. no. 707 α-amylase or variant thereof, and one or more of a surfactant, detergent builder, a complexing agent, a polymer, a bleaching system, a stabilizer, a foam booster, a suds suppressor, an anti-corrosion agent, a soil-suspending agent, an anti-soil redeposition agent, a dye, a bactericide, a hydrotope, a tarnish inhibitor, and a perfume. The dishwashing compositions can be a composition used for manual or automatic dishwashing.

Another aspect contemplates a laundry detergent additive comprising a *Bacillus* sp. no. 707 α-amylase or variant thereof.

Another aspect contemplates a laundry detergent comprising the above listed detergent additive, and further comprising one or more of the following and one or more of a surfactant, detergent builder, a complexing agent, a polymer, a bleaching system, a stabilizer, a foam booster, a suds suppressor, an anti-corrosion agent, a soil-suspending agent, an anti-soil redeposition agent, a dye, a bactericide, a hydrotope, an optical brightener, a fabric conditioner, and a perfume.

Yet another aspect contemplates an isolated nucleic acid encoding a *Bacillus* sp. no. 707 α-amylase or a variant thereof, wherein the variant has a residue substitution or deletion selected from the group consisting of substitutions of M202, M208, S255, R172, and/or M261 of SEQ ID NO: 3. The M202 variant can be a substitution variant selected from the group consisting of: M202L, M202V, M202S, M202T, M202I, M202Q, and M202W. The S255 variant can be the substitution of S255N. The R172 substitution can be R172Q. Also contemplated are variants with combinations of these substitutions. Also contemplated are α-amylase polypeptides (with and without the signal sequence) with these substitutions in the polypeptide sequence.

Another aspect contemplates a vector comprising a nucleic acid encoding any of the aforementioned variants. Also contemplated are isolated cells wherein the nucleic acid, is inserted, for example via a vector. The isolated host cell can be a microorganism for example such as a bacterium or fungus. The bacterium can be a Gram positive bacterium selected from the group consisting of *Bacillus subtilis, B. licheniformis, B. lentus, B. brevis, B. stearothermophilus, B. alkalophilus, B. amyloliquefaciens, B. coagulans, B. circulans, B. lautus, B. thuringiensis, Streptomyces lividans* or *S. murinus;* or a Gram negative bacterium, wherein said Gram negative bacterium is *Escherichia coli* or a *Pseudomonas* species.

Another aspect contemplated is the use of the polypeptides described here for laundry washing and/or dishwashing. These polypeptide variants can be optionally in the form of a non-dusting granulate, microgranulate, stabilized liquid, or protected enzyme. Another aspect contemplates that the detergent additive or detergent composition further comprise an enzyme selected from the group consisting of: a cellulase, a protease, an acyltransferase, an aminopeptidase, an amylase, a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination of the enzymes. Other amylases contemplated for use in the composition include two or more other α-amylases, a β-amylase, an isoamylase, or a glucoamylase.

Also contemplated are methods of cleaning textiles and dishes or other hard surfaces using any of the abovementioned compositions.

Another aspect contemplates the use of the α-amylase described herein or any of the α-amylase variants in a textile desizing composition, wherein the composition is an aqueous solution. Also contemplated are methods of desizing textiles using said compositions.

A further embodiment contemplates a composition for starch processing comprising a *Bacillus* sp. no. 707 α-amylase or variant thereof in an aqueous solution. Also contemplated is a method of using such a composition to process starch. The method and composition may further comprise a glucoamylase, an isoamylase, a pullulanase, phytase or a combination thereof. Yet another aspect contemplates a biofilm hydrolyzing composition comprising a *Bacillus* sp. no. 707 α-amylase or variant thereof in a solution or gel, and optionally further comprising a cellulase, a hemicellulase, a xylanase, a lipase, a protease, a pectinase, an antimicrobial agent, or any combination thereof. Also contemplated are methods of hydrolyzing biofilms using said compositions.

Another aspect contemplated is a composition for saccharifying starch comprising a *Bacillus* sp. no. 707 α-amylase or variant thereof in a solution. Therefore, also contemplated is a method of saccharifying starch comprising administering the composition of claim 33 for a period sufficient to saccharify said starch.

Another embodiment contemplated is a composition for liquefying starch comprising a *Bacillus* sp. no. 707 α-amylase or variant thereof in a solution. Also contemplated is a method of liquefying a starch comprising administering the composition of using said composition for a period sufficient to liquefy said starch.

In yet a further aspect, a baking composition is contemplated using a *Bacillus* sp. no. 707 α-amylase or variant thereof in a solution or in a gel. Also contemplated is a method of baking using such a baking composition.

## BRIEF DESCRIPTION OF THE DRAWING

The accompanying drawings are incorporated in and constitute a part of this specification, illustrate embodiments. The figures are illustrative of the embodiments disclosed.

FIG. 1. Rice Swatch Assay at pH 8.0 comparing Stainzyme® (▲), OxAm (■) (Purastar®), and the *Bacillus* sp. no. 707 α-amylase (□).

FIG. 2. Rice swatch assay at pH 10.1 comparing Stainzyme® (pink ■), OxAm (▲) (Purastar®), and the *Bacillus* sp. no. 707 α-amylase (orange ■).

FIG. 3. FIG. 3A depicts the Pre-LAT signal peptide-encoding DNA sequence (SEQ ID NO: 1). FIG. 3B depicts the native *Bacillus* sp. no. 707 α-amylase gene (SEQ ID NO: 2).

FIG. 4. Diagram of pICatH plasmid.

FIG. 5. Diagram of the pICatH plasmid containing the Amy707 gene sequence.

FIG. 6. Depicts the results of a Terg-o-tometer assay. Detergent compositions comprising Tide Active (1.8 g/L), Tide Inactivated (1.8 g/L), or AATCC (1.5 g/L) were tested in the presence of *Bacillus* sp. no. 707 α-amylase (0.1 ppm) or Stainzyme® (0.1 ppm). In some samples, Purafect® Prime (0.5 ppm) was also present. The assay was conducted in a 5 mM HEPES buffer, with 6 gpg (grains per gallon) water hardness, at 74°F at a pH of 7.5. The swatches tested were colored starch cotton (EMPA161, 13-02). Percentage SRI (%SRI) stands for percentage stain removal index or the percentage of stain removed from a cleaning process. Stain removal was calculated by measuring an unsoiled swatch, a fully soiled swatch, and the soiled swatch after some cleaning treatment has been administered. Measurements are made by reflectometry using the CIE L*a*b* color space. The %SRI is calculated by a ratio of the difference between the color of the cleaned and soiled swatches, and the difference between the color of the unsoiled and soiled swatches. "dE" stands for the square root of the sum of the squares of the differences of each color component in the CIE L*a*b* color space. Every perceivable color can be represented by L*a*b* coordinate in the color space. "L*" represents the lightness or grey scale value on a scale of 0 to 100, pure black to pure white. "a* represents the magenta to greet shift, wherein large positive values represent a very magenta hue and large negative values represent a very green hue. "b*" represents the yellow to blue shift where large positive values represent a very yellow hue and large positive values represent a very blue hue. When both a* and b* values are 0, there is an absence of color, leaving pure grey colors with their lightness defined by the L* value. The swatches tested were either CS-2 (072, with cocoa stains), CS-37 (005 with full egg stains), or colored starch cotton (EMPA161, 13-02).

FIG. 7. Lauder-o-meter assay results conducted with 5 g/L IEC A* with bleach, at 12 gpg water hardness at 40°F. *Bacillus* sp. no. 707 α-amylase and Stainzyme® were present in the amount of 0.1 ppm. Compositions were tested on 5 different stained swatches.

FIG. 8. Depicts automatic dish washing performance under European conditions of *Bacillus* sp. no. 707 α-amylase, Stainzyme®, and OxAm (Purastar®). Wash performance of removing rice milk is measured in soil removal capacity as compared to mg active protein.

FIG. 9. Depicts automatic dish washing performance under European conditions of *Bacillus* sp. no. 707 α-amylase, Stainzyme®, and OxAm (Purastar®). Wash performance of removing mixed starch is measured in soil removal capacity as compared to mg active protein.

FIG. 10. An assay was prepared on the M202→L (M202L) variant of *Bacillus* sp. no. 707 α-amylase in the presence or absence of bleach at 20°C. Absorbance of the cleaning assay was measured at 488 nm with increasing amounts of enzyme (measured in parts per million, ppm). The M202L variant was compared to the wild-type α-amylase of *Bacillus* sp. no. 707.

## DETAILED DESCRIPTION

The following relates to compounds, compositions, methods of making said compounds, and methods of using said compounds and compositions, wherein the compounds are a *Bacillus* sp. no. 707 α-amylase or variants thereof. The *Bacillus* sp. no 707 α-amylases form as well as variants thereof were sought that have a high performance in for example laundry and dishwashing tests.

The α-amylase of *Bacillus* sp. no. 707 has a pH optimum of 8.8 and is stable over a broad pH range (*i.e.,* pH 4.7 to 10.8). The polypeptide had a temperature optimum of 45°C. The enzyme has activity at lower temperatures, e.g., 15-20°C. After incubation for 30 minutes at temperatures over 55°C, the enzyme retains less than 20% of its activity.

## 1. Abbreviations and Definitions

In accordance with this detailed description, the following abbreviations and definitions apply. It must be noted that as used herein, the singular forms "a", "an", and "the" include plural referents unless the context clearly dictates otherwise. Thus, for example, reference to "an enzyme" includes a plurality of such enzymes and reference to "the formulation" includes reference to one or more formulations and equivalents thereof known to those skilled in the art, and so forth.

Unless defined otherwise, all technical and scientific terms used herein have the same meaning as commonly understood by one of ordinary skill in the art. The following terms are provided below.

### 1.1 Definitions

"Amylase" is meant to include any amylase such as glucoamylases, α-amylases, β-amylases and wild-type α-amylases of bacteria such as *Bacillus* sp., such as *B. licheniformis* and *B. subtilis.* "Amylase" shall mean an enzyme that is, among other things, capable of catalyzing the degradation of starch. Amylases are hydrolases that cleave the α-D-(1→4) O-glycosidic linkages in starch. Generally, α-amylases (EC 3.2.1.1; α-D-(1→4)-glucan glucanohydrolase) are defined as endo-acting enzymes cleaving α-D-(1→4) O-glycosidic linkages within the starch molecule in a random fashion. In contrast, the exo-acting amylolytic enzymes, such as β-amylases (EC 3.2.1.2; α-D-(1→4)-glucan maltohydrolase) and some product-specific amylases like maltogenic α-amylase (EC 3.2.1.133) cleave the starch molecule from the non-reducing end of the substrate. β-Amylases, α-glucosidases (EC 3.2.1.20; α-D-glucoside glucohydrolase), glucoamylases (EC 3.2.1.3; α-D-(1→4)-glucan glucohydrolase), and product-specific amylases can produce malto-oligosaccharides of a specific length from starch.

*"Bacillus* sp. no. 707 α-amylase" is an α-amylase derived from *Bacillus* sp. no. 707. The gene encoding the α-amylase can be the wild-type gene or a codon optimized polynucleotide that encodes the α-amylase. By *"Bacillus* sp. no. 707 α-amylase variants" is meant a variant of the wild-type *Bacillus* sp. no. 707 α-amylase, which includes a sequence substitution, addition or deletion from the parent polypeptide sequence of *Bacillus* sp. no. 707. The mature α-amylase of *Bacillus* sp. no. 707 is (amino to carboxy orientation) (SEQ ID NO: 3):

```
hhngtngtmm qyfewylpnd gnhwnrlnsd asnlkskgit avwippawkg    50
asqndvgyga ydlydlgefn qkgtvrtkyg trsqlqaavt slknngiqvy   100
gdvvmnhkgg adatemvrav evnpnnrnqe vtgeytieaw trfdfpgrgn   150
thssfkwrwy hfdgvdwdqs rrlnnriykf rghgkawdwe vdtengnydy   200
lmyadidmdh pevvnelrnw gvwytntlgl dgfridavkh ikysftrdwi   250
nhvrsatgkn mfavaefwkn dlgaienylq ktnwnhsvfd vplhynlyna   300
sksggnydmr nifngtvvqr hpshavtfvd nhdsqpeeal esfveewfkp   350
layaltltre qgypsvfygd yygipthgvp amrskidpil earqkyaygk   400
qndyldhhni igwtregnta hpnsglatim sdgaggskwm fvgrnkagqv   450
wsditgnrtg tvtinadgwg nfsvnggsvs iwvnk                   485
```

As used herein, "parent enzyme" and "parent polypeptide" shall mean the polypeptide of *Bacillus* sp. no. 707. By "parent nucleic acid" is meant a nucleic acid sequence encoding said parent polypeptide. The *Bacillus* sp. no. 707 α-amylase can further include mutations in the signal sequence of the parent polypeptide, or elsewhere in the α-amylase parent polypeptide. Thus, the *Bacillus* sp. no. 707 α-amylase can be in the form of a fusion protein containing a heterologous α-amylase polypeptide. It can also include chimeras (i.e., the combination of at least two α-amylases). For example, the *Bacillus* sp. no. 707 α-amylase can comprise the signal peptide from another α-amylase, such as *B. licheniformis* (LAT). *See e.g.,* FIG. 3A, which depicts the coding sequence for the LAT signal peptide. Also contemplated are more than one amino acid substituted for the first alanine (any substitution other than valine), such as for example one, two or more threonines. The term "variant" is used interchangeably with the term "mutant". Variants shall include polypeptides as well as the nucleic acids that encode additional substitutions, tranversions, insertions, and deletions to the *Bacillus* sp. no. 707 α-amylase. Variants can include sequences that are complementary to sequences that are capable of hybridizing to the nucleotide sequences presented herein. For example, a variant nucleic acid sequence is complementary to sequences capable of hybridizing under stringent conditions (e.g., 50°C and 0.2X SSC {1X SSC = 0.15 M NaCl, 0.015 M $Na_3$ citrate, pH 7.0}) to the nucleotide sequences presented herein. The term variant nucleic acid sequence encompasses sequences that are complementary to sequences that are capable of hybridizing under high stringent conditions (e.g., 65°C and 0.1X SSC {1X SSC = 0.15 M NaCl, 0.015 M $Na_3$ citrate, pH 7.0}) to the nucleotide sequences presented herein.

The terms "recovered", "isolated", and "separated" as used herein refer to a compound, protein, cell, nucleic acid or amino acid that is removed from at least one component with which it is naturally associated and found in nature.

By "purified" is meant that the material is in a relatively pure state, *e.g.,* at least about 90% pure, or at least about 95% pure, or at least about 98% pure.

By "thermostable" is meant the ability of the enzyme to retain activity after exposure to elevated temperatures. The thermostability of an enzyme, such as an α-amylase enzymes, is measured by its half-life. The half-life ($t_{1/2}$) is the time in minutes, hours, or days, during which half the enzyme activity is lost under defined conditions. The half-life value is calculated by measuring the residual α-amylase activity.

By "pH range" is meant the ability of the enzyme to exhibit catalytic activity from acidic to basic conditions spanning 5 or more pH units.

As used herein, "pH stable" relates to the ability of the enzyme to retain activity over a wide range of pHs for a predetermined period of time (*e.g.*, 15 min., 30 min., 1 hour).

As used herein, "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme". The conventional one-letter or three-letter code for amino acid residues are used herein.

The term "nucleic acid" encompasses DNA, RNA, single stranded or double stranded and chemical modifications thereof. The terms "nucleic acid" and "polynucleotide" may be used interchangeably herein.

As used herein, "nucleotide sequence" or "nucleic acid sequence" refers to an oligonucleotide sequence or polynucleotide sequence encoding a *Bacillus* sp. no. 707 α-amylase polypeptide or variant thereof, and fragments and derivatives thereof (such as portions thereof). The nucleotide sequence may be of genomic, synthetic, or recombinant origin, and may be double-stranded or single-stranded whether representing the sense or anti-sense strand. As used herein, the term nucleotide sequence includes genomic DNA, cDNA, synthetic DNA, and RNA. For example, the DNA can be a cDNA sequence coding for a *Bacillus* sp. no. 707 α-amylase or variant thereof. Because the genetic code is degenerate, more than one codon may be used to encode a particular amino acid, and the present invention encompasses nucleotide sequences which encode a particular amino acid sequence.

By "homologue" shall mean an entity having a certain degree of identity with the subject amino acid sequences and the subject nucleotide sequences. A homologous sequence is taken to include an amino acid sequence at least 75%, 80%, 85% or 90% identical, or at least 95%, 96%, 97%, 98% or 99% identical to the subject sequence. Typically, homologues will comprise the same active sites as the subject amino acid sequence.

As used herein, "hybridization" shall include the process by which a strand of nucleic acid joins with a complementary strand through base pairing, as well as the process of amplification as carried out in polymerase chain reaction (PCR) technologies. The α-amylase or variant thereof nucleic acid may exist as single- or double-stranded DNA or RNA, an RNA/DNA heteroduplex or an RNA/DNA copolymer.

As used herein, "synthetic" shall refer to that which is produced by *in vitro* chemical or enzymatic synthesis. It includes, but is not limited to, nucleic acids encoding *Bacillus* sp. no. 707 α-amylase or variants thereof made with optimal codon usage for host organisms, such as the methylotrophic yeasts *Pichia, Hansenula, Streptomyces, Trichoderma* (*e.g., T. reesei*) or other expression hosts.

By "water hardness" is meant the amount of calcium and magnesium in the water for washing purposes (1.4-1.8 mmol/L) and an amount for purposes of dishwashing.

As used herein, the terms "transformed", "stably transformed" and "transgenic" used in reference to a cell means the cell has a non-native (e.g., heterologous) nucleic acid sequence integrated into its genome or as an episomal plasmid that is maintained through multiple generations.

The term "introduced" in the context of inserting a nucleic acid sequence into a cell, means "transfection", or "transformation" or "transduction" and includes reference to the incorporation of a nucleic acid sequence into a eukaryotic or prokaryotic cell wherein the nucleic acid sequence may be incorporated into the genome of the cell (e.g., chromosome, plasmid, plastid, or mitochondrial DNA), converted into an autonomous replicon, or transiently expressed (e.g., transfected mRNA).

As used herein, "transformed cell" shall include cells that have been genetically altered by use of recombinant DNA techniques. Transformation typically occurs by insertion of one or more nucleotide sequences into a cell. The inserted nucleotide sequence may be a heterologous nucleotide sequence (*i.e.* is a sequence that is not natural to the cell that is to be transformed, such as a DNA sequence encoding a fusion protein or a non-native sequence).

As used herein, "operably linked" shall mean that the components described are in a relationship permitting them to function in their intended manner. A regulatory sequence operably linked to a coding sequence is ligated in such a way that expression of the coding sequence is achieved under condition compatible with the control sequences.

As used herein, "biologically active" shall refer to a sequence having a similar structural function (but not necessarily to the same degree), and/or similar regulatory function (but not necessarily to the same degree) and/or similar biochemical function (but not necessarily to the same degree) of the naturally occurring sequence.

"Host strain" or "host cell" means a suitable host for an expression vector or DNA construct comprising a polynucleotide encoding a variant alpha-amylase enzyme according to the present disclosure. Specifically, host strains are preferably bacterial cells. In a preferred embodiment of the invention, "host cell" means both the cells and protoplasts created from the cells of a microbial strain and particularly a *Bacillus* sp.

The term "selective marker" refers to a gene capable of expression in a host that allows for ease of selection of those hosts containing an introduced nucleic acid or vector. Examples of selectable markers include but are not limited to antimicrobials (e.g., hygromycin, bleomycin, or chloramphenicol) and/or genes that confer a metabolic advantage, such as a nutritional advantage on the host cell.

The term "culturing" refers to growing a population of microbial cells under suitable conditions in a liquid or solid medium. In one embodiment, culturing refers to fermentative bioconversion of a starch substrate containing granular starch to an end-product (typically in a vessel or reactor). Fermentation is the enzymatic and anaerobic breakdown of organic substances by microorganisms to produce simpler organic compounds. While fermentation occurs under anaerobic conditions it is not intended that the term be solely limited to strict anaerobic conditions, as fermentation also occurs in the presence of oxygen.

A "gene" refers to a DNA segment that is involved in producing a polypeptide and includes regions preceding and following the coding regions as well as intervening sequences (introns) between individual coding segments (exons).

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" as used herein means a DNA construct comprising a DNA sequence which is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

A "promoter" is a regulatory sequence that is involved in binding RNA polymerase to initiate transcription of a gene. The promoter may be an inducible promoter or a constitutive promoter. A preferred promoter used in the invention is *Bacillus licheniformis* alpha-amylase (AmyL).

The term "operably linked" refers to juxtaposition wherein the elements are in an arrangement allowing them to be functionally related. Thus, as used herein, "operably linked" means that the components described are in a relationship permitting them to function in their intended manner. For example, a regulatory sequence operably linked to a coding sequence is ligated in such a way that expression of the coding sequence is achieved under conditions compatible with the control sequences.

"Under transcriptional control" is a term well understood in the art that indicates that transcription of a polynucleotide sequence, usually a DNA sequence, depends on its being operably linked to an element which contributes to the initiation of, or promotes transcription.

"Under translational control" is a term well understood in the art that indicates a regulatory process that occurs after mRNA has been formed.

A "signal sequence" means a sequence of amino acids bound to the N-terminal portion of a protein, which facilitates the secretion of the mature form of the protein outside the cell. The definition of a signal sequence is a functional one. The mature form of the extracellular protein lacks the signal sequence which is cleaved off during the secretion process.

As used herein when describing proteins and genes that encode them, the term for the gene is italicized, (e.g., the gene that encodes amyL (*B. licheniformis* AA) may be denoted as *amyL*). The term for the protein is generally not italicized and the first letter is generally capitalized, (e.g., the protein encoded by the *amyL* gene may be denoted as AmyL or amyL). Similarly, the amylase gene and protein from *Bacillus* sp. strain 707 provided for herein are *amy707* and Amy707, respectively.

The term "heterologous" with reference to a polynucleotide or protein refers to a polynucleotide or protein that does not naturally occur in a host cell. In some embodiments, the protein is a commercially important industrial protein. It is intended that the term encompass proteins that are encoded by naturally occurring genes, mutated genes, and/or synthetic genes.

The term "endogenous" with reference to a polynucleotide or protein refers to a polynucleotide or protein that occurs naturally in the host cell.

As used herein, the term "expression" refers to the process by which a polypeptide is produced based on the nucleic acid sequence of a gene. The process includes both transcription and translation.

As used herein the term "specific activity" means an enzyme unit defined as the number of moles of substrate converted to product by an enzyme preparation per unit time under specific conditions. Specific activity is expressed as units (U)/mg of protein.

"ATCC" refers to American Type Culture Collection located at Manassas, Va. 20108 (ATCC).
"NRRL" refers to the Agricultural Research Service Culture Collection, National Center for Agricultural Utilization Research (and previously known as USDA Northern Regional Research Laboratory), Peoria, I11.
As used herein the term "comprising" and its cognates are used in their inclusive sense; that is, equivalent to the term "including" and its corresponding cognates.

1.2 Abbreviations

The following abbreviations apply unless indicated otherwise:

| | |
|---|---|
| AE | alcohol ethoxylate |
| AEO | alcohol ethoxylate |
| AEOS | alcohol ethoxysulfate |
| AES | alcohol ethoxysulfate |
| AFAU | acid fungal α-amylase units |
| AGU | glucoamylase activity unit |
| AOS | α-olefinsulfonate |
| AS | alcohol sulfate |
| BAA | *Bacillus amyloliquefaciens* α-amylase |
| BLA | *Bacillus licheniformis* (or LAT) |
| BSA | bovine serum albumin |
| cDNA | complementary DNA |
| CMC | carboxymethylcellulose |
| DNA | deoxyribonucleic acid |
| DP3 | degree of polymerization with three subunits |
| DPn | degree of polymerization with n subunits |
| DS | dry solids |
| DTMPA | diethyltriaminepentaacetic acid |
| EC | enzyme commission for enzyme classification |
| EDTA | ethylenediaminetetraacetic acid |
| EO | ethylene oxide |
| F&HC | fabric and household care |
| FAU | fungal amylase unit |
| GA | glucoamylase |

| | |
|---|---|
| gpg | grains per gallon |
| HFCS | high fructose corn syrup |
| HFSS | high fructose starch based syrup |
| IKW | Industrieverband Koerperpflege- und Waschmittel e. V. |
| IPTG | isopropyl β-D-1-thiogalactopyranoside |
| LAS | linear alkylbenezenesulfonate |
| LOM | Launder-O-meter |
| LU | Liquiphon unit |
| MW | molecular weight |
| MWU | modified Wohlgemuth unit |
| NOBS | nonanoyloxybenzenesulfonate |
| NTA | nitrilotriacetic acid |
| PCR | polymerase chain reaction |
| PEG | polyethyleneglycol |
| PVA | poly(vinyl alcohol) |
| PVP | poly(vinylpyrrolidone) |
| RNA | ribonucleic acid |
| SAS | secondary alkane sulfonates |
| TAED | tetraacetylethylenediamine |
| TCA | trichloroacetic acid |
| TSB | tryptic soy broth |
| UFC | ultrafiltration concentrate |
| w/v | weight/volume |
| w/w | weight/weight |
| wt | wild-type |

## 1.3 Nomenclature

In the present description and claims, the conventional one-letter and three-letter codes for amino acid residues are used. For ease of reference, alpha-amylase variants of the invention are described by use of the following nomenclature:

Original amino acid(s): position(s): substituted amino acid(s)

According to this nomenclature, for instance the substitution of serine by an alanine in position 242 is shown as:

Ser242Ala or S242A
a deletion of alanine in position 30 is shown as:
Ala30* or A30* or ΔA30
and insertion of an additional amino acid residue, such as lysine, is shown as:
Ala30AlaLys or A30AK

A deletion of a consecutive stretch of amino acid residues, such as amino acid residues 30-33, is indicated as (30-33)* or Δ(A30-N33) or Δ30-33. A deletion of two consecutive amino acids, such as amino acid residues R180-S181, is indicated as ΔRS or Δ180-181.
Where a specific alpha-amylase contains a "deletion" in comparison with other alpha-amylases and an insertion is made in such a position this is indicated as:

*36Asp or *36D

for insertion of an aspartic acid in position 36.
Multiple mutations are separated by plus signs, i.e.:

Ala30Asp+Glu34Ser or A30N+E34S

representing mutations in positions 30 and 34 substituting alanine and glutamic acid for asparagine and serine, respectively.
When one or more alternative amino acid residues may be inserted in a given position it is indicated as
A30N,E or
A30N or A30E
Furthermore, when a position suitable for modification is identified herein without any specific modification being suggested, it is to be understood that any amino acid residue may be substituted for the amino acid residue present in the position. Thus, for instance, when a modification of an alanine in position 30 is mentioned, but not specified, it is to be understood that the alanine may be deleted or substituted for any other amino acid, i.e., any one of:
R, N, D, A, C, Q, E, G, H, I, L, K, M, F, P, S, T, W, Y, V.
Further, "A30X" means any one of the following substitutions:

A30R, A30N, A30D, A30C, A30Q, A30E, A30G, A30H, A30I, A30L, A30K, A30M, A30F, A30P, A30S, A30T, A30W, A30Y, or A30 V;

or in short: A30R,N,D,C,Q,E,G,H,I,L,K,M,F,P,S,T,W,Y,V.
If the parent enzyme--used for the numbering--already has the amino acid residue in question suggested for substitution in that position the following nomenclature is used:

"X30N" or "X30N,V"

in the case where for instance one of N or V is present in the wildtype. Thus, it means that other corresponding parent enzymes are substituted to an "Asn" or "Val" in position 30.

## 1.4 Characteristics of Amino Acid Residues

Charged amino acids:

Asp, Glu, Arg, Lys, His

Negatively charged amino acids (with the most negative residue first):

Asp, Glu

Positively charged amino acids (with the most positive residue first):

Arg, Lys, His

Neutral amino acids:

Gly, Ala, Val, Leu, lie, Phe, Tyr, Trp, Met, Cys, Asn, Gln, Ser, Thr, Pro

Hydrophobic amino acid residues (with the most hydrophobic residue listed last):

Gly, Ala, Val, Pro, Met, Leu, lie, Tyr, Phe, Trp,

Hydrophilic amino acids (with the most hydrophilic residue listed last):

Thr, Ser, Cys, Gln, Asn

**1.5 Homology (Identity)**

A polynucleotide or a polypeptide having a certain percent (e.g. 80%, 83%, 85%, 90%, 95%, 96%, 97%, 98%, or 99%) of sequence identity with another sequence means that, when aligned, that percentage of bases or amino acid residues are the same in comparing the two sequences. This alignment and the percent homology or identity can be determined using any suitable software program known in the art, for example those described in CURRENT PROTOCOLS IN MOLECULAR BIOLOGY (F. M. Ausubel et al. (eds) 1987, Supplement 30, section 7.7.18). Preferred programs include the Vector NTI Advance™ 9.0 (Invitrogen Corp. Carlsbad, CA), GCG Pileup program, FASTA (Pearson et al. (1988) Proc. Natl, Acad. Sci USA 85:2444-2448), and BLAST (BLAST Manual, Altschul et al., Natl Cent. Biotechnol. Inf., Natl Lib. Med. (NCIB NLM NIH), Bethesda, Md., and Altschul et al., (1997) NAR 25:3389-3402). Another preferred alignment program is ALIGN Plus (Scientific and Educational Software, PA), preferably using default parameters. Another sequence software program that finds use is the TFASTA Data Searching Program available in the Sequence Software Package Version 6.0 (Genetics Computer Group, University of Wisconsin, Madison, WI).

The homology may be determined as the degree of identity between the two sequences indicating a derivation of the first sequence from the second. The homology may suitably be determined by means of computer programs known in the art such as GAP provided in the GCG program package (described above). Thus, Gap GCG v8 may be used with the default scoring matrix for identity and the following default parameters: GAP creation penalty of 5.0 and GAP extension penalty of 0.3, respectively for nucleic acidic sequence comparison, and GAP creation penalty of 3.0 and GAP extension penalty of 0.1, respectively, for protein sequence comparison. GAP uses the method of Needleman and Wunsch, (1970), J.Mol. Biol. 48:443-453, to make alignments and to calculate the identity.

A structural alignment between Amy707 (SEQ ID NO: 1) and, e.g., another alpha-amylase may be used to identify equivalent/corresponding positions in other alpha-amylases having a high degree of homology, e.g., 80%, 85%, 90%, 95%, 97% or 99%, with Amy707. One method of obtaining said structural alignment is to use the Pile Up programme from the GCG package using default values of gap penalties, i.e., a gap creation penalty of 3.0 and gap extension penalty of 0.1. Other structural alignment methods include the hydrophobic cluster analysis (Gaboriaud et al., (1987), FEBS LETTERS 224, pp. 149-155) and reverse threading (Huber, T; Torda, AE, PROTEIN SCIENCE Vol. 7, No. 1 pp. 142-149 (1998).

**1.6 Hybridisation**

The oligonucleotide probe used in the characterization of Amy707, above, may suitably be prepared on the basis of the full or partial nucleotide or amino acid sequence of the alpha-amylase in question.

Suitable conditions for testing hybridization involve pre-soaking in 5X SSC and prehybridizing for 1 hour at 40°C in a solution of 20% formamide, 5X Denhardt's solution, 50 mM sodium phosphate, pH 6.8, and 50 mg of denatured sonicated calf thymus DNA, followed by hybridization in the same solution supplemented with 100 mM ATP for 18 hours at 40°C, followed by three times washing of the filter in 2X SSC, 0.2% SDS at 40°C for 30 minutes (low stringency), preferred at 50°C (medium stringency), more preferably at 65°C (high stringency), even more preferably at 75°C (very high stringency). More details about the hybridization method can be found in Sambrook et al., Molecular Cloning: A Laboratory Manual, 2nd Ed., Cold Spring Harbor, 1989.

In the present context, "derived from" is intended not only to indicate an alpha-amylase produced or producible by a strain of the organism in question, but also an alpha-amylase encoded by a DNA sequence isolated from such strain and produced in a host organism transformed with said DNA sequence. Finally, the term is intended to indicate an alpha-amylase, which is encoded by a DNA sequence of synthetic and/or cDNA origin and which has the identifying characteristics of the alpha-amylase in question. The term is also intended to indicate that the parent alpha-amylase may be a variant of a naturally occurring alpha-amylase, i.e., a variant, which is the result of a modification (insertion, substitution, deletion) of one or more amino acid residues of the naturally occurring alpha-amylase.

One skilled in the art will recognize that sequences encompassed by the invention are also defined by the ability to hybridize under stringent hybridization conditions with the exemplified *amy707* sequence (e.g., **SEQ ID NO:3** shown in Figure 3). A nucleic acid is hybridizable to another nucleic acid sequence when a single stranded form of the nucleic acid can anneal to the other nucleic acid under appropriate conditions of temperature and solution ionic strength. Hybridization and washing conditions are well known in the art (See, e.g., Sambrook (1989) supra, particularly chapters 9 and 11). In some embodiments, stringent conditions correspond to a Tm of 65°C and 0.1×SSC, 0.1% SDS.

## 1.7 Parent Alpha-Amylases

According to the present disclosure any Amy707 alpha-amylase, as defined above, may be used as the parent (i.e., backbone) alpha-amylase. In a preferred embodiment the parent alpha-amylase is derived from *Bacillus* sp. strain 707, e.g., one of those referred to above, such as the 707 alpha-amylase having the amino acid sequence shown in SEQ ID NO: 1 (see Figure 1).

## 1.8 Altered Properties

The following section describes the relationship between mutations, which are present in a variant described herein, and desirable alterations in properties (relative to those of a parent 707 alpha-amylase), which may result therefrom.

As mentioned above the invention relates to an alpha-amylase derivable from *Bacillus sp* strain 707 and mutants thereof with altered properties.

Parent 707 alpha-amylases specifically contemplated in connection with the specifically contemplated altered properties are the above mentioned parent 707 alpha-amylase and parent hybrid alpha-amylases which comprise at least a portion of a 707 alpha-amylase.

The *Bacillus sp* strain 707 alpha-amylase (SEQ ID NO: 1) is used as the starting point, but corresponding positions in other Bacillus alpha-amylases having a high degree of homology should be understood as disclosed and specifically contemplated too.

In an aspect the invention relates to a variant with altered properties as mentioned above.

In the first aspect a variant of a parent *Bacillus sp.* strain alpha-amylase, comprising at least one, or at least two, of the following alterations:

(a) truncation of the C-terminus,
(b) substitution of amino acid 202 (i.e., M202), using SEQ ID NO:1 for numbering, or
(c) deletion of at least two residues selected from the group consisting of R181, G182, H182 and G184, using SEQ ID NO: for numbering, and wherein the variant has alpha-amylase activity.

### 1.8.1 Stability

In the context of the variants described herein, mutations (including amino acid substitutions and deletion) of importance with respect to achieving altered stability (i.e., higher or lower), in particular improved stability, at especially high temperatures (i.e., 70-120°C) and/or extreme pH (i.e. low or high pH, i.e, pH 4-6 or pH 8-11, respectively), in particular at free (i.e., unbound, therefore in solution) calcium concentrations below 60 ppm, include any of the mutations listed in the "Altered Properties" section. The stability may be determined as described in the "Methods" section below.

### 1.8.2 $Ca^{2+}$ Stability

Altered $Ca^{2+}$ stability means the stability of the enzyme under $Ca^{2+}$ depletion has been improved, i.e., higher or lower stability. In the context of the presently described variants, mutations (including amino acid substitutions and deletions) of importance with respect to achieving altered $Ca^{2+}$ stability, in particular improved $Ca^{2+}$ stability, i.e., higher or lower stability, at especially high pH (i.e., pH 8-10.5) include any of the mutations listed in the in "Altered Properties" section.

### 1.8.3 Specific Activity

In a further aspect, important mutations (including amino acid substitutions and deletions) with respect to obtaining variants exhibiting altered specific activity, in particular increased or decreased specific activity, especially at temperatures from 10-60°C, preferably 20-50°C, especially 30-40°C, include any of the mutations listed in the in "Altered properties" section. The specific activity may be determined as described in the "Methods" section below.

### 1.8.4 Oxidation Stability

The described variants may have altered oxidation stability, in particular higher oxidation stability, in comparison to the parent alpha-amylase. Increased oxidation stability is advantageous in, e.g., detergent compositions and decreased oxidation stability may be advantageous in composition for starch liquefaction. Oxidation stability may be determined as described in the "Methods" section below.

### 1.8.5 Altered pH Profile

Important positions and mutations with respect to obtaining variants with altered pH profile, in particular improved activity at especially high pH (i.e., pH 8-10.5) or low pH (i.e., pH 4-6) include mutations of amino residues located close to the active site residues.

Preferred specific mutations/substitutions are the ones listed above in the section "Altered Properties" for the positions in question. Suitable assays are described in the "Methods" section below.

### 1.8.6 Wash Performance

Important positions and mutations with respect to obtaining variants with improved wash performance at especially high pH (i.e., pH 8.5-11) include the specific mutations/substitutions listed above in the section "Altered Properties" for the positions in question. The wash performance may be tested as described below in the "Methods" section.

## 2. *Bacillus* sp. no. 707 α-Amylase, Variants Thereof, and Methods of Producing Same

Thus, one aspect provides for *Bacillus* sp. no. 707 α-amylase sequence in creating recombinant forms that include other previously determined amino acid substitutions, deletions, transversions, insertions, and combinations thereof to produce variants of the *Bacillus* sp. no. 707 α-amylase. These variants can have additional production enhancement, increased pH stability, increased temperature stability, reduced requirements for $Ca^{2+}$, increased specific activity, increased dishwashing or washing performance, increased solubility, increased storage stability, or combinations thereof. Methods of recombinantly generating the variants could be performed using the provided sequences and vectors, or using other modalities known in the art.

### 2.1 Cloning a DNA Sequence Encoding an α-amylase

The DNA sequence encoding a parent α-amylase may be isolated from any cell or microorganism producing the α-amylase in question, using various methods well known in the art. First, a genomic DNA and/or cDNA library should be constructed using chromosomal DNA or messenger RNA from the organism that produces the α-amylase to be studied. Then, if the amino acid sequence of the α-amylase is known, homologous, labelled oligonucleotide probes may be synthesized and used to identify α-amylase-encoding clones from a genomic library prepared from the organism in question. Alternatively, a labelled oligonucleotide probe containing sequences homologous to a known α-amylase gene could be used as a probe to identify α-amylase-encoding clones, using hybridization and washing conditions of lower stringency.

Yet another method for identifying α-amylase-encoding clones would involve inserting fragments of genomic DNA into an expression vector, such as a plasmid, transforming α-amylase-negative bacteria with the resulting genomic DNA library, and then plating the transformed bacteria onto agar containing a substrate for α-amylase, thereby allowing clones expressing the α-amylase to be identified.

Alternatively, the DNA sequence encoding the enzyme may be prepared synthetically by established standard methods, e.g. the phosphoamidite method described by S. L. Beaucage and M. H. Caruthers (1981) or the method described by Matthes et al. (1984). In the phosphoamidite method, oligonucleotides are synthesized, e.g. in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in U.S. Pat. No. 4,683,202 or R. K. Saiki et al. (1988).

### 2.2 Site-directed Mutagenesis

Once an α-amylase-encoding DNA sequence has been isolated, and desirable sites for mutation identified, mutations may be introduced using synthetic oligonucleotides. These oligonucleotides contain nucleotide sequences flanking the desired mutation sites; mutant nucleotides are inserted during oligonucleotide synthesis. In a specific method, a single-stranded gap of DNA, bridging the α-amylase-encoding sequence, is created in a vector carrying the α-amylase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al. (1984). U.S. Pat. No. 4,760,025 discloses the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method of introducing mutations into α-amylase-encoding DNA sequences is described in Nelson and Long (1989). It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

### 3. Production of α-Amylase Variants

A DNA sequence encoding the *Bacillus* sp. no. 707 α-amylase or variant thereof produced by methods described herein, or by any alternative methods known in the art, can be expressed, in enzyme form, using an expression vector that typically includes control sequences encoding a suitable promoter, operator, ribosome binding site, translation initiation signal, and, optionally, a repressor gene and/or various activator genes.

The recombinant expression vector carrying the DNA sequence encoding an α-amylase variant may be any vector that may conveniently be subjected to recombinant DNA procedures, and the choice of vector will often depend on the host cell into which it is to be introduced. Thus, the vector may be an autonomously replicating vector, *i.e.*, a vector that exists as an extrachromosomal entity, the replication of which is independent of chromosomal replication, *e.g.*, a plasmid, a bacteriophage or an extrachromosomal element, mini-chromosome or an artificial chromosome. Alternatively, the vector may be one which, when introduced into a host cell, is integrated into the host cell genome and replicates with the chromosome(s) into which it has been integrated. The integrated gene may also be amplified to create multiple copies of the gene in the chromosome by use of an amplifiable construct driven by antibiotic selection or other selective pressure, such as an essential regulatory genes or by complementation through dose effect of essential metabolic pathway genes.

In the vector, the DNA sequence should be operably connected to a suitable promoter sequence. The promoter may be any DNA sequence that shows transcriptional activity in the host cell of choice and may be derived from genes encoding proteins either homologous or heterologous to the host cell. Exemplary promoters for directing the transcription of the DNA sequence encoding an α-amylase variant, especially in a bacterial host, are the promoter of the lac operon of *E. coli,* the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, *A. niger* acid stable α-amylase, *A. niger* glucoamylase, *Rhizomucor miehei* lipase, *A. oryzae* alkaline protease, *A. oryzae* triose phosphate isomerase, or *A. nidulans* acetamidase. When the gene encoding the *Bacillus* sp. no. 707 α-amylase or variant thereof is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. For expression in *Trichoderma reesei,* the CBHII promoter may be used.

The expression vector may also comprise a suitable transcription terminator and, in eukaryotes, polyadenylation sequences operably connected to the DNA sequence encoding the *Bacillus* sp. no. 707 α-amylase or variant thereof. Termination and polyadenylation sequences suitably, but necessarily, are derived from the same sources as the promoter.

The vector may further comprise a DNA sequence enabling the vector to replicate in the host cell. Examples of such sequences are the origins of replication of plasmids pBR322, pUC19, pACYC177, pUB110, pE194, pAMB1, pICatH (FIG. 4, Genencor International, Inc.), pHPLT, and pIJ702, as well as vectors constructed from essential elements known to those skilled in the art.

The vector may also comprise a selectable marker, *e.g.* a gene the product of which complements a defect in the host cell, such as the *dal* genes from *B. subtilis* or *B. licheniformis,* or a gene that confers antibiotic resistance such as ampicillin, kanamycin, chloramphenicol, or tetracyclin resistance. Furthermore, the vector may comprise *Aspergillus* selection markers such as *amdS, argB, niaD* and *xxsC,* a marker giving rise to hygromycin resistance, or the selection may be accomplished by co-transformation, such as known in the art. *See, e.g.,* International PCT Application WO 91/17243.

While intracellular expression or solid state fermentation may be advantageous in some respects, e.g., when using certain bacteria or fungi as host cells, generally the expression of the enzyme is extracellular and into the culture medium. In general, the *Bacillus* sp. no. 707 α-amylase and variants thereof mentioned herein comprise a signal polypeptide format permitting secretion of the expressed enzyme into the culture medium. If desirable, the signal polypeptide peptide may be replaced by a different signal polypeptide, conveniently accomplished by substitution of the DNA sequences encoding the respective signal polypeptide. The signal polypeptides are typically characterized as having three domains, an N-terminal domain, an H-domain, and a C-terminal domain and range from 18 to 35 residues in length. These can be from any wild-type signal α-amylase or other secreted protein from a bacterium or from an eukaryote.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Generally, all signal sequences used will target the material to the cell culture medium for easier enzyme collection, and if applicable, purification.

The procedures used to ligate the DNA construct encoding the *Bacillus* sp. no. 707 α-amylase variant thereof, the promoter, terminator and other elements, respectively, and to insert them into suitable vectors containing the information necessary for replication, are well known to persons skilled in the art (cf., for instance, Sambrook et al., MOLECULAR CLONING: A LABORATORY MANUAL, 2nd ed., Cold Spring Harbor, 1989 and 3rd ed., 2001).

An isolated cell, either comprising a DNA construct or an expression vector, is advantageously used as a host cell in the recombinant production of the *Bacillus* sp. no. 707 α-amylase or variant thereof. The cell may be transformed with the DNA construct encoding the variant, conveniently by integrating the DNA construct (in one or more copies) in the host chromosome. This integration is generally an advantage as the DNA sequence is more likely to be stably maintained in the cell. Integration of the DNA constructs into the host chromosome may be performed according to conventional methods, *e.g.*, by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus lautus, Bacillus megaterium,* and *Bacillus thuringiensis; Streptomyces* species such as *Streptomyces murinus;* lactic acid bacterial species including *Lactococcus* spp. such as *Lactococcus lactis; Lactobacillus* spp. including *Lactobacillus reuteri; Leuconostoc* spp.; *Pediococcus* spp.; and *Streptococcus* spp. Alternatively, strains of a Gram negative bacterial species belonging to *Enterobacteriaceae* including *E. coli,* or to *Pseudomonadaceae* can be selected as the host organism. A suitable yeast host organism can be selected from the biotechnologically relevant yeasts species such as but not limited to yeast species such as *Pichia* sp., *Hansenula* sp., or *Kluyveromyces, Yarrowinia* species or a species of *Saccharomyces,* including *Saccharomyces cerevisiae* or a species belonging to *Schizosaccharomyces* such as, for example, *S. pombe* species. A strain of the methylotrophic yeast species *Pichia pastoris* can be used as the host organism. Alternatively, the host organism can be a *Hansenula* species. Suitable host organisms among filamentous fungi include species of *Aspergillus, e.g. Aspergillus niger, Aspergillus oryzae, Aspergillus tubigensis, Aspergillus awamori,* or *Aspergillus nidulans.* Alternatively, strains of a *Fusarium* species, *e.g. Fusarium oxysporum* or of a *Rhizomucor* species such as *Rhizomucor miehei* can be used as the host organism. Other suitable strains include *Thermomyces* and *Mucor* species.

Exemplary yeast species include a species of *Saccharomyces* or *Schizosaccharomyces, e.g., Saccharomyces cerevisiae.* The filamentous fungus may advantageously belong to a species of *Aspergillus, e.g., A. oryzae* or *A. niger.* Fungal cells may be transformed by a process involving protoplast formation and transformation/transfection of the protoplasts followed by regeneration of the cell wall in a manner known *per se.* A suitable procedure for transformation of *Aspergillus* host cells includes for example those described in EP 238023.

In a yet further aspect, a method of producing a *Bacillus* sp. no. 707 α-amylase or variant thereof is provided, which method comprises cultivating a host cell as described above under conditions conducive to the production of the variant and recovering the variant from the cells and/or culture medium.

The medium used to cultivate the cells may be any conventional medium suitable for growing the host cell in question and obtaining expression of the *Bacillus* sp. no. 707 α-amylase or variant thereof. Suitable media and media components are available from commercial suppliers (*e.g.*, Difco®) or may be prepared according to published recipes *(e.g.,* as described in catalogues of the American Type Culture Collection). The medium used would be that most suitable for the host cell being used. For example, the media discussed below for culturing *Bacillus licheniformis.* The growth medium in that case can consist of corn steep solids and soy flour as sources of organic compounds, along with inorganic salts as a source of sodium, potassium, phosphate, magnesium and sulfate, as well as trace elements. Typically, a carbohydrate source such as glucose is also part of the initial medium. Once the culture has established itself and begins growing, the carbohydrate is metered into the tank to maintain the culture as is known in the art. Samples are removed from the fermenter at regular intervals to measure enzyme titer using, for example, a colorimetric assay method. The fermentation process is halted when the enzyme production rate stops increasing according to the measurements.

*A Bacillus* sp. no. 707 α-amylase or variant thereof secreted from the host cells may be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

Host cells may be cultured under suitable conditions that allow expression of the *Bacillus* sp. no. 707 α-amylase or variant thereof. Expression of the proteins may be constitutive such that they are continually produced, or inducible, requiring a stimulus to initiate expression. In the case of inducible expression, protein production can be initiated when required by, for example, addition of an inducer substance to the culture medium, for example dexamethasone, IPTG, or Sepharose. Polypeptides can also be produced recombinantly in an *in vitro* cell-free system, such as the TnT™ (Promega) rabbit reticulocyte system.

*A Bacillus* sp. no. 707 α-amylase or variant thereof expressing host also can be cultured in the appropriate medium for the host, under aerobic conditions. Shaking or a combination of agitation and aeration can be provided, with production occurring at the appropriate temperature for that host, *e.g.*, from about 30°C to about 75°C, depending on the needs of the host and production of the desired enzyme. Culturing can occur from about 12 to about 100 hours (and any hour value there between) or greater, or for example from 24 to 72 hours, or alternatively from 75 hour to about 120 hours. Typically, the culture broth is at a pH of about 5.5 to about 8.0, again depending on the culture conditions needed for the host cell relative to production of the enzyme.

Assays are known in the art for determining activity. See for example U.S. Patent No. 6,297,037. For example, a soluble substrate assay can be performed to determine activity of a variant. This can be done for example using a rate assay which was developed based on an endpoint assay kit supplied by Megazyme (Aust.) Pty. Ltd. Substrate (p-nitrophenyl maltoheptaoside, BPNPG7) is dissolved in 10 mL of sterile water, followed by a 1 to 4 dilution in assay buffer (50 mM maleate buffer, pH 6.7, 5 mM calcium chloride, 0.002% Tween 20). Assays are performed by adding 10 μL of α-amylase to 790 μL of the substrate in a cuvette at 25°C. Rates of hydrolysis are measured as the rate of change of absorbance at 410 nm, after a delay of 75 seconds. The assay typically is linear up to rates of 0.4 absorption units/min. Enzyme concentration can be measured using for example a Bio-Rad assay (Bio-Rad Laboratories) based on the method of M. Bradford, Anal. Biochem. 72:248 (1976) and using bovine serum albumin standards.

Another assay which can be implemented to assess variant activity is a starch hydrolysis assay. This can be done for example as follows. The standard method for assaying α-amylase activity of Spezyme® AA20 can be used, or other alternatives known in the art. The Spezyme® AA20 assay is described for example in Example 1 of U.S. Application No. 07/785,624, incorporated herein by reference. Native starch forms a blue color with iodine, but fails to do so when it is hydrolyzed into shorter dextrin molecules. The substrate can be soluble Lintner starch 5 g/L in phosphate buffer, pH 6.2 (42.5 gm/liter potassium dihydrogen phosphate, 3.16 gm/liter sodium hydroxide). The sample is added in 25 mM calcium chloride and activity is measured as the time taken to give a negative iodine test upon incubation at 30°C. Activity is recorded in liquefons per gram or mL (LU) calculated according to the formula:

$$\text{LU/mL or LU/g} = \frac{(570)}{V \times t} \times D$$

Where LU = liquefon unit; V = volume of sample (5 mL); t = dextrinization time (minutes); D = dilution factor = dilution volume/mL or g of added enzyme.

4. **<u>Purification of α-Amylase Variants</u>**

Fermentation, separation, and concentration techniques are known in the art and conventional methods can be used in order to prepare the concentrated *Bacillus* sp. no. 707 α-amylase or variant thereof containing solution.

After fermentation, a fermentation broth is obtained, the microbial cells and various suspended solids, including residual raw fermentation materials, are removed by conventional separation techniques in order to obtain an enzyme containing solution. Filtration, centrifugation, microfiltration, rotary vacuum filtration, ultrafiltration, extraction or chromatography, or the like are generally used.

It is desirable to concentrate the enzyme containing solution in order to optimize recovery. Use of unconcentrated solutions requires increased incubation time in order to collect the purified enzyme containing precipitate.

The *Bacillus* sp. no. 707 α-amylase or variant thereof containing solution is concentrated using any available means known in the art until the desired enzyme level is obtained. For example, concentration of the enzyme containing solution may be achieved by any of the techniques discussed above. For example, rotary vacuum evaporation and/or ultrafiltration or ultrafiltration alone can be used.

By "precipitation agent" for purposes of purification is meant a compound effective to precipitate the *Bacillus* sp. no. 707 α-amylase or variant thereof from the concentrated enzyme solution in solid form, whatever its nature may be, *i.e.* crystalline, amorphous, or blend of both.

Precipitation can be performed using, for example, a metal halide precipitation agent. Metal halide precipitation agents include but are not limited to: alkali metal chlorides, alkali metal bromides and blends of two or more of these metal halides. Exemplary metal halides include sodium chloride, potassium chloride, sodium bromide, potassium bromide and blends of two or more of these metal halides. Or, the metal halide can be chosen from amongst sodium chloride and potassium chloride. For sodium chloride, it can be used both as a metal halide precipitation agent and a preservative.

The metal halide precipitation agent is used in an amount effective to precipitate the *Bacillus* sp. no. 707 α-amylase or variant thereof. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art after routine testing.

Generally, at least about 5% w/v (weight/volume) to about 25% w/v of metal halide is added to the concentrated enzyme containing solution, and usually at least 8% w/v. Generally, no more than about 25% w/v of metal halide is added to the concentrated enzyme containing solution and usually no more than about 20% w/v. The optimal concentration of the metal halide precipitation agent will depend, among others, on the nature of the *Bacillus* sp. no. 707 α-amylase or variant thereof and its concentration.

Another alternative to effect precipitation of the enzyme is to use of organic compounds, which can be added to the concentrated enzyme solution. The organic compound precipitating agent can include: 4-hydroxybenzoic acid, alkali metal salts of 4-hydroxybenzoic acid, alkyl esters of 4-hydroxybenzoic acid, and blends of two or more of these organic compounds. The addition of said organic compound precipitation agents can take place prior to, simultaneously with or subsequent to the addition of the metal halide precipitation agent, and the addition of both precipitation agents, organic compound and metal halide, may be carried out sequentially or simultaneously.

For further descriptions, *see, e.g.,* U.S. Patent No. 5,281,526. Generally, the organic compound precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be for example linear or branched alkyl esters of 4-hydroxybenzoic acid, wherein the alkyl group contains from 1 to 10 carbon atoms, and blends of two or more of these organic compounds. For example, the organic compounds can be linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl ester of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are amylase preservative agents.

Addition of the said organic compound precipitation agent provides the advantage of high flexibility of the precipitation conditions with respect to pH, temperature, enzyme concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least 0.01 % w/v (weight/volume) of organic compound precipitation agent is added to the concentrated enzyme containing solution and usually at least 0.02% w/v. Generally, no more than 0.3% w/v (weight/volume) of organic compound precipitation agent is added to the concentrated enzyme solution and usually no more than 0.2% w/v.

The concentrated enzyme solution, containing the metal halide precipitation agent and the organic compound precipitation agent, can be adjusted to a pH, which will of necessity depend on the enzyme to be purified. Generally, the pH is adjusted at a level near the isoelectric point of the *Bacillus* sp. no. 707 α-amylase or variant thereof. Generally, the pH is adjusted at a pH in a range from about 2.5 pH units below the isoelectric point (pI) up to about 2.5 pH units above the isoelectric point. For purposes of illustration, the concentrated enzyme containing solution can be adjusted to a pH of between about 5.5 and 9.7 or between about 6.5 and 9.0. Changes in pH range for a *Bacillus* sp. no. 707 α-amylase or variants thereof can similarly be prepared.

The incubation time necessary to obtain a purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less than about 10 hours, and in most cases even about 6 hours.

Generally, the temperature during incubation is between about 4°C and about 50°C. Usually, the method is carried out at a temperature for example between about 10°C and about 45°C, or between about 20°C and about 40°C. The optimal temperature for inducing precipitation varies according to the solution conditions and the enzyme or precipitation agent(s) used.

The overall recovery of purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration or the like. Further purification of the purified enzyme precipitate can be obtained by washing the precipitate with water. For example, the purified enzyme precipitate is washed with water containing the metal halide precipitation agent, or with water containing the metal halide and the organic compound precipitation agents.

During the culturing, the amylase accumulates extracellularly in the culture broth. For the isolation and purification of the desired *Bacillus* sp. no. 707 α-amylase or variant thereof, the culture broth is centrifuged or filtered to eliminate cells, and the resulting cell-free liquid is used for the purification of the enzyme. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme active fraction. For further purification, a conventional procedure such as ion exchange chromatography may be used. Alternatively, organic floccing agents can be used to floc cells or cell debris for removal of the cells and cell debris by filtration or centrifugation. The floccing agents can be used alone or in combination with metal halides or any of the procedures described herein. Generally, enzyme concentration may be on the order of 4 g/L or more and upwards of 25 g/L or more in some applications.

Purified enzyme is useful for all applications in which the enzymes are generally utilized. For example, they can be used in laundry detergents and spot removers, in the food industry, in starch processing and baking, and in pharmaceutical compositions as digestive aids. They can be made into a final product that is either liquid (solution, slurry) or solid (granular, powder). Formulations of the purified *Bacillus* sp. no. 707 α-amylase or variant thereof, are described herein.

Thus, the enzyme may be partially purified as generally described herein. This consists of removing cells by flocculation with polymers or microfiltration by concentrating by ultrafiltration using membranes and available materials. For some applications, the enzyme may not require purification and whole broth culture may be lysed and used without further treatment or be processed in to a granule or microgranules.

### 5. Cleaning and Dishwashing Compositions and Use

*A Bacillus* sp. no. 707 α-amylase or variant thereof, discussed herein can be formulated in detergent compositions for use in cleaning dishes or other cleaning compositions. These can be powders or liquids. For example, the composition can be in the form of a granulate or microgranulate. The polypeptide may further be stabilized in accordance with known methods. The compositions can comprise a *Bacillus* sp. no. 707 α-amylase or variant thereof, alone, other amylolytic enzymes, other cleaning enzymes, and other components common to cleaning compositions.

The composition may comprise a *Bacillus* sp. no. 707 α-amylase or variant thereof as the major enzymatic component, *e.g.*, a mono-component composition. Alternatively, the composition may comprise multiple enzymatic activities, such as an aminopeptidase, amylase, carbohydrase, carboxypeptidase, catalase, cellulase, chitinase, cutinase, cyclodextrin glycosyltransferase, deoxyribonuclease, esterase, α-galactosidase, β-galactosidase, glucoamylase, α-glucosidase, β-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase. The additional enzyme(s) may be producible by means of a microorganism belonging to the genera *Aspergillus, Trichoderma, Humicola,* and *Fusarium.* Exemplary members of the *Aspergillus* genus include *A. aculeatus, A. awamori, A. niger,* or *A. oryzae.* An exemplary member of the genus *Humicola* includes *Humicola insolens;* exemplary members of the genus *Fusarium* includes *F. bactridioides, F. cerealis, F. crookwellense, F. culmorum, F. graminearum, F. graminum, F. heterosporum, F. negundinis, F. oxysporum, F. reticulatum, F. roseum, F. sambucinum, F. sarcochroum, F. sulphureum, F. torulosum, F. trichothecioides,* and *F. venenatum.*

Thus, a dishwashing detergent composition can comprise a surfactant. The surfactant may be anionic, non-ionic, cationic, amphoteric or a mixture of these types. The detergent can contain 0% to about 90% of a non-ionic surfactant, such as low- to non-foaming ethoxylated propoxylated straight-chain alcohols.

In the detergent applications, a *Bacillus* sp. no. 707 α-amylase or variant thereof, can be used in a liquid composition containing propylene glycol. A *Bacillus* sp. no. 707 α-amylase or variant thereof, is solubilized, for example, in propylene glycol by circulating in a 25% volume/volume propylene glycol solution containing 10% calcium chloride.

The detergent composition may contain detergent builder salts of inorganic and/or organic types. The detergent builders may be subdivided into phosphorus-containing and non-phosphorus-containing types. The detergent composition usually contains about 1% to about 90% of detergent builders. Examples of phosphorus-containing inorganic alkaline detergent builders, when present, include the water-soluble salts, especially alkali metal pyrophosphates, orthophosphates, and polyphosphates. An example of phosphorus-containing organic alkaline detergent builder, when present, includes the water-soluble salts of phosphonates. Examples of non-phosphorus-containing inorganic builders, when present, include water-soluble alkali metal carbonates, borates, and silicates, as well as the various types of water-insoluble crystalline or amorphous alumino silicates, of which zeolites are the best-known representatives.

Examples of suitable organic builders include the alkali metal; ammonium and substituted ammonium; citrates; succinates; malonates; fatty acid sulfonates; carboxymethoxy succinates; ammonium polyacetates; carboxylates; polycarboxylates; aminopolycarboxylates; polyacetyl carboxylates; and polyhydroxysulfonates.

Other suitable organic builders include the higher molecular weight polymers and copolymers known to have builder properties, for example appropriate polyacrylic acid, polymaleic and polyacrylic/polymaleic acid copolymers, and their salts.

The cleaning composition may contain bleaching agents of the chlorine/bromine-type or the oxygen-type. Examples of inorganic chlorine/bromine-type bleaches are lithium, sodium or calcium hypochlorite, and hypobromite, as well as chlorinated trisodium phosphate. Examples of organic chlorine/bromine-type bleaches are heterocyclic N-bromo- and N-chloro-imides such as trichloroisocyanuric, tribromoisocyanuric, dibromoisocyanuric, and dichloroisocyanuric acids, and salts thereof with water-solubilizing cations such as potassium and sodium. Hydantoin compounds are also suitable.

The cleaning composition may contain oxygen bleaches, for example in the form of an inorganic persalt, and optionally further containing a bleach precursor or as a peroxy acid compound. Typical examples of suitable peroxy bleach compounds are alkali metal perborates, both tetrahydrates and monohydrates, alkali metal percarbonates, persilicates, and perphosphates. Exemplary activator materials include TAED and glycerol triacetate.

The cleaning composition may be stabilized using conventional stabilizing agents for the enzyme(s), *e.g.* a polyol such as, *e.g.*, propylene glycol, a sugar or a sugar alcohol, lactic acid, boric acid, or a boric acid derivative (e.g. an aromatic borate ester).

The cleaning composition may also contain other conventional detergent ingredients, *e.g.* deflocculant material, filler material, foam depressors, anti-corrosion agents, soil-suspending agents, sequestering agents, anti-soil redeposition agents, dehydrating agents, dyes, bactericides, fluorescers, thickeners, and perfumes.

*A Bacillus* sp. no. 707 α-amylase or variant thereof, that may be used in conventional dishwashing detergents, *e.g.*, in any of the detergents described in any of the following patent publications with the consideration that the *Bacillus* sp. no. 707 α-amylase or variant thereof, can comprise a signal sequence, for example from any of the *Bacillus* α-amylases listed in for example the following patents and patent applications: CA 2006687, GB 2200132, GB 2234980, GB 2228945, DE 3741617, DE 3727911, DE 4212166, DE 4137470, DE 3833047, DE 4205071, WO 93/25651, WO 93/18129, WO 93/04153, WO 92/06157, WO 92/08777, WO 93/21299, WO 93/17089, WO 93/03129, EP 481547, EP 530870, EP 533239, EP 554943, EP 429124, EP 346137, EP 561452, EP 318204, EP 318279, EP 271155, EP 271156, EP 346136, EP 518719, EP 518720, EP 518721, EP 516553, EP 561446, EP 516554, EP 516555, EP 530635, EP 414197, U.S. Patent No. 5,112,518, U.S. Patent No. 5,141,664, and U.S. Patent No. 5,240,632.

These compositions can be used in both manual and automatic dishwashing conditions. Exemplary formulations include but are not limited to the following:

1) POWDER AUTOMATIC DISHWASHING COMPOSITION: Nonionic surfactant 0.4-2.5%; sodium metasilicate 0-20%; sodium disilicate 3-20%; sodium triphosphate 20-40%; sodium carbonate 0-20%; sodium perborate 2-9%; tetraacetyl ethylene diamine (TAED) 1-4%; sodium sulfate 5-33%; and enzymes 0.0001-0.1%.

2) POWDER AUTOMATIC DISHWASHING COMPOSITION: Nonionic surfactant (e.g., alcohol ethoxylate) 1-2%; sodium disilicate 2-30%; sodium carbonate 10-50%; sodium phosphonate 0-5%; trisodium citrate dihydrate 9-30%; nitrilotrisodium acetate (NTA) 0-20%; sodium perborate monohydrate 5-10%; tetraacetylethylenediamine (TAED); 1-2% polyacrylate polymer (e.g., maleic acid/acrylic acid 6-25% copolymer); enzymes 0.0001-0.1%; perfume 0.1-0.5%; and water 5%-10%.

3) POWDER AUTOMATIC DISHWASHING COMPOSITION: Nonionic surfactant (e.g., alcohol ethoxylate) 1-2%; sodium disilicate 2-30%; sodium carbonate 10-50%; sodium phosphonate 0-5%; trisodium citrate dihydrate 9-30%; nitrilotrisodium acetate (NTA) 0-20%; sodium perborate monohydrate 5-10%; tetraacetylethylenediamine (TAED) 1-2%; polyacrylate polymer (e.g. maleic acid/acrylic acid 6-25% copolymer); enzymes 0.0001-0.1%; perfume 0.1-0.5%; and water 5%-10%.

4) POWDER AUTOMATIC DISHWASHING COMPOSITION: Nonionic surfactant 1-2%; zeolite MAP 15-42%; sodium disilicate 30-34%; sodium citrate 0-12%; sodium carbonate 0-20%; sodium perborate monohydrate 7-15%; tetraacetylethylenediamine (TAED) 0-3%; polymer 0-4%; maleic acid/acrylic acid copolymer 0-5%; organic phosphonate 0-4%; clay 1-2%; enzymes 0.0001-0.1%; sodium sulfate for the balance of the formulation.

5) POWDER AUTOMATIC DISHWASHING COMPOSITION: Nonionic surfactant 1-2%; zeolite MAP 15-42%; sodium disilicate 30-34%; sodium citrate 0-12%; sodium carbonate 0-20%; sodium perborate monohydrate 7-15%; tetraacetylethylenediamine (TAED) 0-3%; polymer 0-4%; maleic acid/acrylic acid copolymer 0-5%; organic phosphonate 0-4%; clay 1-2%; enzymes 0.0001-0.1%; sodium sulfate for the balance of the formulation.

6) POWDER AUTOMATIC DISHWASHING COMPOSITION: Nonionic surfactant 1-2%; zeolite MAP 15-42%; sodium disilicate 30-34%; sodium citrate 0-12%; sodium carbonate 0-20%; sodium perborate monohydrate 7-15%; tetraacetylethylenediamine (TAED) 0-3%; polymer 0-4%; maleic acid/acrylic acid copolymer 0-5%; organic phosphonate 0-4%; clay 1-2%; enzymes 0.0001-0.1%; sodium sulfate for the balance of the formulation.

7) NON-AQUEOUS LIQUID AUTOMATIC DISHWASHING COMPOSITION: Liquid nonionic surfactant (e.g., alcohol ethoxylates) 2.0-10.0%; alkali metal silicate 3.0-15.0%; alkali metal phosphate 20.0-40.0%; liquid carrier selected from higher glycols, polyglycols 25.0-45.0%; polyoxides, glycol ether stabilizer (e.g., a partial ester of phosphoric acid and a 0.5-7.0% $C_{16}$-$C_{18}$ alkanol); foam suppressor (e.g., silicone) 0-1.5%; and enzymes 0.0001-0.1%.

8) NON-AQUEOUS LIQUID DISHWASHING COMPOSITION: Liquid nonionic surfactant (e.g., alcohol ethoxylates) 2.0-40.0%; sodium silicate 3.0-15.0%; alkali metal carbonate 7.0-20.0%; sodium citrate 0.0-1.5%; stabilizing system (e.g., mixtures of finely divided 0.5-7.0% silicone and low molecular weight dialkyl polyglycol ethers); low molecule weight polyacrylate polymer 5.0-15.0%; clay gel thickener (e.g., bentonite) 0.0-10.0%; hydroxypropyl cellulose polymer 0.0-0.6%; enzymes 0.0001-0.1%; and liquid carrier selected from higher lycols, poly- balance glycols, polyoxides, and glycol ethers.

9) NON-AQUEOUS LIQUID DISHWASHING COMPOSITION: Liquid nonionic surfactant (e.g., alcohol ethoxylates) 2.0-40.0%; sodium silicate 3.0-15.0%; alkali metal carbonate 7.0-20.0%; sodium citrate 0.0-1.5%; stabilizing system (e.g. mixtures of finely divided 0.5-7.0% silicone and low molecular weight dialkyl polyglycol ethers); low molecular weight polyacrylate polymer 5.0-15.0%; clay gel thickener (e.g., bentonite) 0.0-10.0%; hydroxypropyl cellulose polymer 0.0-0.6%; enzymes 0.0001-0.1%; liquid carrier selected from higher glycols, poly- balance glycols, polyoxides, and glycol ethers.

10) LIQUID AUTOMATIC DISHWASHING COMPOSITION: Alcohol ethoxylate 0-20%; fatty acid ester sulfonate 0-30%; sodium dodecyl sulfate 0-20%; alkyl polyglycoside 0-21%; oleic acid 0-10%; sodium disilicate monohydrate 18-33%; sodium citrate dihydrate 18-33%; sodium stearate 0-2.5%; sodium perborate monohydrate 0-13%; tetraacetylethylenediamine (TAED) 0-8%; maleic acid/acrylic acid copolymer 4-8%; and enzymes 0.0001-0.1%.

11) LIQUID AUTOMATIC DISHWASHING COMPOSITION: Alcohol ethoxylate 0-20%; fatty acid ester sulfonate 0-30%; sodium dodecyl sulfate 0-20%; alkyl polyglycoside 0-21%; oleic acid 0-10%; sodium disilicate monohydrate 18-33%; sodium citrate dihydrate 18-33%; sodium stearate 0-2.5%; sodium perborate monohydrate 0-13%; tetraacetylethylenediamine (TAED) 0-8%; maleic acid/acrylic acid copolymer 4-8%; and enzymes 0.0001-0.1%.

12) Automatic dishwashing compositions as described in 1), 2) 3), 4), 6) and 10), wherein perborate is replaced by percarbonate.

13) Automatic dishwashing compositions as described in 1)-6) which additionally contain a manganese catalyst.

## 6. Laundry Detergent Compositions and Use

According to the embodiment, one or more *Bacillus* sp. no. 707 α-amylases or variants thereof, may typically be a component of a laundry detergent composition. As such, it may be included in the detergent composition in the form of a non-dusting granulate, a stabilized liquid, or a protected enzyme. The dry formulations may be in the form of a granulate or microgranulate. Non-dusting granulates may be produced, *e.g.* as disclosed in U.S. Patent Nos. 4,106,991 and 4,661,452 and may optionally be coated by methods known in the art. Examples of waxy coating materials are poly (ethylene oxide) products (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB Patent No. 1483591. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in for example EP Appln. No. 238,216. Polyols have long been recognized as stabilizers of proteins as well as improving solubility of proteins. *See, e.g.*, J. K. Kaushik et al., "Why is trehalose an exceptional protein stabilizer? An analysis of the thermal stability of proteins in the presence of the compatible osmolyte trehalose," J. Biol. Chem. 278:

26458-65 (2003) and the references cited therein; and Monica Conti et al., "Capillary isoelectric focusing: the problem of protein solubility," J. Chromatography 757: 237-245 (1997).

The composition may comprise a *Bacillus* sp. no. 707 α-amylase or variants thereof as the major enzymatic component, e.g., a mono-component composition. Alternatively, the composition may comprise multiple enzymatic activities, such as an aminopeptidase, amylase, carbohydrase, carboxypeptidase, catalase, cellulase, chitinase, cutinase, cyclodextrin glycosyltransferase, deoxyribonuclease, esterase, α-galactosidase, β-galactosidase, glucoamylase, α-glucosidase, β-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase, as well as other enzymes discussed below. The additional enzyme(s) may be producible by means of a microorganism belonging to the genera *Aspergillus, Trichoderma, Humicola* (*e.g., H. insolens*), and *Fusarium.* Exemplary members of the *Aspergillus* genus include *Aspergillus aculeatus, Aspergillus awamori, Aspergillus niger,* or *Aspergillus oryzae.* Exemplary members of the genus *Fusarium* include *Fusarium bactridioides, Fusarium cerealis, Fusarium crookwellense, Fusarium culmorum, Fusarium graminearum, Fusarium graminum, Fusarium heterosporum, Fusarium negundinis, Fusarium oxysporum, Fusarium reticulatum, Fusarium roseum, Fusarium sambucinum, Fusarium sarcochroum, Fusarium sulphureum, Fusarium torulosum, Fusarium trichothecioides,* and *Fusarium venenatum.*

The detergent composition may be in any useful form, e.g., powders, granules, pastes, or liquids. A liquid detergent may be aqueous, typically containing up to about 70% of water, and 0% to about 30% of organic solvent. It can also be a in the form of a compact gel type containing only about 30% water. Enzymes may be used in any detergent composition compatible with the stability of the enzyme. Enzymes can be protected against generally deleterious components by known forms of encapsulation as for example by granulation or sequestration in hydro gels. Enzymes and specifically α-amylases are not limited to laundry and dish washing applications, but can also be used in surface cleaners, ethanol production from starch or biomass.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); α-olefinsulfonate (AOS); alkyl sulfate (fatty alcohol sulfate) (AS); alcohol ethoxysulfate (AEOS or AES); secondary alkanesulfonates (SAS); α-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide (as described for example in WO 92/06154).

The detergent composition may additionally comprise one or more other enzymes, such as lipase, cutinase, protease, cellulase, peroxidase, and/or laccase in any combination.

The detergent may optionally contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (e.g. SKS-6 from Hoechst). The detergent may also be unbuilt, i.e. essentially free of detergent builder.

The detergent may optionally comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may optionally contain a bleaching system, which may comprise a $H_2O_2$ source such as perborate or percarbonate, which may be combined with a peracid-forming bleach activator such as tetraacetylethylenediamine (TAED) or nonanoyloxybenzenesulfonate (NOBS). Alternatively, the bleaching system may comprise peroxy acids of *e.g.* the amide, imide, or sulfone type. The bleaching system can also be an enzymatic bleaching system, where a perhydrolase activates peroxide, as described in for example WO 2005/056783.

The enzymes of the detergent composition may be stabilized using conventional stabilizing agents, *e.g.*, a polyol such as propylene glycol or glycerol; a sugar or sugar alcohol; lactic acid; boric acid or a boric acid derivative such as, *e.g.*, an aromatic borate ester; and the composition may be formulated as described in, *e.g.*, WO 92/19709 and WO 92/19708.

The detergent may also contain other conventional detergent ingredients such as, *e.g.*, fabric conditioners including clays, foam boosters, suds suppressors, anti-corrosion agents, soil-suspending agents, anti-soil redeposition agents, dyes, bactericides, optical brighteners, or perfume.

The pH (measured in aqueous solution at use concentration) is usually neutral or alkaline, *e.g.*, pH about 7.0 to about 11.0.

Particular forms of detergent compositions comprising a *Bacillus* sp. no. 707 α-amylase or variant thereof, can be formulated to include:

1) A detergent composition formulated as a granulate having a bulk density of at least 600 g/L comprising linear alkylbenzenesulfonate (calculated as acid) about 7% to about 12%; alcohol ethoxysulfate (*e.g.*, $C_{12-18}$ alcohol, 1-2

ethylene oxide (EO)) or alkyl sulfate (*e.g.*, $C_{16-18}$) about 1% to about 4%; alcohol ethoxylate (*e.g.*, $C_{14-15}$ alcohol, 7 EO) about 5% to about 9%; sodium carbonate (*e.g.*, $Na_2CO_3$) about 14% to about 20%; soluble silicate (*e.g.*, $Na_2O$, $2SiO_2$) about 2 to about 6%; zeolite (*e.g.*, $NaAlSiO_4$) about 15% to about 22%; sodium sulfate *(e.g.,* $Na_2SO_4$) 0% to about 6%; sodium citrate/citric acid (*e.g.*, $C_6H_5Na_3O_7$/$C_6H_8O_7$) about 0% to about 15%; sodium perborate (*e.g.*, $NaBO_3H_2O$) about 11% to about 18%; TAED about 2% to about 6%; carboxymethylcellulose (CMC) and 0% to about 2%; polymers (*e.g.*, maleic/acrylic acid, copolymer, PVP, PEG) 0-3%; enzymes (calculated as pure enzyme) 0.0001-0.1% protein; and minor ingredients (*e.g.*, suds suppressors, perfumes, optical brightener, photobleach) 0-5%.

2) A detergent composition formulated as a granulate having a bulk density of at least 600 g/L comprising linear alkylbenzenesulfonate (calculated as acid) about 6% to about 11%; alcohol ethoxysulfate (*e.g.*, $C_{12-18}$ alcohol, 1-2 EO) or alkyl sulfate (*e.g.*, $C_{16-18}$) about 1% to about 3%; alcohol ethoxylate (*e.g.*, $C_{14-15}$ alcohol, 7 EO) about 5% to about 9%; sodium carbonate (*e.g.*, $Na_2CO_3$) about 15% to about 21%; soluble silicate (*e.g.*, $Na_2O$, $2SiO_2$) about 1% to about 4%; zeolite (*e.g.*, $NaAlSiO_4$) about 24% to about 34%; sodium sulfate (*e.g*,. $Na_2SO_4$) about 4% to about 10%; sodium citrate/citric acid (*e.g.*, $C_6H_5Na_3O_7$/ $C_6H_8O_7$) 0% to about 15%; carboxymethylcellulose (CMC) 0% to about 2%; polymers (*e.g.*, maleic/acrylic acid copolymer, PVP, PEG) 1-6%; enzymes (calculated as pure enzyme protein) 0.0001-0.1%; minor ingredients *(e.g.,* suds suppressors, perfume) 0-5%.

3) A detergent composition formulated as a granulate having a bulk density of at least 600 g/L comprising linear alkylbenzenesulfonate (calculated as acid) about 5% to about 9%; alcohol ethoxylate (*e.g.*, $C_{12-15}$ alcohol, 7 EO) about 7% to about 14%; Soap as fatty acid *(e.g.,* $C_{16-22}$ fatty acid) about 1 to about 3%; sodium carbonate (as $Na_2CO_3$) about 10% to about 17%; soluble silicate (*e.g.*, $Na_2O$, $2SiO_2$) about 3% to about 9%; zeolite (as $NaAlSiO_4$) about 23% to about 33%; sodium sulfate (*e.g.*, $Na_2SO_4$) 0% to about 4%; sodium perborate (*e.g.*, $NaBO_3H_2O$) about 8% to about 16%; TAED about 2% to about 8%; phosphonate (*e.g.*, EDTMPA) 0% to about 1%; carboxymethylcellulose (CMC) 0% to about 2%; polymers (*e.g.*, maleic/acrylic acid copolymer, PVP, PEG) 0-3%; enzymes (calculated as pure enzyme protein) 0.0001-0.1%; minor ingredients (*e.g.*, suds suppressors, perfume, optical brightener) 0-5%.

4) A detergent composition formulated as a granulate having a bulk density of at least 600 g/L comprising linear alkylbenzenesulfonate (calculated as acid) about 8% to about 12%; alcohol ethoxylate (*e.g.*, $C_{12-15}$ alcohol, 7 EO) about 10% to about 25%; sodium carbonate (as $Na_2CO_3$) about 14% to about 22%; soluble silicate (*e.g.*, $Na_2O$, $2SiO_2$) about 1% to about 5%; zeolite (*e.g.*, $NaAlSiO_4$) about 25% to about 35%; sodium sulfate (*e.g.*, $Na_2SO_4$) 0% to about 10%; carboxymethylcellulose (CMC) 0% to about 2%; polymers (*e.g.*, maleic/acrylic acid copolymer, PVP, PEG) 1-3%; enzymes (calculated as pure enzyme protein) 0.0001-0.1%; and minor ingredients (*e.g.*, suds suppressors, perfume) 0-5%.

5) An aqueous liquid detergent composition comprising linear alkylbenzenesulfonate (calculated as acid) about 15% to about 21%; alcohol ethoxylate (e.g., $C_{12-15}$ alcohol, 7 EO or $C_{12-15}$ alcohol, 5 EO) about 12% to about 18%; soap as fatty acid (*e.g.*, oleic acid) about 3% to about 13%; alkenylsuccinic acid ($C_{12-14}$) 0% to about 13%; aminoethanol about 8% to about 18%; citric acid about 2% to about 8%; phosphonate 0% to about 3%; polymers (e.g., PVP, PEG) 0% to about 3%; borate (e.g., $B_4O_7$) 0% to about 2%; ethanol 0% to about 3%; propylene glycol about 8% to about 14%; enzymes (calculated as pure enzyme protein) 0.0001-0.1%; and minor ingredients (*e.g.*, dispersants, suds suppressors, perfume, optical brightener) 0-5%.

6) An aqueous structured liquid detergent composition comprising linear alkylbenzenesulfonate (calculated as acid) about 15% to about 21%; alcohol ethoxylate (e.g., $C_{12-15}$ alcohol, 7 EO, or $C_{12-15}$ alcohol, 5 EO) 3-9%; soap as fatty acid (e.g., oleic acid) about 3% to about 10%; zeolite (as $NaAlSiO_4$) about 14% to about 22%; potassium citrate about 9% to about 18%; borate (e.g., $B_4O_7$) 0% to about 2%; carboxymethylcellulose (CMC) 0% to about 2%; polymers *(e.g.,* PEG, PVP) 0% to about 3%; anchoring polymers such as, *e.g.*, lauryl methacrylate/acrylic acid copolymer; molar ratio 25:1, MW 3800) 0% to about 3%; glycerol 0% to about 5%; enzymes (calculated as pure enzyme protein) 0.0001-0.1%; and minor ingredients (*e.g.*, dispersants, suds suppressors, perfume, optical brighteners) 0-5%.

7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate (*e.g.*, $Na_2CO_3$) about 5% to about 10%; Soluble silicate (*e.g.*, $Na_2O$, $2SiO_2$) about 1% to about 4%; zeolite (*e.g.*, $NaAlSiO_4$) about 20% to about 40%; Sodium sulfate (*e.g.*, $Na_2SO_4$) about 2% to about 8%; sodium perborate (*e.g.*, $NaBO_3H_2O$) about 12% to about 18%; TAED about 2% to about 7%; polymers (*e.g.*, maleic/acrylic acid copolymer, PEG) about 1% to about 5%; enzymes (calculated as pure enzyme protein) 0.0001-0.1%; and minor ingredients (*e.g.*, optical brightener, suds suppressors, perfume) 0-5%.

8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate (*e.g.*, $Na_2CO_3$) about 4% to about 10%; soluble silicate ($Na_2O$, $2SiO_2$) about 1% to about 4%; zeolite (*e.g.*, $NaAlSiO_4$) about 30% to about 50%; sodium sulfate (*e.g.*, $Na_2SO_4$) about 3% to about 11%; sodium citrate (*e.g.*, $C_6H_5Na_3O_7$) about 5% to about 12%; polymers (*e.g.*, PVP, maleic/acrylic acid copolymer, PEG) about 1% to about 5%; enzymes (calculated as pure enzyme protein) 0.0001-0.1%; and minor ingredients (*e.g.*, suds suppressors, perfume) 0-5%.

9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate (*e.g.*, $Na_2CO_3$) about 14% to about 22%; zeolite (*e.g.*, $NaAlSiO_4$) about 18% to about 32%; sodium sulfate (*e.g.*, $Na_2SO_4$) about 5% to about 20%; sodium citrate (*e.g.*, $C_6H_5Na_3O_7$) about 3% to about 8%; sodium perborate (*e.g.*, $NaBO_3H_2O$) about 4% to about 9%; bleach activator (*e.g.*, NOBS or TAED) about 1% to about 5%; carboxymethylcellulose (CMC) 0% to about 2%; polymers (*e.g.*, polycarboxylate or PEG) about 1% to about 5%; enzymes (calculated as pure enzyme protein) 0.0001-0.1%; and minor ingredients (*e.g.*, optical brightener, perfume) 0-5%.

10) An aqueous liquid detergent composition comprising linear alkylbenzenesulfonate (calculated as acid) about 15% to about 23%; alcohol ethoxysulfate (*e.g.*, $C_{12-15}$ alcohol, 2-3 EO) about 8% to about 15%; alcohol ethoxylate (*e.g.*, $C_{12-15}$ alcohol, 7 EO, or $C_{12-15}$ alcohol, 5 EO) about 3% to about 9%; soap as fatty acid (*e.g.*, lauric acid) 0% to about 3%; aminoethanol about 1% to about 5%; sodium citrate about 5% to about 10%; hydrotrope (*e.g.*, sodium toluensulfonate) about 2% to about 6%; borate (*e.g.*, $B_4O_7$) 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (*e.g.*, polymers, dispersants, perfume, optical brighteners) 0-5%.

11) An aqueous liquid detergent composition comprising linear alkylbenzenesulfonate (calculated as acid) about 20% to about 32%; alcohol ethoxylate (*e.g.*, $C_{12-15}$ alcohol, 7 EO, or $C_{12-15}$ alcohol, 5 EO) 6-12%; aminoethanol about 2% to about 6%; citric acid about 8% to about 14%; borate (*e.g.,* $B_4O_7$) about 1% to about 3%; polymer (*e.g.*, maleic/acrylic acid copolymer, anchoring polymer such as, *e.g.*, lauryl methacrylate/acrylic acid copolymer) 0% to about 3%; glycerol about 3% to about 8%; enzymes (calculated as pure enzyme protein) 0.0001-0.1%; and minor ingredients (*e.g.*, hydrotropes, dispersants, perfume, optical brighteners) 0-5%.

12) A detergent composition formulated as a granulate having a bulk density of at least 600 g/L comprising anionic surfactant (linear alkylbenzenesulfonate, alkyl sulfate, $\alpha$-olefinsulfonate, $\alpha$-sulfo fatty acid methyl esters, alkanesulfonates, soap) about 25% to about 40%; nonionic surfactant (*e.g.*, alcohol ethoxylate) about 1% to about 10%; sodium carbonate *(e.g.,* $Na_2CO_3$) about 8% to about 25%; soluble silicates (*e.g.*, $Na_2O$, $2SiO_2$) about 5% to about 15%; sodium sulfate (*e.g.*, $Na_2SO_4$) 0% to about 5%; zeolite ($NaAlSiO_4$) about 15% to about 28%; sodium perborate (*e.g.*, $NaBO_3.4H_2O$) 0% to about 20%; bleach activator (TAED or NOBS) about 0% to about 5%; enzymes (calculated as pure enzyme protein) 0.0001-0.1%; minor ingredients (*e.g.*, perfume, optical brighteners) 0-3%.

13) Detergent compositions as described in compositions 1)-12) *supra,* wherein all or part of the linear alkylbenzenesulfonate is replaced by ($C_{12}$-$C_{18}$) alkyl sulfate.

14) A detergent composition formulated as a granulate having a bulk density of at least 600 g/L comprising ($C_{12}$-$C_{18}$) alkyl sulfate about 9% to about 15%; alcohol ethoxylate about 3% to about 6%; polyhydroxy alkyl fatty acid amide about 1% to about 5%; zeolite (*e.g.,* $NaAlSiO_4$) about 10% to about 20%; layered disilicate (*e.g.*, SK56 from Hoechst) about 10% to about 20%; sodium carbonate (*e.g.*, $Na_2CO_3$) about 3% to about 12%; soluble silicate *(e.g.,* $Na_2O$, $2SiO_2$) 0% to about 6%; sodium citrate about 4% to about 8%; sodium percarbonate about 13% to about 22%; TAED about 3% to about 8%; polymers (*e.g.*, polycarboxylates and PVP) 0% to about 5%; enzymes (calculated as pure enzyme protein) 0.0001-0.1%; and minor ingredients (*e.g.*, optical brightener, photobleach, perfume, suds suppressors) 0-5%.

15) A detergent composition formulated as a granulate having a bulk density of at least 600 g/L comprising ($C_{12}$-$C_{18}$) alkyl sulfate about 4% to about 8%; alcohol ethoxylate about 11% to about 15%; soap about 1% to about 4%; zeolite MAP or zeolite A about 35% to about 45%; sodium carbonate (as $Na_2CO_3$) about 2% to about 8%; soluble silicate (*e.g.*, $Na_2O$, $2SiO_2$) 0% to about 4%; sodium percarbonate about 13% to about 22%; TAED 1-8%; carboxymethylcellulose (CMC) 0% to about 3%; polymers (*e.g.*, polycarboxylates and PVP) 0% to about 3%; enzymes (calculated as pure enzyme protein) 0.0001-0.1%; and minor ingredients (e.g., optical brightener, phosphonate, perfume) 0-3%.

16) Detergent formulations as described in 1)-15) supra, which contain a stabilized or encapsulated peracid, either as an additional component or as a substitute for already specified bleach systems.

17) Detergent compositions as described supra in 1), 3), 7), 9), and 12), wherein perborate is replaced by percarbonate.

18) Detergent compositions as described supra in 1), 3), 7), 9), 12), 14), and 15), which additionally contain a manganese catalyst. The manganese catalyst for example is one of the compounds described in "Efficient manganese catalysts for low-temperature bleaching," Nature 369: 637-639 (1994).

19) Detergent composition formulated as a non-aqueous detergent liquid comprising a liquid nonionic surfactant such as, *e.g.*, linear alkoxylated primary alcohol, a builder system (*e.g.*, phosphate), an enzyme(s), and alkali. The detergent may also comprise anionic surfactant and/or a bleach system.

20) An aqueous liquid laundry detergent composition comprising:; A) at least one surfactant selected from the group consisting of anionic surfactants, nonionic surfactants, zwitterionic surfactants, amphoteric surfactants, and combinations thereof;; B) droplets of a blend of silicone materials, which blend comprises:; i) an amine or ammonium group-containing functionalized polysiloxane material which:; a) has been prepared by a process which intrinsically leaves curable/reactive groups in the functionalized polysiloxane material which is produced;; b) has a molar ratio of curable/reactive group-containing silicon atoms to terminal silicon atoms containing no reactive/curable groups which is less than about 30%;; c) has a nitrogen content of from about 0.05% to about 0.30% by weight; and; d) has viscosity at 20 C. ranging from about 0.00002 m; 2; /s to about 0.2 m; 2; /s; and; ii) a nitrogen-free, non-functionalized polysiloxane material having a viscosity of from about 0.01 m; 2; /s to about 2.0 m; 2; /s and present in an amount such that within said blend the weight ratio of functionalized polysiloxane material to non-functionalized polysiloxane material ranges from about 100:1 to about 1:100; and; C) at least one additional non-silicone laundry adjunct selected from the group consisting of dye transfer inhibiting agents, optical brighteners, and combinations thereof.

*A Bacillus* sp. no. 707 α-amylase or variant thereof, may be incorporated in concentrations conventionally employed in detergents. It is at present contemplated that, in the detergent composition, a *Bacillus* sp. no. 707 α-amylase or variant thereof, may be added in an amount corresponding to 0.00001-1.0 mg (calculated as pure enzyme protein) of enzyme per liter of wash liquor.

In another embodiment, a 2,6-β-D-fructan hydrolase can be incorporated in detergent compositions and used for removal/cleaning of biofilm present on household and/or industrial textile/laundry.

The detergent composition may for example be formulated as a hand or machine laundry detergent composition, including a laundry additive composition suitable for pre-treatment of stained fabrics and a rinse added fabric softener composition, or be formulated as a detergent composition for use in general household hard surface cleaning operations, or be formulated for manual or machine laundry operations.

In a specific aspect, the detergent composition can further comprise 2,6-β-D-fructan hydrolase, one or more α-amylases in addition to the *Bacillus* sp. no. 707 α-amylase or variant thereof, and one or more other cleaning enzymes, such as a protease, a lipase, a cutinase, a carbohydrase, a cellulase, a pectinase, a mannanase, an arabinase, a galactanase, a xylanase, an oxidase, a laccase, and/or a peroxidase, and/or combinations thereof.

In general the properties of the chosen enzyme(s) should be compatible with the selected detergent, (*e.g.*, pH-optimum, compatibility with other enzymatic and non-enzymatic ingredients, *etc.*), and the enzyme(s) should be present in effective amounts.

Proteases: suitable proteases include those of animal, vegetable or microbial origin. Chemically modified or protein engineered mutants are included as well as naturally processed proteases. The protease may be a serine protease or a metalloprotease, such as an alkaline microbial protease, a trypsin-like protease, or a chymotrypsin-like protease. Examples of alkaline proteases are subtilisins, especially those derived from *Bacillus, e.g.,* subtilisin Novo, subtilisin Carlsberg, subtilisin 309, subtilisin 147, and subtilisin 168 (*see, e.g.,* WO 89/06279). Examples of trypsin-like proteases are trypsin *(e.g.,* of porcine or bovine origin), and *Fusarium* proteases (*see, e.g.,* WO 89/06270 and WO 94/25583). Examples of useful proteases also include but are not limited to the variants described in WO 92/19729, WO 98/20115, WO 98/20116, and WO 98/34946. Exemplary commercially available protease enzymes include Alcalase®, Savinase®, Primase™, Duralase™, Esperase®, and Kannase™ (Novo Nordisk A/S), Maxatase®, Maxacal™, Maxapem™, Properase®, Purafect®, Purafect OxP™, FN2™, and FN3™ (Genencor International, Inc.).

Lipases: suitable lipases include those of bacterial or fungal origin. Chemically modified, proteolytically modified, or protein engineered mutants are included. Examples of useful lipases include but are not limited to lipases from *Humicola* (synonym *Thermomyces*), *e.g.* from *H. lanuginosa* (*T. lanuginosus*) (*see e.g.*, EP 258068 and EP 305216), from *H. insolens* (*see e.g.,* WO 96/13580); a *Pseudomonas* lipase (*e.g.* from *P. alcaligenes* or *P. pseudoalcaligenes*; *see, e.g.,* EP 218 272), *P. cepacia* (*see e.g.,* EP 331 376), *P. stutzeri* (*see e.g.,* GB 1,372,034), *P. fluorescens, Pseudomonas* sp. strain SD 705 (*see e.g.,* WO 95/06720 and WO 96/27002), *P. wisconsinensis* (*see e.g.* WO 96/12012); a *Bacillus* lipase *(e.g.* from *B. subtilis; see e.g.,* Dartois et al. Biochemica et Biophysica Acta, 1131: 253-360 (1993)), *B. stearothermophilus* (*see e.g.,* JP 64/744992), or *B. pumilus* (*see e.g.,* WO 91/16422). Additional lipase variants contemplated for use in the formulations include those described for example in: WO 92/05249, WO 94/01541, WO 95/35381, WO 96/00292, WO 95/30744, WO 94/25578, WO 95/14783, WO 95/22615, WO 97/04079, WO 97/07202, EP 407225, and EP 260105. Some commercially available lipase enzymes include Lipolase® and Lipolase Ultra™ (Novo Nordisk A/S).

Polyesterases: suitable polyesterases can be included in composition. Suitable polyesterases include for example those described in WO 01/34899 and WO 01/14629.

Amylases: The compositions can be combined with other α-amylases, such as non-production enhanced α-amylase. These can include commercially available amylases, such as but not limited to Duramyl®, Termamyl®, Fungamyl® and BAN™ (Novo Nordisk A/S); Rapidase®, and Purastar® (from Genencor International, Inc.).

Cellulases: Cellulases can be added to the compositions. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.* the fungal cellulases produced from *Humicola insolens, Myceliophthora thermophila* and *Fusarium oxysporum* disclosed for example in U.S. Patent Nos. 4,435,307; 5,648,263; 5,691,178; and 5,776,757; and International PCT No. WO 89/09259. Exemplary cellulases contemplated for use are those having color care benefit for the textile. Examples of such cellulases are cellulases described in, for example, EP 0495257, EP 0531372, WO 96/11262, WO 96/29397, and WO 98/08940. Other examples are cellulase variants, such as those described in WO 94/07998; WO 98/12307; WO 95/24471; PCT/DK98/00299; EP 531315; U.S. Patent Nos. 5,457,046; 5,686,593; and 5,763,254. Commercially available cellulases include Celluzyme® and Carezyme® (Novo Nordisk A/S); Clazinase™ and Puradax HA® (Genencor International, Inc.); and KAC-500(B)™ (Kao Corporation).

Peroxidases/Oxidases: Suitable peroxidases/oxidases contemplated for use in the compositions include those of plant, bacterial or fungal origin. Chemically modified or protein engineered mutants are included. Exemplary peroxidases include peroxidases from *Coprinus, e.g.* from *C. cinereus,* and variants thereof as those described in WO 93/24618, WO 95/10602, and WO 98/15257. Commercially available peroxidases include for example Guardzyme™ (Novo Nordisk A/S).

The detergent enzyme(s) may be included in a detergent composition by adding separate additives containing one or more enzymes, or by adding a combined additive comprising all of these enzymes. A detergent additive, *i.e.* a separate additive or a combined additive, can be formulated *e.g.*, as a granulate, a liquid, a slurry, *etc.* Exemplary detergent additive formulations are granulates, in particular non-dusting granulates, liquids, in particular stabilized liquids, or slurries.

Non-dusting granulates may be produced, *e.g.*, as disclosed in U.S. Patent Nos. 4,106,991 and 4,661,452, and may optionally be coated by methods known in the art. Examples of waxy coating materials are poly(ethylene oxide) products (*e.g.*, polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may be stabilized for example, by adding a polyol such as propylene glycol, a sugar or sugar alcohol, lactic acid or boric acid according to established methods. Protected enzymes may be prepared according to the method disclosed in EP 238216.

Generally, the detergent composition may be in any convenient form, *e.g.*, a bar, a tablet, a powder, a granule, a paste, or a liquid. A liquid detergent may be aqueous, typically containing up to about 70% water, and 0% to about 30% organic solvent. Compact detergent gels contained for example about 30% water or less.

The detergent composition comprises one or more surfactants, which may be non-ionic including semi-polar and/or anionic and/or cationic and/or zwitterionic. The surfactants are typically present in the wide range from about 0.1% to 60% by weight.

When included therein, the detergent will usually contain from about 1% to about 40% of an anionic surfactant, such as linear alkylbenzenesulfonate, α-olefinsulfonate, alkyl sulfate (fatty alcohol sulfate), alcohol ethoxysulfate, secondary alkanesulfonate, α-sulfo fatty acid methyl ester, alkyl- or alkenylsuccinic acid, or soap.

When included therein, the detergent will typically contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (e.g., SKS-6 from Hoechst).

The detergent may comprise one or more polymers. Examples are carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates e.g., polyacrylates, maleic/acrylic acid copolymers), and lauryl methacrylate/acrylic acid copolymers.

The detergent may contain a bleaching system that may comprise a $H_2O_2$ source, such as perborate or percarbonate, which may be combined with a peracid-forming bleach activator (*e.g.*, tetraacetylethylenediamine or nonanoyloxybenzenesulfonate). Alternatively, the bleaching system may comprise peroxyacids (*e.g.* the amide, imide, or sulfone type peroxyacids). The bleaching system can also be an enzymatic bleaching system.

The enzyme(s) of the detergent composition may be stabilized using conventional stabilizing agents, *e.g.*, as polyol *(e.g.,* propylene glycol or glycerol), a sugar or sugar alcohol, lactic acid, boric acid, or a boric acid derivative (*e.g.*, an aromatic borate ester), or a phenyl boronic acid derivative (*e.g.*, 4-formylphenyl boronic acid). The composition may be formulated as described in WO 92/19709 and WO 92/19708.

The detergent may also contain other conventional detergent ingredients such as *e.g.*, fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions, in particular a *Bacillus* sp. no. 707 α-amylase or variant thereof, may be added in an amount corresponding to about 0.01 to about 100 mg of enzyme protein per liter of wash liquor, for example about 0.05 to about 5.0 mg of enzyme protein per liter of wash liquor, or about 0.1 to about 1.0 mg of enzyme protein per liter of wash liquor.

## 7. Methods

### 7.1 Filter Screening Assays

The assays discussed below may be used in the screening of Amy707 alpha-amylase variants having altered stability at high or low pH and/or under $Ca^{2+}$ depleted conditions compared to the parent alpha-amylase enzyme.

### 7.2 High pH Filter Assay

Bacillus libraries are plated on a sandwich of cellulose acetate (OE 67, Schleicher & Schuell, Dassel, Germany)-- and nitrocellulose filters (Protran-Ba 85, Schleicher & Schuell, Dassel, Germany) on TY agar plates with 10 micro g/ml kanamycin at 37°C for at least 21 hours. The cellulose acetate layer is located on the TY agar plate.

Each filter sandwich is specifically marked with a needle after plating, but before incubation in order to be able to localize positive variants on the filter and the nitrocellulose filter with bound variants is transferred to a container with glycin-NaOH buffer, pH 8.6-10.6 and incubated at room temperature (can be altered from 10-60°C) for 15 min. The cellulose acetate filters with colonies are stored on the TY-plates at room temperature until use. After incubation, residual activity is detected on plates containing 1% agarose, 0.2% starch in glycin-NaOH buffer, pH 8.6-10.6. The assay plates with nitrocellulose filters are marked the same way as the filter sandwich and incubated for 2 hours at room temperature. After removal of the filters the assay plates are stained with 10% Lugol solution. Starch degrading variants are detected as white spots on dark blue background and then identified on the storage plates. Positive variants are rescreened twice under the same conditions as the first screen.

### 7.3 Low Calcium Filter Assay

Bacillus libraries are plated on a sandwich of cellulose acetate (OE 67, Schleicher & Schuell, Dassel, Germany)-- and nitrocellulose filters (Protran-Ba 85, Schleicher & Schuell, Dassel, Germany) on TY agar plates with a relevant antibiotic, e.g., kanamycin or chloramphenicol, at 37°C for at least 21 hours. The cellulose-acetate layer is located on the TY agar plate.

Each filter sandwich is specifically marked with a needle after plating, but before incubation in order to be able to localize positive variants on the filter and the nitrocellulose filter with bound variants is transferred to a container with carbonate/bicarbonate buffer pH 8.5-10 and with different EDTA concentrations (0.001 mM-100 mM). The filters are incubated at room temperature for 1 hour. The cellulose acetate filters with colonies are stored on the TY-plates at room temperature until use. After incubation, residual activity is detected on plates containing 1% agarose, 0.2% starch in carbonate/bicarbonate buffer pH 8.5-10. The assay plates with nitrocellulose filters are marked the same way as the filter sandwich and incubated for 2 hours at room temperature. After removal of the filters the assay plates are stained with 10% Lugol solution. Starch degrading variants are detected as white spots on dark blue background and then identified on the storage plates. Positive variants are rescreened twice under the same conditions as the first screen.

### 7.4 Low pH Filter Assay

*Bacillus* libraries are plated on a sandwich of cellulose acetate (OE 67, Schleicher & Schuell, Dassel, Germany)-- and nitrocellulose filters (Protran-Ba 85, Schleicher & Schuell, Dasseli Germany) on TY agar plates with 10 micro g/ml chloramphenicol at 37°C for at least 21 hours. The cellulose acetate layer is located on the TY agar plate.

Each filter sandwich is specifically marked with a needle after plating, but before incubation in order to be able to localize positive variants on the filter, and the nitrocellulose filter with bound variants is transferred to a container with citrate buffer, pH 4.5 and incubated at 80°C for 20 minutes (when screening for variants in the wild type backbone) or 85°C for 60 minutes (when screening for variants of the parent alpha-amylase). The cellulose acetate filters with colonies are stored on the TY-plates at room temperature until use. After incubation, residual activity is detected on assay plates containing 1% agarose, 0.2% starch in citrate buffer, pH 6.0. The assay plates with nitrocellulose filters are marked the same way as the filter sandwich and incubated for 2 hours at 50°C. After removal of the filters the assay plates are stained with 10% Lugol solution. Starch degrading variants are detected as white spots on dark blue background and then identified on the storage plates. Positive variants are re-screened twice under the same conditions as the first screen.

### 7.5 Secondary Screening

Positive transformants after rescreening are picked from the storage plate and tested in a secondary plate assay. Positive transformants are grown for 22 hours at 37°C in 5 ml LB+chloramphenicol. The Bacillus culture of each positive transformant and as a control a clone expressing the corresponding backbone are incubated in citrate buffer, pH 4.5 at 90°C and samples are taken at 0, 10, 20, 30, 40, 60 and 80 minutes. A 3 micro liter sample is spotted on an assay plate. The assay plate is stained with 10% Lugol solution. Improved variants are seen as variants with higher residual activity (detected as halos on the assay plate) than the backbone. The improved variants are determined by nucleotide sequencing.

### 7.6 Stability Assay of Unpurified Variants

The stability of the variants may be assayed as follows: *Bacillus* cultures expressing the variants to be analyzed are grown for 21 hours at 37°C in 10 ml LB+chloramphenicol. 800 micro liter culture is mixed with 200 microliters citrate buffer, pH 4.5. A number of 70 microliter aliquots corresponding to the number of sample time points are made in PCR tubes and incubated at 70°C or 90°C for various time points (typically 5, 10, 15, 20, 25 and 30 minutes) in a PCR machine. The 0 min sample is not incubated at high temperature. Activity in the sample is measured by transferring 20 microliter to 200 microliter of the alpha-amylase PNP-$G_7$ substrate MPR3 ((Boehringer Mannheim Cat. no. 1660730) as described below under "Assays for Alpha-Amylase Activity". Results are plotted as percentage activity (relative to the 0 time point) versus time, or stated as percentage residual activity after incubation for a certain period of time.

### 7.7 Fermentation and Purification of Alpha-Amylase Variants

A *B. subtilis* strain harboring the relevant expression plasmid may be fermented and purified as follows: The strain is streaked on a LB-agar plate with 10 micro g/ml kanamycin from -80°C stock, and grown overnight at 37°C. The colonies are transferred to 100 ml PS-1 media supplemented with 10 micro g/ml chloramphinicol in a 500 ml shaking flask.

| Composition of PS-1 medium | |
|---|---|
| Pearl sugar | 100 g/l |
| Soy Bean Meal | 40 g/l |
| $Na_2HPO_4$, 12 $H_2O$ | 10 g/l |
| Pluronic ™ PE 6100 | 0.1 g/l |
| $CaCO_3$ | 5 g/l |

The culture is shaken at 37°C at 270 rpm for 5 days.

Cells and cell debris are removed from the fermentation broth by centrifugation at 4500 rpm in 20-25 minutes. Afterwards the supernatant is filtered to obtain a completely clear solution. The filtrate is concentrated and washed on a UF-filter (10000 cut off membrane) and the buffer is changed to 20 mM Acetate pH 5.5. The UF-filtrate is applied on a S-sepharose F.F. and elution is carried out by step elution with 0.2M NaCl in the same buffer. The eluate is dialysed against 10 mM Tris, pH 9.0 and applied on a Q-sepharose F.F. and eluted with a linear gradient from 0-0.3M NaCl over 6 column volumes. The fractions that contain the activity (measured by the Phadebas assay) are pooled, pH was adjusted to pH 7.5 and remaining color was removed by a treatment with 0.5% W/vol. active charcoal in 5 minutes.

### 7.8 Specific Activity Determination

The specific activity is determined using the Phadebas® assay (Pharmacia) as activity/mg enzyme. The manufacturers instructions are followed (see also below under "Assay for Alpha-Amylase Activity").

### 7.9 Determination of Isoelectric Point

The pI is determined by isoelectric focusing (ex: Pharmacia, Ampholine, pH 3.5-9.3).

### 7.10 Accelerated Stability Assay

In 50 ml Propylene tubes, 10 ml of detergent of interest was added. Appropriate dilution was made to both Amy707t and Amy707tΔRS so that 180 ppm of each was measured with a pippette into separate tubes containing the detergent. The detergent with each mutant enzyme was vortex for 30 sec and then placed on a RotaMix (ATR RKVS Model) for 10 minutes. 100 micro-liters of the detergent with the mutant enzyme were measured with a pipette and diluted 1:651. The initial activity of the mutants was assayed using Blocked P-Nitro-Phenyl-Maltoheptaose (Blocked PBNPG7) substrate on a Konelab, Model 20XT. The detergent samples were then incubated in a constant temperature incubator set at 37°C. Samples were removed at 1, 2, 4, 7 and 17 days and the enzyme activity assayed.

**7.11 Assays for Alpha-Amylase Activity**

7.11.1 Phadebas Assay

Alpha-amylase activity is determined by a method employing Phadebas® tablets as substrate. Phadebas tablets (Phadebas® Amylase Test, supplied by Pharmacia Diagnostic) contain a cross-linked insoluble blue-colored starch polymer, which has been mixed with bovine serum albumin and a buffer substance and tabletted.

For every single measurement one tablet is suspended in a tube containing 5 ml 50 mM Britton-Robinson buffer (50 mM acetic acid, 50 mM phosphoric add, 50 mM boric acid, 0.1 mM $CaCl_2$, pH adjusted to the value of interest with NaOH). The test is performed in a water bath at the temperature of interest. The alpha-amylase to be tested is diluted in x ml of 50 mM Britton-Robinson buffer. 1 ml of this alpha-amylase solution is added to the 5 ml 50 mM Britton-Robinson buffer. The starch is hydrolyzed by the alpha-amylase giving soluble blue fragments. The absorbance of the resulting blue solution, measured spectrophotometrically at 620 nm, is a function of the alpha-amylase activity.

It is important that the measured 620 nm absorbance after 10 or 15 minutes of incubation (testing time) is in the range of 0.2 to 2.0 absorbance units at 620 nm. In this absorbance range there is linearity between activity and absorbance (Lambert-Beer law). The dilution of the enzyme must therefore be adjusted to fit this criterion. Under a specified set of conditions (temp., pH, reaction time, buffer conditions) 1 mg of a given alpha-amylase will hydrolyze a certain amount of substrate and a blue color will be produced. The color intensity is measured at 620 nm. The measured absorbance is directly proportional to the specific activity (activity/mg of pure alpha-amylase protein) of the alpha-amylase in question under the given set of conditions.

7.11.2 Alternative Method

Alpha-amylase activity is determined by a method employing the PNP-$G_7$ substrate. PNP-$G_7$ which is a abbreviation for p-nitrophenyl-alpha-D-maltoheptaoside is a blocked oligosaccharide which can be cleaved by an endo-amylase. Following the cleavage, the alpha-glucosidase included in the kit digest the substrate to liberate a free PNP molecule which has a yellow color and thus can be measured by visible spectophometry at $\lambda$=405 nm (400-420 nm). Kits containing PNP-$G_7$ substrate and alpha-glucosidase are manufactured by Boehringer-Mannheim (cat. No.1054635).

To prepare the reagent solution 10 ml of substrate/buffer solution is added to 50 ml enzyme/buffer solution as recommended by the manufacturer. The assay is performed by transferring a 20 microliter sample to a 96 well microtitre plate and incubating at 25°C. 200 microliter reagent solution pre-equilibrated to 25°C is added. The solution is mixed and preincubated 1 minute and absorption is measured every 30 seconds over 4 minutes at OD 405 nm in an ELISA reader.

The slope of the time dependent absorption-curve is directly proportional to the activity of the alpha-amylase in question under the given set of conditions.

**7.12 Determination of enzyme Performance in Detergent Compositions**

7.12.1 US Conditions

Use of Terg-o-tometer, United States Testing, Hoboken, N.J.- To simulate washing test under US washing conditions, a dose efficiency curve (DEC) of the mutant enzyme of interest was conducted at 20°C using standard detergents such as Liquid AATCC 2003 Without Optical Brightener and/or Powder AATCC 1993 (American Association of Textile Chemists and Colorists). A corresponding DEC of a comparative alpha-amylase was then conducted to compare the stain removal performance of the inventive mutant enzyme. This process was repeated at 40°C. Typically, 4 swatches of CS-28 Rice Starch stain (CFT of Holland) were placed in a steel container of the Terg-o-tometer, which was filled with 1 Liter of DI water and 1.5 g of Liquid AATCC. When Powder AATCC was used, 1.5g of the detergent powder was weighed out on an analytical balance (Model PM4800, Mettler Instrument Corp., Highstown, N.J. 08520 and added to the Terg-o-tometer. Two replicates were run at the same time. Unless otherwise stated, the tests were carried out for 12 minutes and rinsed for 3 minutes. After washing, the swatches were air-dried and the reflectance of the test swatches was measured with a Chroma Meter Model CR-410 manufactured by Konica Minolta. The data collected were treated with appropriate statistical analysis.

7.12.2 European Conditions

Use of Launder-O-meter, manufactured by Atlas Company, Atlanta, Georgia - To simulate the washing test under European washing conditions, a dose efficiency curve (DEC) of the mutant enzyme of interest was conducted at 40°C using standard European testing detergents, IEC A and IEC A with Bleach ( TAED-Tetra-Acetyl-ethylene-diamine acetate) and Sodium Perborate. A corresponding DEC curve of a comparative mutant enzyme was then conducted to compare the stain removal performance of the inventive mutant enzyme. This process was repeated at higher wash temperature if desirable. Typically, 4 swatches of EMPA 161, Maize starch (EMPA, Switzerland) were placed in a steel container with 250 ml of DI water containing 6.8g/L of the IEC A detergent or 8.0g/L of the IEC A with Bleach detergent. Two replicates were run at the same time. Unless otherwise stated the tests were carried out for 45 minutes and rinsed for 5 minutes. After washing, the swatches were air-dried and the reflectance of the test swatches was measured with a Chroma Meter Model CR-410. The data collected were treated with appropriate statistical analysis.

7.12.3 Microswatch Method of Assessing Detergent Compositions

Numerous α-amylase cleaning assays exist. Exemplary description of testing cleaning includes the following.

A "swatch" is a piece of material such as a fabric that has a stain applied thereto. The material can be, for example, fabrics made of cotton, polyester or mixtures of natural and synthetic fibers. The swatch can further be paper, such as filter paper or nitrocellulose, or a piece of a hard material such as ceramic, metal, or glass. For amylases, the stain is starch based, but can include blood, milk, ink, grass, tea, wine, spinach, gravy, chocolate, egg, cheese, clay, pigment, oil, or mixtures of these compounds.

A "smaller swatch" is a section of the swatch that has been cut with a single-hole punch device, or has been cut with a custom manufactured 96-hole punch device, where the pattern of the multi-hole punch is matched to standard 96-well microtiter plates, or the section has been otherwise removed from the swatch. The swatch can be of textile, paper, metal, or other suitable material. The smaller swatch can have the stain affixed either before or after it is placed into the well of a 24-, 48- or 96-well microtiter plate. The "smaller swatch" can also be made by applying a stain to a small piece of material. For example, the smaller swatch can be a stained piece of fabric 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived of to deliver simultaneously swatches to any format plate, including but not limited to 24-well, 48-well, and 96-well plates. In another conceivable method, the soiled test platform can be a bead made of either metal, plastic, glass, ceramic, or other suitable material that is coated with the soil substrate for use in testing cleaning compositions for materials other than textiles. The one or more coated beads are then placed into wells of 96-, 48-, or 24- well plates or larger formats, containing suitable buffer and enzyme. In this case, supernatant can be examined for released soil either by direct absorbance measurement or after a secondary color development reaction. Analysis of the released soil might also be taken by mass spectral analysis. A further microscreening assay can be to deliver and secure a swatch, for example an indigo dyed denim, to a well of a multi-well plate, and add particles such as sand or larger particles such as for example garnet sieved to include particle 6 to 8, or 9 gauge, and agitate the plate so as to cause abrasion of the swatch by the added particles. This assay has found use in the assessment of cellulases in stone washing applications. The effectiveness of the enzyme can be judged by either color release (*e.g.*, released indigo is dissolved in dimethylsulfoxide and absorbance at $A_{600}$ nm is measured) to the reaction buffer or by reflectance measurements of the abraded swatch.

When, for example, untreated BMI (blood/milk/ink) swatches are washed in detergent without bleach, a large portion of the ink is released even without the help of a protease. Adding a protease leads to a small increase in ink release, which can be hard to quantify over the large background. One aspect provides a treatment protocol that allows one to control the degree of fixation of a stain. As a result, it is possible to produce swatches that, for example, release varying amounts of stain when washed in the absence of the enzyme being tested. The use of fixed swatches leads to a dramatic improvement of the signal-to-noise ratio in the wash assays. Furthermore, by varying the degree of fixation, one can generate stains that give optimum results under the various cleaning conditions.

Swatches having stains of known "strength" on various types of material are commercially available (EMPA, St. Gallen, Switzerland; wfk--Testgewebe GmbH, Krefeld Germany; or Center for Test Materials, Vlaardingen, The Netherlands) and/or can be made by the practitioner (Morris and Prato, Textile Research Journal 52(4): 280 286 (1982)). Other test swatches include but are not limited to blood/milk/ink (BMI) stain(s) on a cotton-containing fabric, a spinach stain on a cotton-containing fabric, or grass on a cotton-containing fabric, and chocolate/milk/soot on a cotton-containing fabric.

A BMI stain can be fixed to cotton with 0.0003% to 0.3% hydrogen peroxide. Other combinations include grass or spinach fixed with 0.001% to 1% glutaraldehyde, gelatin and Coomassie Brilliant Blue stain fixed with 0.001% to 1% glutaraldehyde, or chocolate, milk and soot fixed with 0.001% to 1% glutaraldehyde.

The swatch can also be agitated during incubation with the enzyme and/or detergent formulation. Wash performance data is dependent on the orientation of the swatches in the wells (horizontal versus vertical), particularly in the 96-well plate. This would indicate that mixing was insufficient during the incubation period. Although there are a number of ways to ensure sufficient agitation during incubation, a plate holder in which the microtiter plate is sandwiched between two plates of aluminum can be constructed. This can be as simple as placing, for example, an adhesive plate sealer over the wells then clamping the two aluminum plates to the 96-well plate with any type of appropriate, commercially available clamps. It can then be mounted in a commercial incubator shaker. Setting the shaker to about 400 rpm results in very efficient mixing, while leakage or cross-contamination is efficiently prevented by the holder.

Trinitrobenzenesulfonic acid (TNBS) can be used to quantify the concentration of amino groups in the wash liquor. This can serve as a measure of the amount of protein that was removed from the swatch (*see e.g.*, Cayot and Tainturier, Anal. Biochem. 249: 184-200 (1997)). However, if a detergent or an enzyme sample leads to the formation of unusually small peptide fragments (for example, from the presence of peptidases in the sample), then one will obtain a larger TNBS signal, *i.e.*, more "noise".

Another means of measuring wash performance of blood/milk/ink or other stain that is based on ink release. Proteolysis of protein on the swatches leads to the release of ink particles that can be quantified by measuring the absorbance of the wash liquor. The absorbance can be measured at any wavelength between 350 and 800 nm. The wavelength is measured at 410 nm or 620 nm. The wash liquor can also be examined to determine the wash performance on stains containing grass, spinach, gelatin or Coomassie Brilliant Blue stain. Exemplary wavelengths for these stains include and 670 nm for spinach or grass and 620 nm for gelatin or Coomassie Brilliant Blue. For example, an aliquot of the wash liquor (typically 100 to 150 μL from a 96-well microplate, for example) is removed and placed in a cuvette or multiwell microplate. This is then placed in a spectrophotometer and the absorbance is read at an appropriate wavelength.

The system can also be used to determine an enhanced enzyme and/or detergent composition for dish washing, for example, using a blood/milk/ink stain on a suitable substrate such as cloth, plastic or ceramic.

In one aspect, the a BMI stain is fixed to cotton by applying 0.3% hydrogen peroxide to the BMI/cotton swatch for 30 minutes at 25°C or by applying 0.03% hydrogen peroxide to the BMI/cotton swatch for 30 minutes at 60°C. Smaller swatches of approximately 0.25" are cut from the BMI/cotton swatch and placed in the wells of a 96-well microtiter plate. Into each well, a known mixture of a detergent composition and an enzyme such as a variant protein is placed. After placing an adhesive plate sealer onto the top of the microtiter plate, the microtiter plate is clamped to an aluminum plate and agitated on an orbital shaker at approximately 250 rpm for about 10 to 60 minutes. At the end of this time, the supernatants are transferred to wells in a new microtiter plate and the absorbance of the ink at 620 nm is measured. This can be similarly tested with spinach stains or grass stains fixed to cotton by applying 0.01% glutaraldehyde to the spinach/cotton swatch or grass/cotton swatch for 30 minutes at 25°C. The same can be done with chocolate, milk, and/or soot stains. Additional blood/milk/ink assays and conditions are provided in U.S. Patent No. 7,122,334 (Genencor International, Inc.).

### 7.13 Determination of LAS Sensitivity

The variant is incubated with different concentrations of LAS (linear alkyl benzene sulphonate; Nansa 1169/P) for 10 minutes at 40°C.

The residual activity is determined using the Phadebas® assay method or the alternative method employing the PNP-$G_7$ substrate.

LAS is diluted in 0.1 M phosphate buffer pH 7.5.

The following concentrations are used:

500 ppm, 250 ppm, 100 ppm, 50 ppm, 25 ppm, and 10 ppm or no LAS.

The variant is diluted in the different LAS buffers to concentration of 0.01-5 mg/l in a total volume of 10 ml and incubated for 10 minutes in a temperature controlled water bath. The incubation is stopped by transferring a small aliquot into cold assay buffer. It is important that during activity measurement the LAS concentration is below 1 ppm, in order not to affect the activity measurement.

Then the residual activity is determined in duplicate using the above mentioned Phadebas® assay or alternative method.

The activity is measured after subtraction of the blank.

The activity with no LAS is 100%.

### 8. <u>Biofilm Removal Compositions and Use</u>

The composition may comprise a *Bacillus* sp. no. 707 α-amylase or variant thereof, as the major enzymatic component, *e.g.*, a mono-component composition for use in removing biofilms. Alternatively, the composition may comprise multiple enzymatic activities, such as multiple amylases, or a cocktail of enzymes including any combination of the following: aminopeptidase, amylase (β-, or α-, or gluco-amylase), carbohydrase, carboxypeptidase, catalase, cellulase, chitinase, cutinase, cyclodextrin glycosyltransferase, deoxyribonuclease, esterase, α-galactosidase, β-galactosidase, glucoamylase, α-glucosidase, β-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, and/or xylanase, or any combination thereof for removing biofilms. The additional enzyme(s) may be producible by means of a microorganism belonging to the genera *Aspergillus*, *Trichoderma*, *Humicola* (*e.g., H. insolens*), and *Fusarium*. Exemplary members from the *Aspergillus* genus include *Aspergillus aculeatus*, *A. awamori*, *A. niger*, and *A. oryzae*. Exemplary members of the *Fusarium* genus include *F. bactridioides*, *F. cerealis*, *F. crookwellense*, *F. culmorum*, *F. graminearum*, *F. graminum*, *F. heterosporum*, *F. negundinis*, F. *oxysporum*, *F. reticulatum*, *F. roseum*, *F. sambucinum*, *F. sarcochroum*, *F. sulphureum*, *F. torulosum*, *F. trichothecioides*, and *F. venenatum*.

The *Bacillus* sp. no. 707 α-amylase or variant thereof, comprising compositions may be prepared in accordance with methods known in the art and may be in the form of a liquid or a dry composition. For instance, the *Bacillus* sp. no. 707 α-amylase or variant thereof, containing composition may be in the form of a granulate or a microgranulate. The polypeptide to be included in the composition may be stabilized in accordance with methods known in the art.

Examples are given below of exemplary uses of the polypeptide compositions. The dosage of the *Bacillus* sp. no. 707 α-amylase or variant thereof, containing composition and other conditions under which the composition is used may be determined using methods known in the art.

The *Bacillus* sp. no. 707 α-amylases or variants thereof, are further contemplated for use in a composition along with a 2,6-β-D-fructan hydrolase or variant thereof.

Another aspect contemplates compositions and methods for disintegrating and/or removing biofilms. The term "disintegration" as used herein is to be understood as hydrolysis of polysaccharides in a biofilm matrix connecting and binding together individual microbial cells in the biofilm, whereby the microbial cells can be released and removed from the biofilm. The biofilm is typically present at a surface and the disintegration of the biofilm can be achieved by bringing the surface in contact, *e.g.*, by immersing, covering or splashing the surface with an aqueous medium comprising a *Bacillus* sp. no. 707 α-amylase or variant thereof, or one or more other enzymes responsible for breaking down biofilms, such as but not limited to 2,6-β-D-fructan hydrolase. The composition can be used to hydrolyse slime, *e.g.*, in white waters in the pulping and paper industry.

The *Bacillus* sp. no. 707 α-amylases or variants thereof, may be present in the amount of 0.0001 to 10000 mg/L; 0.001-1000 mg/L; 0.01-100 mg/L; or 0.1-10 mg/L. Additional enzymes and enzyme variants may be present in similar amounts or less.

The process may suitably be performed at temperatures from about ambient temperature to about 70°C. Exemplary temperature ranges include from about 30°C to about 60°C, *e.g.*, about 40°C to about 50°C.

A suitable pH for the hydrolyzing biofilms lies within from about 3.5 to about 8.5. Exemplary pH ranges include from about 5.5 to about 8, *e.g.* from about 6.5 to about 7.5. The contact time or reaction time for the enzyme to effectively removing a biofilm may vary considerably, depending on the biofilm properties and the frequency of which a surface is treated with the enzyme alone or in combination with other biofilm degrading enzymes, such as 2,6-β-D-fructan hydrolase. Exemplary reaction time can include within about 0.25 to about 25 hours, and from about 1 to about 10 hours, *e.g.* about 2 hours.

Additional biofilm degrading enzymes that can be combined with the *Bacillus* sp. no. 707 α-amylase or variants thereof, and 2,6-β-D-fructan hydrolases include but are not limited to cellulases, hemicellulases, xylanases, other amylases including other α-amylases, lipases, proteases, and/or pectinases.

The *Bacillus* sp. no. 707 α-amylase or variants thereof, can further be combined with antimicrobial agents such as enzymatic or non-enzymatic biocides. An enzymatic biocide may, *e.g.*, be a composition comprising an oxidoreductase, *e.g.* a laccase or a peroxidase, especially haloperoxidase, and optionally an enhancing agent, such as an alkyl syringate, as described for example in International PCT applications WO 97/42825 and DK 97/1273.

The surface from which a biofilm for example can be removed and/or cleaned off is a hard surface, which by definition relates to any surface that is essentially non-permeable to microorganisms. Examples of surfaces are surfaces made from metal, *e.g.* stainless steel alloys, plastics/synthetic polymers, rubber, board, glass, wood, paper, textile, concrete, rock, marble, gypsum and ceramic materials which optionally may be coated, *e.g.* with paint, enamel, polymers and the like. Accordingly, the surface may be a member of a system holding, transporting, processing, or in contact with aqueous solutions such as water supply systems, food processing systems, cooling systems, chemical processing systems or pharmaceutical processing systems. The compositions and methods of using the compositions for removing biofilm in the wood processing industry, such as the pulp and/or paper industry. Accordingly, the enzyme and compositions containing the enzyme are useful in a conventional cleaning-in-place (C-I-P) system. The surface may a member of a system unit such as pipes, tanks, pumps, membranes, filters, heat exchangers, centrifuges, evaporators, mixers, spray towers, valves and reactors. The surface may also be or be a part of utensils used in the medical science and industry such as contaminated endoscopes, prosthetic devices or medical implants.

The compositions for biofilm removal is also contemplated for preventing so-called bio-corrosion occurring when a metal surface, *e.g.* a pipeline, is attacked by a microbial biofilm, that is by disintegrating the biofilm thereby preventing the microbial cells of the biofilm from creating a biofilm environment, which corrodes the metal surface to which it is attached.

Another application for anti-biofilm compositions is for oral care. The surface may however also be of biological origin, such as mucous membranes, skin, teeth, hair, nails *etc.*

Teeth with dental plaque, *e.g.*, by incorporating the enzymes into toothpaste, and contaminated contact lenses are encompassed as surfaces. Accordingly, a *Bacillus* sp. no. 707 $\alpha$-amylase or variants thereof, can be used for compositions and processes for making a medicament for disintegration of plaque present on a human or animal tooth. A further use is disintegration of biofilm from mucous membranes, such as biofilm in lungs in patients suffering from cystic fibrosis.

Accordingly, in a still further aspect relates to an oral care composition comprising a recombinant enzyme, such as a purified enzyme that is essentially free of any active contaminants. An oral care composition may suitably comprise an amount of a recombinant enzyme.

Other biofilm degrading enzymes for use in oral care compositions include but are not limited to 2,6-$\beta$-D-fructan hydrolase activity in the oral care composition. Contemplated enzyme activities include activities from the group of enzymes comprising dextranase; mutanases; oxidases, such as glucose oxidase, L-amino acid oxidase, peroxidases, such as *e.g.* the *Coprinus* sp. peroxidases described in WO 95/10602, or lactoperoxidase, haloperoxidases, especially haloperoxidase derivable from *Curvularia* sp., in particular *C. verruculosa* and *C. inaequalis*; laccases; proteases such as papain, acidic protease (*e.g.* the acidic proteases described in WO 95/02044, endoglucosidases, lipases, amylases, including amyloglucosidases, such as AMG (Novo Nordisk A/S); anti-microbial enzymes, and mixtures thereof.

The oral care composition may have any suitable physical form (*i.e.* powder, paste, gel, liquid, ointment, tablet *etc.*). An "oral care composition" includes a composition, which can be used for maintaining or improving the oral hygiene in the mouth of humans and animals, by preventing dental caries, preventing the formation of dental plaque and tartar, removing dental plaque and tartar, preventing and/or treating dental diseases *etc.* At least in the context oral care compositions do also encompass products for cleaning dentures, artificial teeth and the like. Examples of such oral care compositions includes toothpaste, dental cream, gel or tooth powder, odontic mouth washes, pre- or post brushing rinse formulations, chewing gum, lozenges, and candy. Toothpastes and tooth gels typically include abrasive polishing materials, foaming agents, flavoring agents, humectants, binders, thickeners, sweetening agents, whitening/bleaching/stain removing agents, water, and optionally additional enzymes and enzyme combinations.

Mouthwashes, including plaque-removing liquids, typically comprise a water/alcohol solution, flavor, humectant, sweetener, foaming agent, colorant, and optionally additional enzymes or enzyme combinations.

Abrasive polishing material might also be incorporated into the oral care composition such as a dentifrice.

Accordingly, abrasive polishing material can include alumina and hydrates thereof, such as alpha alumina trihydrate; magnesium trisilicate; magnesium carbonate; kaolin; aluminosilicates, such as calcined aluminum silicate and aluminum silicate; calcium carbonate; zirconium silicate; and also powdered plastics, such as polyvinyl chloride; polyamides; polymethyl methacrylate; polystyrene; phenol-formaldehyde resins; melamine-formaldehyde resins; urea-formaldehyde resins; epoxy resins; powdered polyethylene; silica xerogels; hydrogels and aerogels and the like. Also suitable as abrasive agents are calcium pyrophosphate; water-insoluble alkali metaphosphates; dicalcium phosphate and/or its dihydrate, dicalcium orthophosphate; tricalcium phosphate; particulate hydroxyapatite and the like. It is also possible to employ mixtures of these substances.

Dependent on the oral care composition, the abrasive product may be present in from about 0% to about 70% by weight, or from about 1% to about 70%. For toothpastes, the abrasive material content typically lies in the range of from 10% to 70% by weight of the final toothpaste.

Humectants are employed to prevent loss of water from e.g. tooth pastes. Suitable humectants for use in oral care compositions include the following compounds and mixtures thereof: glycerol; polyol; sorbitol; polyethylene glycols (PEG); propylene glycol; 1,3-propanediol; 1,4-butanediol; hydrogenated partially hydrolyzed polysaccharides and the like. Humectants are in general present in from 0% to about 80%, or from about 5% to about 70% by weight in toothpaste.

Silica, starch, tragacanth gum, xanthan gum, extracts of Irish moss, alginates, pectin, cellulose derivatives, such as hydroxyethyl cellulose, sodium carboxymethyl cellulose and hydroxypropyl cellulose, polyacrylic acid and its salts, polyvinylpyrrolidone, can be mentioned as examples of suitable thickeners and binders, which helps stabilizing a dentifrice product. Thickeners may be present in toothpaste creams and gels in an amount of from about 0.1% to about 20% by weight, and binders to the extent of from about 0.01 to about 10% by weight of the final product.

As foaming agent soap, anionic, cationic, non-ionic, amphoteric and/or zwitterionic surfactants can be used. These may be present at levels of from 0% to about 15%, from about 0.1% to about 13%, or from about 0.25% to about 10% by weight of the final product.

Surfactants are only suitable to the extent that they do not exert an inactivation effect on the *Bacillus* sp. no.

707 α-amylase or variants thereof. Surfactants include fatty alcohol sulfates, salts of sulfonated mono-glycerides or fatty acids having 10 to 20 carbon atoms, fatty acid-albumen condensation products, salts of fatty acids amides and taurines and/or salts of fatty acid esters of isethionic acid.

Suitable sweeteners include saccharin for use in the formulations.

Flavors, such as spearmint, are usually present in low amounts, such as from about 0.01% to about 5% by weight, especially from about 0.1% to about 5%. Whitening/bleaching agents include $H_2O_2$ and may be added in amounts less that about 5%, or from about 0.25% to about 4%, calculated by the weight of the final product. The whitening/bleaching agents may be an enzyme, such as an oxidoreductase. Examples of suitable teeth bleaching enzymes, such as those described in WO 97/06775.

Water is usually added in an amount giving e.g. toothpaste a flowable form.

Further water-soluble anti-bacterial agents, such as chlorohexidine digluconate, hexetidine, alexidine, Triclosan®, quaternary ammonium anti-bacterial compounds and water-soluble sources of certain metal ions such as zinc, copper, silver and stannous (*e.g.*, zinc, copper and stannous chloride, and silver nitrate) may also be included.

Also contemplated is the addition of compounds that can be used as fluoride source, dyes/colorants, preservatives, vitamins, pH-adjusting agents, anti-caries agents, desensitizing agents, *etc.*

Biofilm degrading enzymes provide several benefits when used for cleansing of the oral cavity. Proteases break down salivary proteins, which are adsorbed onto the tooth surface and form the pellicle, the first layer of resulting plaque. Proteases along with lipases destroy bacteria by lysing proteins and lipids, which form the structural components of bacterial cell walls and membranes.

Dextranase and other carbohydrases, such as the 2,6-β-D-fructan hydrolase, break down the organic skeletal structure produced by bacteria that forms a matrix for bacterial adhesion. Proteases and amylases, not only prevent plaque formation, but also prevent the development of calculus by breaking-up the carbohydrate-protein complex that binds calcium, preventing mineralization.

A toothpaste may typically comprise the following ingredients (in weight % of the final toothpaste composition): abrasive material to about 70%; humectant: 0% to about 80%; thickener: about 0.1% to about 20%; binder: about 0.01% to about 10%; sweetener: about 0.1% to about 5%; foaming agent: 0% to about 15%; whitener: 0% to about 5%; and enzymes: about 0.0001% to about 20%.

In a specific embodiment, a toothpaste has a pH in the range from about 6.0 to about 8.0, and comprises: a) about 10% to about 70% abrasive material; b) 0% to about 80% humectant; c) 0.1% to about 20% thickener; d) 0.01% to about 10% binder; e) about 0.1% to about 5% sweetener; f) 0% to about 15% foaming agent; g) 0% to about 5% whitener; i) about 0.0001% to about 20% enzymes.

Said enzymes referred to under i) include a *Bacillus* sp. no. 707 α-amylase or variants thereof, alone, or in combination with other biofilm degrading enzymes, such as 2,6-β-D-fructan hydrolase, and optionally other types of enzymes mentioned above known to be used in toothpastes and the like.

A mouth wash may typically comprise the following ingredients (in weight % of the final mouth wash composition): 0% to about 20% humectant; 0% to about 2% surfactant; 0% to about 5% enzymes; 0% to about 20% ethanol; 0% to about 2% other ingredients (*e.g.* flavor, sweetener active ingredients such as fluorides). The composition can also contain from about 0% to about 70% water.

The mouth wash composition may be buffered with an appropriate buffer e.g. sodium citrate or phosphate in the pH-range of about 6.0 to about 7.5. The mouth wash may be in non-diluted form (i.e. must be diluted before use).

The oral care compositions may be produced using any conventional method known to the art of oral care.

### 9. Starch Processing Compositions and Use

In another aspect, compositions with a disclosed *Bacillus* sp. no. 707 α-amylase or variants thereof, can be utilized for starch liquefaction or saccharification.

One aspect contemplates compositions and uses of compositions to produce sweeteners from starch. A "traditional" process for conversion of starch to fructose syrups normally consists of three consecutive enzymatic processes, viz. a liquefaction process followed by a saccharification process, and an isomerization process. During the liquefaction process, starch is degraded to dextrins by a *Bacillus* sp. no. 707 α-amylase or variants thereof, at pH values between about 5.5 and about 6.2 and at temperatures of about 95°C to about 160°C for a period of approximately 2 hours. In order to ensure optimal enzyme stability under these conditions, 1 mM of calcium is added (40 ppm free calcium ions). Starch processing is useful for producing alcohol (*e.g.*, cereal liquefaction for fuel and potable alcohol, alcohol brewing), starch liquefaction for sweetener production, cane sugar processing, and other food related starch processing goals. Other conditions can be used for different *Bacillus* sp. no. 707 α-amylases or variants thereof.

After the liquefaction process, the dextrins are converted into dextrose by addition of a glucoamylase (*e.g.* AMG™) and a debranching enzyme, such as an isoamylase or a pullulanase (*e.g.*, Promozyme®). Before this step, the pH is reduced to a value below about 4.5, maintaining the high temperature (above 95°C), and the liquefying *Bacillus* sp. no. 707 α-amylase or variant thereof, activity is denatured. The temperature is lowered to 60°C, and a glucoamylase and a debranching enzyme can be added. The saccharification process proceeds typically for about 24 to about 72 hours.

After the saccharification process, the pH is increased to a value in the range of about 6.0 to about 8.0, *e.g.*, pH 7.5, and the calcium is removed by ion exchange. The dextrose syrup is then converted into high fructose syrup using, *e.g.*, an immobilized glucose isomerase (such as Sweetzyme®).

At least one enzymatic improvement of this process can be performed. Reduction of the calcium dependency of the liquefying *Bacillus* sp. no. 707 α-amylase or variant thereof. Addition of free calcium is required to ensure adequately high stability of the *Bacillus* sp. no. 707 α-amylase or variant thereof, but free calcium strongly inhibits the activity of the glucose isomerase and needs to be removed, by means of an expensive unit operation, to an extent that reduces the level of free calcium to below 3-5 ppm. Cost savings can be obtained if such an operation could be avoided, and the liquefaction process could be performed without addition of free calcium ions.

For example, a less calcium-dependent enzyme, which is stable and highly active at low concentrations of free calcium (<40 ppm) can be utilized in the composition and procedures. Such a *Bacillus* sp. no. 707 α-amylase or variant thereof should have a pH optimum at a pH in the range of about 4.5 to about 6.5, or in the range of about 4.5 to about 5.5.

A *Bacillus* sp. no. 707 α-amylase or variant thereof can be used in laboratory and in industrial settings to hydrolyze starch or any maltodextrine-comprising compound for a variety of purposes. These *Bacillus* sp. no. 707 α-amylases or variants thereof can be used alone to provide specific hydrolysis or can be combined with other amylases to provide a "cocktail" with a broad spectrum of activity. Exemplary uses include the removal or partial or complete hydrolysis of starch or any maltodextrine-comprising compound from biological, food, animal feed, pharmaceutical, or industrial samples.

Another aspect contemplates compositions and methods of using the compositions in a fermentation process, wherein a starch substrate is liquefied and/or saccharified in the presence of the *Bacillus* sp. no. 707 α-amylase or variant thereof to produce glucose and/or maltose suitable for conversion into a fermentation product by a fermenting organism, such as a yeast. Such fermentation processes include a process for producing ethanol for fuel or drinking ethanol (portable alcohol), a process for producing a beverage, a process for producing desired organic compounds (*e.g.*, such as citric acid, itaconic acid, lactic acid, gluconic acid, sodium gluconate, calcium gluconate, potassium gluconate, glucono delta lactone, or sodium erythorbate), ketones, amino acids (such as glutamic acid, sodium monoglutaminate), but also more complex compounds (*e.g.*, antibiotics, such as penicillin, tetracyclin), enzymes, vitamins (*e.g.*, riboflavin, vitamin $B_{12}$, β-carotene), and hormones, which are difficult to produce synthetically.

The starch to be processed may be a highly refined starch quality, such as at least 90%, at least 95%, at least 97%, or at least 99.5% pure. Alternatively, the starch can be a more crude starch containing material comprising milled whole grain including non-starch fractions such as germ residues and fibers. The raw material, such as whole grain, is milled in order to open up the structure and allowing for further processing. Two milling processes can be used: wet and dry milling. Also, corn grits such as milled corn grits may be applied.

Dry milled grain will, in addition to starch, comprise significant amounts of non-starch carbohydrate compounds. When such a heterogeneous material is processed by jet cooking *Bacillus* sp. no. 707g often only a partial gelatinization of the starch is achieved. As the *Bacillus* sp. no. 707 α-amylase or variant thereof has a high activity towards ungelatinized starch, the enzyme(s) may be advantageously applied in a process comprising liquefaction and/or saccharification jet cooked dry milled starch.

Furthermore, due to the superior hydrolysis activity of the *Bacillus* sp. no. 707 α-amylases or variants thereof, the need for glucoamylase during the saccharification step is greatly reduced. This allows saccharification to be performed at very low levels of glucoamylase activity. Glucoamylase activity is either absent, or if present, then present in an amount of no more than or even less than 0.5 AGU/g DS, or no more than or even less than 0.4 AGU/g DS, or no more than or even less than about 0.3 AGU/g DS, or less than 0.1 AGU, such as no more than or even less than about 0.05 AGU/g DS of starch substrate. "DS" is the unit of enzyme added per gram of dry solid substrate. Expressed in mg enzyme protein, the enzyme having glucoamylase activity is either absent or present in an in an amount of no more than or even less than about 0.5 mg EP/g DS, or no more than or even less than about 0.4 mg EP/g DS, or no more than or even less than about 0.3 mg EP/g DS, or no more than or even less than about 0.1 mg EP/g DS (e.g., no more than or even less than about 0.05 mg EP/g DS or no more than or even less than 0.02 mg EP/g DS of starch substrate). The glucoamylase may be derived from a strain within *Aspergillus* sp., *Talaromyces* sp., *Pachykytospora* sp., or *Trametes* sp., with exemplary examples being *Aspergillus niger*, *Talaromyces emersonii*, *Trametes cingulata*, or *Pachykytospora papyracea*.

The process may comprise a) contacting a starch substrate with a *Bacillus* sp. no. 707 α-amylase or variant thereof comprising a catalytic module having α-amylase activity and a carbohydrate-binding module, *e.g.*, the polypeptide of the first aspect; b) incubating said starch substrate with said enzyme for a time and at a temperature sufficient to achieve conversion of at least 90%, or at least 92%, at least 94%, at least 95%, at least 96%, at least 97%, at least 98%, at least 99%, at least 99.5% w/w of said starch substrate into fermentable sugars; c) fermenting to produce a fermentation product; and d) optionally recovering the fermentation product. During the process steps b) and/or c), an enzyme having glucoamylase activity is either absent or present in an amount from 0.001 to 2.0 AGU/g DS, from 0.01 to 1.5 AGU/g DS, from 0.05 to 1.0 AGU/g DS, from 0.01 to 0.5 AGU/g DS. The enzyme having glucoamylase activity can either absent or present in an in an amount of no more than or even less than 0.5 AGU/g DS, or no more than or even less than 0.4 AGU/g DS, or no more than or even less than 0.3 AGU/g DS, or no more than or even less than 0.1 AGU/g DS (*e.g.*, no more than or even less than 0.05 AGU/g DS of starch substrate). Expressed in mg enzyme protein, the enzyme having glucoamylase activity is either absent or present in an in an amount of no more than or even less than 0.5 mg EP/g DS, or no more than or even less than 0.4 mg EP/g DS, or no more than or even less than 0.3 mg EP/g DS, or no more than or even less than 0.1 mg EP/g DS (*e.g.*, no more than or even less than 0.05 mg EP/g DS or no more than or even less than 0.02 mg EP/g DS of starch substrate). In the process steps a), b), c), and/or d) may be performed separately or simultaneously.

In another aspect the process may comprise: a) contacting a starch substrate with a yeast cell transformed to express a *Bacillus* sp. no. 707 α-amylase or variant thereof comprising a catalytic module having α-amylase activity and a carbohydrate-binding module; b) incubating said starch substrate with said yeast for a time and at a temperature sufficient to achieve conversion of at least 90% w/w of said starch substrate into fermentable sugars; c) fermenting to produce ethanol; d) optionally recovering ethanol. The steps a), b), and c) may performed separately or simultaneously.

In yet another aspect, the process comprising hydrolysis of a slurry of gelatinized or granular starch, in particular hydrolysis of granular starch into a soluble starch hydrolysate at a temperature below the initial gelatinization temperature of said granular starch. In addition to being contacted with a polypeptide comprising a catalytic module having α-amylase activity and a carbohydrate-binding module. The starch can be contacted with any one or more of the following a fungal α-amylase (EC 3.2.1.1) and one or more of the following: a β-amylase (EC 3.2.1.2), and a glucoamylase (EC 3.2.1.3). In a further aspect, another amylolytic enzyme or a debranching enzyme, such as an isoamylase (EC 3.2.1.68), or a pullulanases (EC 3.2.1.41) may be added to the *Bacillus* sp. no. 707 α-amylase or variant thereof.

In an embodiment, the process is conducted at a temperature below the initial gelatinization temperature. Such processes are oftentimes conducted at least at 30°C, at least 31°C, at least 32°C, at least 33°C, at least 34°C, at least 35°C, at least 36°C, at least 37°C, at least 38°C, at least 39°C, at least 40°C, at least 41 °C, at least 42°C, at least 43°C, at least 44°C, at least 45°C, at least 46°C, at least 47°C, at least 48°C, at least 49°C, at least 50°C, at least 51°C, at least 52°C, at least 53°C, at least 54°C, at least 55°C, at least 56°C, at least 57°C, at least 58°C, at least 59°C, or at least 60°C. The pH at which the process is conducted may in be in the range of about 3.0 to about 7.0, or from about 3.5 to about 6.0, or from about 4.0 to about 5.0. One aspect contemplates a process comprising fermentation, e.g. with a yeast to produce ethanol, *e.g.*, at a temperature around 32°C, such as from 30°C to 35°C.

In another aspect, the process comprises simultaneous saccharification and fermentation, *e.g.*, with a yeast to produce ethanol, or another suitable fermentation organism to produce a desired organic compound, such as at a temperature from 30°C to 35°C, *e.g.*, at around 32°C.

In the above fermentation processes, the ethanol content reaches at least about 7%, at least about 8%, at least about 9%, at least about 10%, at least about 11%, at least about 12%, at least about 13%, at least about 14%, at least about 15% such as at least about 16% ethanol.

The starch slurry to be used in any of the above aspects may have about 20% to about 55% dry solids granular starch, about 25% to about 40% dry solids granular starch, or from about 30% to about 35% dry solids granular starch. After being contacted with a *Bacillus* sp. no. 707 α-amylase or variant thereof, the enzyme converts the soluble starch into a soluble starch hydrolysate of the granular starch in the amount of at least 85%, at least 86%, at least 87%, at least 88%, at least 89%, at least 90%, at least 91 %, at least 92%, at least 93%, at least 94%, at least 95%, at least 96%, at least 97%, at least 98%, or at least 99%.

In another embodiment, a *Bacillus* sp. no. 707 α-amylase or variant thereof comprises a catalytic module having α-amylase activity and a carbohydrate-binding module, *e.g.*, the polypeptide of the first aspect, is used in a process for liquefaction, saccharification of a gelatinized starch, *e.g.*, but not limited to gelatinization by jet cooking. The process may comprise fermentation to produce a fermentation product, *e.g.*, ethanol. Such a process for producing ethanol from starch-containing material by fermentation comprises: (i) liquefying said starch-containing material with a polypeptide comprising a catalytic module having α-amylase activity and a carbohydrate-binding module, *e.g.*, the polypeptide of the first aspect; (ii) saccharifying the liquefied mash obtained; and (iii) fermenting the material obtained in step (ii) in the presence of a fermenting organism. Optionally the process further comprises recovery of the ethanol. The saccharification and fermentation processes may be carried out as a simultaneous saccharification and fermentation process (SSF process). During the fermentation, the ethanol content reaches at least about 7%, at least about 8%, at least about 9%, at least about 10% such as at least about 11%, at least about 12%, at least about 13%, at least about 14%, at least 15% such as at least 16% ethanol.

The starch to be processed in the processes of the above aspects may in particular be obtained from tubers, roots, stems, legumes, cereals or whole grain. More specifically, the granular starch may be obtained from corns, cobs, wheat, barley, rye, milo, sago, cassava, tapioca, sorghum, rice, peas, bean, banana, or potatoes. Also contemplated are both waxy and non-waxy types of corn and barley.

The composition described above may be used for liquefying and/or saccharifying a gelatinized or a granular starch, and a partly gelatinized starch. A partly gelatinized starch is a starch that to some extent is gelatinized, *i.e.*, wherein part of the starch has irreversibly swelled and gelatinized and part of the starch is still present in a granular state.

The composition described above may comprise an acid α-amylase variant present in an amount of 0.01 to 10.0 AFAU/g DS, or 0.1 to 5.0 AFAU/g DS, or 0.5 to 3.0 AFAU/AGU, or 0.3 to 2.0 AFAU/g DS. The composition may be applied in any of the starch processes described above.

As used herein, the term "liquefaction" or "liquefy" means a process by which starch is converted to shorter chain and less viscous dextrins. Generally, this process involves gelatinization of starch simultaneously with or followed by the addition of a *Bacillus* sp. no. 707 α-amylase or variant thereof. Additional liquefaction inducing enzymes may also be added.

As used herein, the term "primary liquefaction" refers to a step of liquefaction when the slurry's temperature is raised to or near its gelatinization temperature. Subsequent to the raising of the temperature, the slurry is sent through a heat exchanger or jet to temperatures from 200-300°F, *e.g.*, 220-235°F. Subsequent to application to a heat exchange or jet temperature, the slurry is held for a period of 3-10 minutes at that temperature. This step of holding the slurry at 200-300°F is primary liquefaction.

As used herein, the term "secondary liquefaction" refers the liquefaction step subsequent to primary liquefaction (heating to 200-300°F), when the slurry is allowed to cool to atmospheric temperature. This cooling step can be 30 minutes to 180 minutes (3 hours), *e.g.* 90 minutes to 120 minutes (2 hours).

As used herein, the term "minutes of secondary liquefaction" refers to the time that has elapsed from the start of secondary liquefaction, to the time that the DE is measured.

Another aspect contemplates the additional use of a β-amylase in the composition comprising *Bacillus* sp. no. 707 α-amylase or variant thereof. β-amylases (EC 3.2.1.2) are exo-acting maltogenic amylases, which catalyze the hydrolysis of 1,4-α-glucosidic linkages in to amylose, amylopectin, and related glucose polymers, thereby releasing maltose.

β-amylases have been isolated from various plants and microorganisms (W. M. Fogarty and C. T. Kelly, PROGRESS IN INDUSTRIAL MICROBIOLOGY, vol. 15, pp. 112-115, 1979). These β-amylases are characterized by having optimum temperatures in the range from 40°C to 65°C, and optimum pH in the range from about 4.5 to about 7.0. Contemplated β-amylases include, but are not limited to, 1β-amylases from barley Spezyme® BBA 1500, Spezyme® DBA, Optimalt® ME, Optimalt® BBA (Genencor International Inc.) and Novozym™ WBA (Novozymes A/S).

Another enzyme contemplated for use in the composition is a glucoamylase (EC 3.2.1.3). Glucoamylases are derived from a microorganism or a plant. Exemplary glucoamylases are of fungal or bacterial origin. Exemplary bacterial glucoamylases are *Aspergillus* glucoamylases, in particular *A. niger* G1 or G2 glucoamylase (Boel et al., EMBO J. 3(5): 1097-1102 (1984), or variants thereof, such as disclosed in WO 92/00381; and WO 00/04136; the *A. awamori* glucoamylase (WO 84/02921); *A. oryzae* (Agric. Biol. Chem., 55(4): 941-949 (1991)), or variants or fragments thereof.

Other contemplated *Aspergillus* glucoamylase variants include variants to enhance the thermal stability: G137A and G139A (Chen et al., Prot. Eng. 9: 499-505 (1996)); D257E and D293E/Q (Chen et al., Prot. Eng. 8: 575-582 (1995)); N182 (Chen et al., Biochem. J. 301: 275-281 (1994)); disulfide bonds, A246C (Fierobe et al., Biochemistry, 35: 8698-8704 (1996)); and introduction of Pro residues in positions A435 and S436 (Li et al., Protein Eng. 10: 1199-1204 (1997)). Other contemplated glucoamylases include and *Talaromyces* glucoamylases, in particular derived from *Talaromyces emersonii* (WO 99/28448), *Talaromyces leycettanus* (U.S. Patent No. RE 32,153), *Talaromyces duponti*, *Talaromyces thermophilus* (U.S. Patent No. 4,587,215). Bacterial glucoamylases contemplated include glucoamylases from the genus *Clostridium*, in particular *C. thermoamylolyticum* (EP 135138) and *C. thermohydrosulfuricum* (WO 86/01831). Exemplary glucoamylases include the glucoamylases derived from *Aspergillus oryzae*. Also contemplated are the commercial glucoamylases such as AMG 200L; AMG 300 L; SAN™ SUPER and AMG™ E (Novozymes); OPTIDEX®300 (from Genencor International, Inc.); AMIGASE® and AMIGASE® PLUS (DSM); G-ZYME® G900 (Enzyme Bio-Systems); G-ZYME® G990 ZR (*A. niger* glucoamylase and low protease content).

Glucoamylases may be added in an amount of 0.02-2.0 AGU/g DS, or 0.1-1.0 AGU/g DS, such as 0.2 AGU/g DS.

Additional enzymes and enzyme variants are also contemplated for inclusion in the composition. One or more α-amylases can be used in addition to a *Bacillus* sp. no. 707 α-amylase or variant thereof, or can further include other enzymes discussed herein.

Another enzyme that can optionally be added is a debranching enzyme, such as an isoamylase (EC 3.2.1.68) or a pullulanase (EC 3.2.1.41). Isoamylase hydrolyses α-1,6-D-glucosidic branch linkages in amylopectin and β-limit dextrins and can be distinguished from pullulanases by the inability of isoamylase to attack pullulan, and by the limited action on α-limit dextrins. Debranching enzymes may be added in effective amounts well known to the person skilled in the art.

The exact composition of the products of the process depends on the combination of enzymes applied as well as the type of granular starch processed. For example, the soluble hydrolysate can be maltose with a purity of at least about 85%, at least about 90%, at least about 95.0%, at least about 95.5%, at least about 96.0%, at least about 96.5%, at least about 97.0%, at least about 97.5%, at least about 98.0%, at least about 98.5, at least about 99.0% or at least about 99.5%. Alternatively, the soluble starch hydrolysate can be glucose or the starch hydrolysate has a DX (glucose percent of total solubilized dry solids) of at least 94.5%, at least 95.0%, at least 95.5%, at least 96.0%, at least 96.5%, at least 97.0%, at least 97.5%, at least 98.0%, at least 98.5, at least 99.0% or at least 99.5%. The process can include a product which is a specialty syrup, such as a specialty syrup containing a mixture of glucose, maltose, DP3 and DPn for use in the manufacture of ice creams, cakes, candies, canned fruit.

Two milling processes are: wet and dry milling. In dry milling, the whole kernel is milled and used. Wet milling gives a good separation of germ and meal (starch granules and protein), and is with a few exceptions, applied at locations where the starch hydrolysate is used in production of syrups. Both dry and wet milling are well known in the art of starch processing and are equally contemplated for use with the compositions and methods disclosed. The process may be conducted in an ultrafiltration system where the retentate is held under recirculation in presence of enzymes, raw starch and water and where the permeate is the soluble starch hydrolysate. Equally contemplated is the process conducted in a continuous membrane reactor with ultrafiltration membranes and where the retentate is held under recirculation in presence of enzymes, raw starch and water, and where the permeate is the soluble starch hydrolysate. Also contemplated is the process conducted in a continuous membrane reactor with microfiltration membranes and where the retentate is held under recirculation in presence of enzymes, raw starch and water, and where the permeate is the soluble starch hydrolysate.

In one regard, the soluble starch hydrolysate of the process is subjected to conversion into high fructose starch-based syrup (HFSS), such as high fructose corn syrup (HFCS). This conversion can be achieved using a glucose isomerase, and by an immobilized glucose isomerase supported on a solid support. Contemplated isomerases include the commercial products Sweetzyme®, IT (Novozymes A/S); G-zyme® IMGI, and G-zyme® G993, Ketomax™, G-zyme® G993 (Rhodia); G-zyme® G993 liquid, GenSweet® IGI (Genencor International, Inc.).

In another aspect, the soluble starch hydrolysate produced by these methods can be used in the production of fuel or potable ethanol. In the process of the third aspect the fermentation may be carried out simultaneously or separately/sequential to the hydrolysis of the granular starch slurry. When the fermentation is performed simultaneous to the hydrolysis, the temperature is between 30°C and 35°C, or between 31°C and 34°C. The process may be conducted in an ultrafiltration system where the retentate is held under recirculation in presence of enzymes, raw starch, yeast, yeast nutrients and water and where the permeate is an ethanol containing liquid. Equally contemplated is the process conducted in a continuous membrane reactor with ultrafiltration membranes and where the retentate is held under recirculation in presence of enzymes, raw starch, yeast, yeast nutrients and water and where the permeate is an ethanol containing liquid.

The soluble starch hydrolysate of the process may also be used for production of a fermentation product comprising fermenting the treated starch into a fermentation product, such as citric acid, monosodium glutamate, gluconic acid, sodium gluconate, calcium gluconate, potassium gluconate, glucono delta lactone, or sodium erythorbate.

The amylolytic activity of a *Bacillus* sp. no. 707 $\alpha$-amylase or variant thereof may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch, the blue color gets weaker and gradually turns into a reddish-brown, which is compared to a colored glass standard.

## 10. Compositions and Methods for Textile Desizing

Also contemplated are compositions and methods of treating fabrics (e.g., to desize a textile) using one or more *Bacillus* sp. no. 707 $\alpha$-amylases or variants thereof. The enzyme can be used in any fabric-treating method, which are well known in the art, *see*, *e.g.*, U.S. Patent No. 6,077,316. For example, in one aspect, the feel and appearance of a fabric is improved by a method comprising contacting the fabric with a *Bacillus* sp. no. 707 $\alpha$-amylase or variant thereof in a solution. In one aspect, the fabric is treated with the solution under pressure.

In one aspect, the enzymes are applied during or after the weaving of textiles, or during the desizing stage, or one or more additional fabric processing steps. During the weaving of textiles, the threads are exposed to considerable mechanical strain. Prior to weaving on mechanical looms, warp yarns are often coated with sizing starch or starch derivatives in order to increase their tensile strength and to prevent breaking. The enzymes can be applied to remove these sizing starch or starch derivatives. After the textiles have been woven, a fabric can proceed to a desizing stage. This can be followed by one or more additional fabric processing steps. Desizing is the act of removing size from textiles. After weaving, the size coating must be removed before further processing the fabric in order to ensure a homogeneous and wash-proof result. Also provided is a method of desizing comprising enzymatic hydrolysis of the size by the action of a *Bacillus* sp. no. 707 $\alpha$-amylase or variant thereof.

The enzymes can be used alone or with other desizing chemical reagents and/or desizing enzymes to desize fabrics, including cotton-containing fabrics, as detergent additives, *e.g.*, in aqueous compositions. A *Bacillus* sp. no. 707 α-amylase or variant thereof can also be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of amylolytic enzymes in order to provide softness to the fabric and make the cotton more accessible to the subsequent enzymatic finishing steps. The enzymes can be used in methods of finishing denim garments (*e.g.*, a "bio-stoning process"), enzymatic desizing and providing softness to fabrics, and/or finishing process. Dosage of the amylase varies depending on the process type. Smaller dosages would require more time than larger dosages of the same enzyme. However, there is no upper limit on the amount of a desizing amylase present other than that dictated by the physical constraints of the solution. Thus, the limit of the enzyme may be the amount capable of solubilization in the solution. Typically, desizing enzymes, such as α-amylases, are incorporated in to the treating composition in an amount from about 0.00001% to about 2% of enzyme protein by weight of the fabric; or from about 0.0001% to about 1% of enzyme protein by weight of the fabric; or from about 0.001% to about 0.5% of enzyme protein by weight of the fabric; and in another example would be from about 0.01% to about 0.2% of enzyme protein by weight of the fabric.

**11. Compositions and Methods for Baking and Food Preparation**

For the commercial and home use of flour for baking and food production, it is important to maintain an appropriate level of α-amylase activity in the flour. A level of activity that is too high may result in a product that is sticky and/or doughy and unmarketable; but flour with insufficient α-amylase activity may not contain enough sugar for proper yeast function, resulting in dry, crumbly bread. To augment the level of endogenous α-amylase activity in flour, an α-amylase may be added to flour in the form of a *Bacillus* sp. no. 707 α-amylase or variant thereof. Therefore, the ability to determine the level of activity of both endogenous (natural) and fungal α-amylase, or other α-amylase, in a flour sample would benefit the food production process and promote more efficient use of flour in food production.

In addition to the use of grains and other plant products in baking, grains such as barley, oats, wheat, as well as plant components such as corn, hops, and rice are used for brewing, both in industry and for home brewing. The components used in brewing may be unmalted or malted, which means partially germinated resulting in an increase in the levels of enzymes including α-amylase. For successful brewing, adequate levels of α-amylase enzyme activity are necessary to ensure the appropriate levels of sugars for fermentation.

As used herein, the term "flour" means milled or ground cereal grain. The term "flour" may also mean Sago or tuber products that have been ground or mashed. In some embodiments, flour may also contain components in addition to the milled or mashed cereal or plant matter. An example of an additional component, although not intended to be limiting, is a leavening agent. Cereal grains include: wheat, oat, rye, and barley. Tuber products can include tapioca flour, cassava flour, and custard powder. The term "flour" also includes ground corn flour, maize-meal, rice flour, whole-meal flour, self-rising flour, tapioca flour, cassava flour, ground rice, enriched flower, and custard powder.

As used herein, the term "stock" means grains and plant components that are crushed or broken. For example, barley used in beer production is a grain that has been coarsely ground or crushed to yield a consistency appropriate for producing a mash for fermentation. As used herein, the term "stock" includes any of the aforementioned types of plants and grains in crushed or coarsely ground forms. The methods described herein may be used to determine α-amylase activity levels in flours, and also in stock, which includes the aforementioned types of grains, tubers, and other plant products that have been crushed.

Also disclosed are methods for measuring α-amylase activity in flour and grain or tuber products and stock. As used herein, the term "α-amylase" means endogenous α-amylase (present in the flour or stock) or a *Bacillus* sp. no. 707 α-amylase or variant thereof that has been added to the flour or stock.

A *Bacillus* sp. no. 707 α-amylase or variant thereof alone or in a combination with other amylases can be added to prevent staling. The anti-staling amylases used may be any amylase that is effective in retarding the staling (crumb firming) of baked products.

The amylase can have a temperature optimum in the presence of starch in the ranges for example of 30-90°C, 50-80°C, 55-75°C, 60-70°C. The temperature optimum may be measured in a 1% solution of soluble starch at pH 5.5.

Additional anti-staling amylases that can be used in combination with a *Bacillus* sp. no. 707 α-amylase include an endo-amylase, *e.g.*, a bacterial endo-amylase from *Bacillus*. For example, the additional amylase can be a maltogenic alpha-amylase (EC 3.2.1.133), *e.g.* from *Bacillus.* Novamyl® is a maltogenic alpha-amylase from *B. stearothermophilus* strain NCIB 11837 and is described in C. Christophersen et al., 1997 Starch 50(1): 39-45.

Other examples of anti-staling endo-amylases can include other bacterial alpha-amylases, derived *e.g.* from *Bacillus,* such as *B. licheniformis* or *B. amyloliquefaciens*.

The anti-staling amylase may be an exo-amylase such as β-amylase, *e.g.* from plant (*e.g.*, soy bean) or from microbial sources (*e.g.*, *Bacillus*).

The α-amylase of *Bacillus* sp. no. 707 can be added alone or with other amylases in an amount effective for retarding the staling (crumb firming) of the baked product. The amount of anti-staling amylase will typically be in the range of 0.01-10 mg of enzyme protein per kg of flour, *e.g.* 1-10 mg/kg.

The baking composition comprising an α-amylase of *Bacillus* sp. no. 707 can further comprise a phospholipase. The phospholipase may have $A_1$ or $A_2$ activity to remove fatty acid from the phospholipid and form a lyso-phospholipid. It may or may not have lipase activity, i.e. activity on triglycerides. The phospholipase can have a temperature optimum in the range of 30-90°C., *e.g.* 30-70°C. The added phospholipases can be of animal origin, *e.g.* from pancreas (*e.g.*, bovine or porcine pancreas), snake venom or bee venom. Alternatively, the phospholipase may be of microbial origin, *e.g.* from filamentous fungi, yeast or bacteria, such as the genus or species *Aspergillus*, *A. niger*; *Dictyostelium*, *D. discoideum*; *Mucor*, *M. javanicus*, *M. mucedo*, *M. subtilissimus*; *Neurospora*, *N. crassa*; *Rhizomucor*, *R. pusillus*; *Rhizopus*, *R. arrhizus*, *R. japonicus*, *R. stolonifer*; *Sclerotinia*, *S. libertiana*; *Trichophyton*, *T. rubrum*; *Whetzelinia*, *W. sclerotiorum*; *Bacillus*, *B. megaterium*, *B. subtilis*; *Citrobacter*, *C. freundii*; *Enterobacter*, *E. aerogenes*, *E. cloacae*; *Edwardsiella*, *E. tarda*; *Erwinia*, *E. herbicola*; *Escherichia*, *E. coli*; *Klebsiella*, *K. pneumoniae*; *Proteus*, *P. vulgaris*; *Providencia*, *P. stuartii*; *Salmonella*, *S. typhimurium*; *Serratia*, *S. liquefasciens*, *S. marcescens*; *Shigella*, *S. flexneri*; *Streptomyces*, *S. violeceoruber*; *Yersinia*, *Y. enterocolitica*; *Fusarium*, *F. oxysporum* (*e.g.*, strain DSM 2672).

The phospholipase is added in an amount that improves the softness of the bread during the initial period after baking, particularly the first 24 hours. The amount of phospholipase will typically be in the range of 0.01-10 mg of enzyme protein per kg of flour (*e.g.* 0.1-5 mg/kg) or 200-5000 LEU/kg of flour (*e.g.* 500-2000 LEU/kg). A phospholipase with lipase activity is generally added in an amount corresponding to a lipase activity of 20-1000 LU/kg of flour, particularly 50-500 LU/kg. One LU (Lipase Unit) is defined as the amount of enzyme required to release 1 μmol butyric acid per minute at 30.0°C; pH 7.0; with gum arabic as emulsifier and tributyrin as substrate.

Compositions of dough generally comprise wheat meal or wheat flour and/or other types of meal, flour or starch such as corn flour, cornstarch, rye meal, rye flour, oat flour, oatmeal, soy flour, sorghum meal, sorghum flour, potato meal, potato flour or potato starch. The dough may be fresh, frozen or par-baked.

The dough is normally a leavened dough or a dough to be subjected to leavening. The dough may be leavened in various ways, such as by adding chemical leavening agents, *e.g.*, sodium bicarbonate or by adding a leaven (fermenting dough). For example, the dough can be leavened by adding a suitable yeast culture, such as a culture of *Saccharomyces cerevisiae* (baker's yeast), *e.g.* a commercially available strain of *S. cerevisiae*.

The dough may also comprise other conventional dough ingredients, *e.g.*, proteins, such as milk powder, gluten, and soy; eggs (either whole eggs, egg yolks or egg whites); an oxidant such as ascorbic acid, potassium bromate, potassium iodate, azodicarbonamide (ADA) or ammonium persulfate; an amino acid such as L-cysteine; a sugar; a salt such as sodium chloride, calcium acetate, sodium sulfate or calcium sulfate.

The dough may comprise fat (triglyceride) such as granulated fat or shortening.

The dough may further comprise an emulsifier such as mono- or diglycerides, diacetyl tartaric acid esters of mono- or diglycerides, sugar esters of fatty acids, polyglycerol esters of fatty acids, lactic acid esters of monoglycerides, acetic acid esters of monoglycerides, polyoxyetliylene stearates, or lysolecithin, but is applicable to a dough which is made without addition of emulsifiers (other than optionally phospholipid).

Optionally, an additional enzyme may be used together with the anti-staling amylase and the phospholipase. The additional enzyme may be a second amylase, such as an amyloglucosidase, a β-amylase, a cyclodextrin glucanotransferase, or the additional enzyme may be a peptidase, in particular an exopeptidase, a transglutaminase, a lipase, a cellulase, a hemicellulase, in particular a pentosanase such as xylanase, a protease, a protein disulfide isomerase, *e.g.*, a protein disulfide isomerase as disclosed in WO 95/00636, a glycosyltransferase, a branching enzyme (1,4-α-glucan branching enzyme), a 4-α-glucanotransferase (dextrin glycosyltransferase) or an oxidoreductase, *e.g.*, a peroxidase, a laccase, a glucose oxidase, a pyranose oxidase, a lipoxygenase, an L-amino acid oxidase or a carbohydrate oxidase.

The additional enzyme may be of any origin, including mammalian and plant origin, and as well as of microbial (bacterial, yeast or fungal) origin and may be obtained by techniques conventionally used in the art.

The xylanase can be microbial origin, *e.g.* derived from a bacterium or fungus, such as a strain of *Aspergillus,* in particular of *A. aculeatus*, *A. niger* (cf. WO 91/19782), *A. awamori* (WO 91/18977), or *A. tubigensis* (WO 92/01793); from a strain of *Trichoderma*, *e.g. T. reesei*, or from a strain of *Humicola*, *e.g. H. insolens* (WO 92/17573). Pentopan® and Novozym 384® are commercially available xylanase preparations produced from *Trichoderma reesei.*

The amyloglucosidase may be an *A. niger* amyloglucosidase (such as AMG®). Other useful amylase products include Grindamyl® A 1000 or A 5000 (available from Grindsted Products, Denmark) and Amylase® H or Amylase® P (available from DSM Gist Brocades, The Netherlands).

The glucose oxidase may be a fungal glucose oxidase, in particular an *Aspergillus niger* glucose oxidase (such as Gluzyme®).

Exemplary proteases are Neutrase® (Novozymes) and Protex OxG (Genencor International, Inc.).

Exemplary lipase can be derived from strains of *Thermomyces* (*Humicola*), *Rhizomucor*, *Candida*, *Aspergillus*, *Rhizopus*, or *Pseudomonas*, in particular from *Thermomyces lanuginosus* (*Humicola lanuginosa*), *Rhizomucor miehei*, *Candida antarctica*, *Aspergillus niger*, *Rhizopus delemar* or *Rhizopus arrhizus* or *Pseudomonas cepacia*. In specific embodiments, the lipase may be Lipase A or Lipase B derived from *Candida antarctica* as described in WO 88/02775, or the lipase may be derived from *Rhizomucor miehei* as described in EP 238,023, or *Humicola lanuginosa* described in EP 305,216, or *Pseudomonas cepacia* as described in EP 214,761 and WO 89/01032.

The process may be used for any kind of baked product prepared from dough, either of a soft or a crisp character of a white, light, or dark type. Examples are bread (in particular white, whole-meal or rye bread), typically in the form of loaves or rolls, French baguette-type bread, pita bread, tortillas, cakes, pancakes, biscuits, cookies, pie crusts, crisp bread, steamed bread, pizza and the like.

Another aspect contemplates the use of the *Bacillus* sp. no. 707 α-amylase or variant thereof in a pre-mix comprising flour together with an anti-staling amylase, a phospholipase and a phospholipid. The pre-mix may contain other dough-improving and/or bread-improving additives, *e.g.* any of the additives, including enzymes, mentioned above.

Another aspect provided is an enzyme preparation comprising an anti-staling amylase and a phospholipase, for use as a baking additive. The enzyme preparation can be in the form of a granulate or agglomerated powder. It can have a narrow particle size distribution with more than 95% (by weight) of the particles in the range from 25 to 500 μm.

Granulates and agglomerated powders may be prepared by conventional methods, e.g. by spraying the amylase onto a carrier in a fluid-bed granulator. The carrier may consist of particulate cores having a suitable particle size. The carrier may be soluble or insoluble, *e.g.* a salt (such as NaCl or sodium sulfate), a sugar (such as sucrose or lactose), a sugar alcohol (such as sorbitol), starch, rice, corn grits, or soy.

Another aspect contemplates the enveloping of a *Bacillus* sp. no. 707 α-amylase. To prepare the enveloped alpha-amylase particles, the enzymes are contacted with a food grade lipid, discussed in further detail below, in sufficient quantity to suspend all of the alpha-amylase particles.

Food grade lipids, as used herein, may be any naturally organic compound that is insoluble in water but is soluble in non-polar organic solvents such as hydrocarbon or diethyl ether. The food grade lipids utilized can include, but are not limited to, triglycerides either in the form of fats or oils that are either saturated or unsaturated. Examples of fatty acids and combinations thereof which make up the saturated triglycerides utilized include, but are not limited to, butyric (derived from milk fat), palmitic (derived from animal and plant fat), and/or stearic (derived from animal and plant fat). Examples of fatty acids and combinations thereof which make up the unsaturated triglycerides utilized include, but are not limited to, palmitoleic (derived from animal and plant fat), oleic (derived from animal and plant fat), linoleic (derived from plant oils), and/or linolenic (derived from linseed oil). Other food grade lipids contemplated and within the scope include, but are not limited to, monoglycerides and diglycerides derived from the triglycerides discussed above, phospholipids and glycolipids.

The food grade lipid, in the liquid form, is contacted with a powdered form of the alpha-amylase particles in such a fashion that the lipid material covers at least a portion of the surface of at least a majority, and for example 100% of the α-amylase particles. Thus, each alpha-amylase particle is individually enveloped in a lipid. For example, all or substantially all of the particles of α-amylase are provided with a thin, continuous, enveloping film of lipid. This can be accomplished by first pouring a quantity of lipid into a container, and then slurrying the α-amylase so that the lipid thoroughly wets the surface of each α-amylase particle. After a short period of stirring, the enveloped α-amylase particles, carrying a substantial amount of the lipids on their surfaces, are recovered. The thickness of the coating so applied to the particles of α-amylase can be controlled by selection of the type of lipid used and by repeating the operation in order to build up a thicker film, when desired.

The storing, handling and incorporation of the loaded delivery vehicle can be accomplished by means of a packaged mix. The packaged mix can comprise the enveloped α-amylase. However, the packaged mix may further contain additional ingredients as required by the manufacturer or baker. After the enveloped α-amylase is incorporated into the dough, the baker continues through the normal production process for that product.

The advantages of enveloping the α-amylase are two-fold. First, the food grade lipid protects the enzyme from thermal denaturation during the baking process for those enzymes that are heat labile. Consequently, while the α-amylase is stabilized and protected during the proving and baking stages, it is released from the protective coating in the final baked good product, where it hydrolyzes the glucosidic linkages in polyglucans. The loaded delivery vehicle also provides a sustained release of the active enzyme into the baked good. That is, following the baking process, active α-amylase is continually released from the protective coating at a rate that counteracts, and therefore reduces the rate of, staling mechanisms.

In general, the amount of lipid applied to the α-amylase particles can vary from a few percent of the total weight of the α-amylase to many times that weight, depending upon the nature of the lipid, the manner in which it is applied to the α-amylase particles, the composition of the dough mixture to be treated, and the severity of the dough-mixing operation involved.

The loaded delivery vehicle (*i.e.*, the lipid-enveloped enzyme) is added to the ingredients used to prepare a baked good in an effective amount to extend the shelf-life of the baked good. The baker computes the amount of enveloped α-amylase, prepared as discussed above, that will be required to achieve the desired anti-staling effect. The amount of the enveloped alpha-amylase required is calculated based on the concentration of enzyme enveloped and on the proportion of α-amylase to flour specified. A wide range of concentrations has been found to be effective, although, as has been discussed, observable improvements in anti-staling do not correspond linearly with the α-amylase concentration, but above certain minimal levels, large increases in α-amylase concentration produce little additional improvement. The α-amylase concentration actually used in a particular bakery production could be much higher than the minimum necessary in order to provide the baker with some insurance against inadvertent under-measurement errors by the baker. The lower limit of enzyme concentration is determined by the minimum anti-staling effect the baker wishes to achieve.

A typical method of preparing a baked good according to the method comprises: a) preparing lipid-coated alpha-amylase particles, wherein substantially 100 percent of the α-amylase particles are coated; b) mixing a dough containing flour; c) adding the lipid-coated α-amylase to the dough before the mixing is complete and terminating the mixing before the lipid coating is removed from the α-amylase; d) proofing the dough; and e) baking the dough to provide the baked good, wherein the α-amylase is inactive during the mixing, proofing and baking stages and is active in the baked good.

Thus, the enveloped α-amylase can be added to the dough near the end of the mix cycle. A feature of the method is that the enveloped α-amylase is added at a point in the mixing stage that allows sufficient distribution of the enveloped α-amylase though-out the dough, however, the mixing stage is terminated before the protective coating becomes stripped from the α-amylase particle(s). Depending on the type and volume of dough, and mixer action and speed, anywhere from one to six minutes or more might be required to mix the enveloped α-amylase into the dough, but two to four minutes is average. Thus, there are several variables that may determine the precise procedure. First, the quantity of enveloped α-amylase must have a total volume sufficient to allow the enveloped α-amylase to be spread throughout the dough mix. If the preparation of enveloped alpha-amylase is highly concentrated, additional oil may need to be added to the pre-mix before the enveloped α-amylase is added to the dough. Recipes and production processes may require specific modifications. However, good results generally can be achieved when 25% of the oil specified in a bread dough formula is held out of the dough and is used as a carrier for a concentrated enveloped alpha-amylase when added near the end of the mix cycle. In bread or other baked goods, recipes which have extremely low fat content (such as French-style breads), it has been found that an enveloped α-amylase mixture of approximately 1% of the dry flour weight is sufficient to properly admix the enveloped α-amylase with the dough, but the range of percentages that may work is extremely wide and is dependent on the formula, finished product, and production methodology requirements of the individual baker rather than upon any known limitations. Second, the enveloped α-amylase suspension must be added to the mix with enough time remaining in the mix cycle for complete mixture into the dough, but not so early that excessive mechanical action will strip the protective lipid coating from a large proportion of the enveloped α-amylase particles.

In another embodiment, bacterial α-amylase (BAA) is added to the lipid-coated enzyme particles. BAA is known to reduce bread to a gummy mass due to its excessive thermostability and retained activity in the fully baked loaf of bread. However, it has been found that when BAA is incorporated into the protected enzyme product, substantial additional anti-staling protection is obtained, even at very low BAA dosage levels. For example, BAA dosages of 150 RAU (Reference Amylase Units) per 100 pounds of flour have been found to be effective. In one aspect, between about 50 to 2000 RAU of BAA is added to the lipid-coated enzyme product. This low BAA dosage level, combined with the ability of the protective coating to keep enzyme in the fully-baked loaf from free contact with the starches, (except when water vapor randomly releases the enzyme from its coating) helps to achieve very high levels of anti-staling activity without the negative side-effects of BAA.

It will be apparent to those skilled in the art that various modifications and variations can be made to the compositions and methods described herein and further exemplified in the examples below without departing from the spirit or scope of the intended use.

## EXAMPLES

### EXAMPLE 1

### Codon Optimization of *Bacillus* sp. no. 707 α-Amylase

A synthetic *Bacillus* sp. no. 707 α-amylase gene, preLAT-Amy707, was ordered from GeneArt (Germany). The gene encoding the *Bacillus* sp. no. 707 α-amylase (A. Tsukamoto et al., 1988 Biochem. Biophys. Res. Commun. 151 (1): 25-31) was cloned into the integration vector, pICatH (*See* FIG. 4) resulting in the product designated pICatH-Amy707 (ori1) (*see* FIG. 5). pICatH contains the following features: (1) a temperature sensitive origin of replication (ori pE194, for replication in *Bacillus*), (2) ori pBR322 (for amplification in *E. coli*), (3) a neomycin resistance gene for selection, and (4) the native *B. licheniformis* chloramphenicol resistance gene (*cat*) for selection, chromosomal integration and cassette amplification. pICatH-Amy707(ori1) was transformed into the host strain, BML780 (BRA7 derivative, cat-, amyL-, spo-, aprL-, endoGluC-), at a permissive temperature (37°C). One neomycin resistant (neoR) and one chloramphenicol resistant (capR) transformant was selected and designated BML780 (pICatH-Amy707(oril)). The plasmid in BML780 (pICatH-707(ori)) was integrated into the *cat* region on the *B. licheniformis* genome by growing the strains at a non-permissive temperature (*i.e.*, ~50°C) in TSB (tryptic soy broth) medium with chloramphenicol. One chloramphenicol resistant clone was selected and designated BML780-pICatH-Amy707(ori1). The selected strain was grown again at the permissive temperature for several generations without antibiotics to loop-out vector sequences, and then one neomycin sensitive (neoS and capR) clone was selected. In this clone, vector sequences of pICatH on the chromosome were excised, including the neomycin resistant gene, leaving only the Amy707-cat cassette. Note that the *cat* gene used is a native *B. licheniformis* gene, and that the Amy707 gene is the only heterologous piece of DNA introduced into the host.

Next, the Amy707-cat cassette on the chromosome was amplified by growing the strain in TSB media with increasing concentrations of chloramphenicol (*i.e.*, 5, 25, 50, and 75 μg/mL chloramphenicol). After various rounds of amplification, two clones were selected (resistant against 75 μg/mL chloramphenicol and producing the largest halo after iodine staining when grown on Heart Infusion agar plates containing soluble starch): BML780-Amy707-CAP75 clone 1 and clone 2. Both strains produce large halos on starch agar plates.

## EXAMPLE 2

### Purification of *Bacillus* sp. no. 707 α-Amylase

Cells transformed with BML780-Amy707-CAP75 were fermented using techniques known in the art. The cells were separated from the fermentation broth, and the broth was concentrated using a Millipore prep scale TFF-6 10K molecular weight cutoff (MWCO) UF membrane cartridge (Millipore Corp., Bedford, MA; Cat. No. CDUF 006 TG) to obtain the UFC. Ultrafiltration concentrate (UFC) containing between 1 and 2 g/L of α-amylase was dialyzed overnight in 10 K dialysis (SpecraPor Cat. No. 132119) tubing against 20 L of 2 mM calcium chloride in 25 mM sodium acetate buffer, pH 5.0. The dialysate was filtered and one-half of the material was applied to a cation exchange column (POROS HS 20 83 mL; Applied Biosystems, California); the cation exchange column had been previously equilibrated against 1 mM calcium chloride in 25 mM sodium acetate buffer, at pH 5.0. The column was extensively washed with the same buffer until the absorbance (A) A280 nm was less than 0.1 OD units, and enzyme activity was eluted with a gradient of 0 to 100 mM sodium chloride in the same buffer. α-amylase activity eluted at 90 mM sodium chloride. The fractions with activity were pooled based on enzymatic activity as measured using a Megazyme® α-amylase kit (Cat. No. K-CERA; Megazyme). The remaining sample was treated similarly and pooled with the first. Ammonium sulfate was added to one-third of this pool to 750 mM and applied to a 96 mL phenyl Sepharose column (Cat. No. 17-0973-10; GE Healthcare), which had been previously equilibrated with 750 mM ammonium sulfate, 2 mM calcium chloride contained in 50 mM MES buffer (4-morpholineethanesulfonic acid buffer) adjusted to pH 6.8. After washing with the same solvent, the α-amylase protein was eluted using a gradient of 750 mM to 0 mM ammonium sulfate. Enzyme activity eluted at the end of this gradient. Fractions with enzyme activity were pooled. The remaining ion exchange pooled fractions were purified similarly. All phenyl Sepharose pools were combined and concentrated in a stirred Amicon cell (Millipore) with a 10 K cellulosic membrane (Millipore). The concentrate was dialyzed using 10 K dialysis tubing against 5 mM calcium chloride contained in a 50 mM hydrogen maleate buffer (pH 6.8) for 16 hours. This dialyzed sample was supplemented with 10% sorbitol to aid enzyme solubility. After sterile filtration, the *Bacillus* sp. no. 707 α-amylase containing sample was quantified by gel densitometry, which indicated a quantity of 2.8 mg/mL.

## EXAMPLE 3

### Performance Characterization

Performance tests were conducted on rice starch stained fabric swatches with an indicator dye bound to the starch (Test Fabrics Cat. No. CS-28; TestFabrics Inc.). Wash performance was judged by the release of the indicator dye into the solution phase. Prior to assays, the dyed test fabric was pre-washed with distilled water for 1 hour and then air-dried. The pre-wash of the test fabrics removed any poorly bound indicator dye prior to the assay.

One-quarter inch disks were cut from the pre-washed fabric and placed in to 96-well plates. 190 μL detergent was added into each well (IEC-A* Base Detergent; Cat. No. 88010-1; WFK Testgewebe GmBH, Germany). The plate with the swatches and detergent was preincubated at 40°C for 15 minutes. The reaction was started with the addition of 0 to 3 ppm *Bacillus* sp. no. 707 α-amylase from Example 2, or control 0 to 3 ppm α-amylase (OxAm (Purastar®) Genencor International, Inc.), which was added in a volume of 10 μL to each well. The plate was then incubated for 10 minutes at approximately 40°C at 750 rpm (Eppendorf Thermomix). After incubation, 150 μL of supernatant was transferred to a new plate and absorbance of the supernatant (which contained released indicator dye) was determined at A488 nm.

For comparison, similar assays were conducted using Stainzyme® (Novozymes) and OxAm (Purastar®; Genencor International, Inc.), a derivative of the *B. licheniformis* α-amylase (Genencor product). 0 to 3.2 ppm (mg/L) was used in the assay with the conditions being the same as described above.

The data for IEC "A" standard detergent at pH 8.0 (laundry conditions) are show in FIG. 1. Absorbance was plotted as a function of α-amylase concentration. This test indicated that *Bacillus* sp. no. 707 α-amylase performs significantly better than OxAm (Purastar®) and equal to Stainzyme® under these conditions.

Using the same assay, the same amounts of *Bacillus* sp. no. 707 α-amylase and amounts of Stainzyme® and OxAm (Purastar®), the assay was repeated with the pH adjusted to 10.1. These conditions (*i.e.*, increased pH) are reminiscent of those in automatic dishwashing. The performance of the *Bacillus* sp. no. 707 α-amylase was again better than OxAm (Purastar®), and surprisingly it was better than Stainzyme® at pH 10.1. *See* FIG. 2.

<u>EXAMPLE 4</u>

<u>Detergent Assays with Terg-o-tometer</u>

***Materials and Methods.***

Swatches Used:

CFT cocoa on cotton, STC CFT CS-2 (TestFabrics)
CFT full egg with pigment on cotton, STC CFT CS-37 (TestFabrics)
CFT colored rice starch on cotton, STC CFT CS-28 (TestFabrics)
2X white bleached cotton per each run
Extra ballast was used to make the regular loading 40 g/L

In 1L Terg-o-tometer

5 mM HEPES pH 8.0, 1.5 g/L
AATCC HDL WOB (2003)
1.8 g/L Tide with bleach alternative (inactivated)
6 gpg water hardness, Temperature at 74°F or 23.3°C

Treatments: 1). AATCC; 2). AATCC + 0.1 ppm 707 α-amylase; 3). AATCC + 0.1 ppm Stainzyme®; 4). Tide; 5). Tide inactivated; 6). Tide Inactivated + 0.1 ppm 707 α-amylase; 7). Tide inactivated + 0.1 ppm Stainzyme®; 8). Tide Inactivated + 0.1 ppm 707 α-amylase + 0.5 ppm Purafect® Prime; Tide inactivated + 0.1 ppm Stainzyme® + 0.5 ppm Purafect® Prime (Genencor). AATCC was present in the amount of 1.5 g/L and the inactivated Tide was present in the amount of 1.8 g/L.

***Results.*** No difference in the cleaning performance was observed between the control and enzyme treatments for the cocoa and egg stains, which suggested saturation of cleaning for both stains, as depicted in FIG. 6. The active Tide produced results comparable to the inactivated Tide at 0.1 ppm using the 707 α-amylase. The 707 α-amylase performed lower than the Stainzyme®. The addition of the Purafect® Prime did not change the cleaning performance of either the Stainzyme® or the 707 α-amylase (FIG. 6).

<u>EXAMPLE 5</u>

<u>α-amylase with Launder-o-meter Testing</u>

***Materials and Methods.***

Swatches Used:

CFT Cocoa on cotton, STC CFT CS-2
CFT full egg with pigment on cotton, STS CFT CS-37
Colored starch on cotton, EMPA 161
CFT colored cornstarch on cotton, STC CFT CS-26
CCFT colored rice starch on cotton, STC CFT CS-28

Additions to Pots:

2X white bleach cotton was added to each pot. Extra ballast was added to each pot to make the regular loading of 42 g/200 mL (bleached cotton interlock). Water had a 12 gpg water hardness. The assay was performed at 40°C for 40 minutes.

Treatments: 8 g/L IEC A* with bleach. These detergent compositions were run either with detergent only, in the presence of detergent plus 0.2 ppm 707 α-amylase (4 mg/mL) or detergent plus 0.2 ppm Stainzyme®. The cleaning was measured using a Minolta reflectometer with a 50 mm aperture both before and after washing.

***Results.*** The assays demonstrated that there was no significant performance difference in cleaning the cocoa and full egg swatches between the control and enzyme treatments (FIG. 7). The IEC A* with bleach control demonstrated that the *Bacillus* sp. no. 707 α-amylase did better than Stainzyme®.

EXAMPLE 6

Dish Detergent

***Materials and Methods.*** The α-amylase of *Bacillus* sp. no. 707 was tested alongside Stainzyme® and OxAm (Purastar®) in automatic dishwashers on rice milk stains. As indicated in FIG. 8, the detergent was WfK C (phosphate containing standard ADD) and bleach (WFK Testgewebe GmBH, Germany); the bleach used was perborate. Stainzyme® was used at a concentration of 25 mg/g equal active enzyme. The *Bacillus* sp. no. 707 was used at a concentration of 4 mg/g. OxAm (Purastar®) had a concentration wherein 3.2 mg = 0.6 % w/w dosage. The porcelain dishes were soiled with mixed starch from IKW and rice milk (Genencor International, Inc.). The Genencor milk-rice stains were baked on the porcelain dishes at 140°C. The soiled dishes were washed in Miele dishwashers at 50°C at a water hardness of 21°GH. The rice milk assay results are shown in FIG. 8. The mixed starch assay results are depicted in FIG. 9. The detergent compositions also had 0.8% properase 4000D added. The alpha-amylases were added in an enzyme dosage of 0.4, 0.8, 1.6, and 3.2 mg total active enzyme in the automatic dish washing conditions.

***Results.*** The a-amylase of *Bacillus* sp. no. 707 met the performance of Stainzyme® in full scale automatic dishwasher (ADW) tests and has at least the Stainzyme® activity within experimental error. The α-amylase of *Bacillus* sp. no. 707 had a higher performance than OxAm (Purastar®) in the automatic dishwashing conditions.

EXAMPLE 7

α-Amylase of *Bacillus* sp. no. 707 and Variants

The codon optimized variant described in Examples 1-2 above can be further recombinantly modified to include any of the variants taught in U.S. Patent Nos. 6,093,562; 6,187,576; 6,197,565; 6,204,232; 6,297,038; 6,309,871; 6,486,113; 6,528,298; 6,623,948; 6,673,589; 6,867,031; and 6,887,986; Published U.S. Application Nos. 20040096952; 20040253676; 20050059131; 20050084937; 20050196853; 2005025664; and 20060035323; European Application Nos. EP 1423513 and EP 1538155; International PCT Application Nos. WO 01/64852; WO 0166712; WO 01/88107; WO 01/96537; WO 02/092797; and WO 02/10355; and Japanese Application Nos. JP2001520006; JP2001521739; JP2002530072; and JP2004505606.

EXAMPLE 8

M202L α-Amylase Variant

The M202L variant was created by substituting a leucine for the methionine at position 202 of the α-amylase sequence using standard methods. Additional variants can include substitutions of different amino acids at M208 and M261 and combinations of one or more substitutions at these 3 sites. Purification of any of the variants can be prepared as described above.

EXAMPLE 9

Cleaning Ability of Amy707 Variants

***Materials and Methods.*** Swatches stained with CS-28 rice starch as discussed above were incubated with or without bleach at 20°C in the presence of the indicated α-amylase enzyme. IEC "A" standard conditions were used for the assay. Variants were prepared by standard methods known to those skilled in the art and were used without purification from the culture supernatant. Concentration of variants was determined by densitometry of SDS PAGE gels. The detergent for this experiment was IEC"A" used at 8 g/L. When present, bleach was supplied by the bleach activation system sodium perporate (13.7 mM) and TAED (1.85 mM).

***Results.*** The M202L variant of *Bacillus* sp. no 707 had higher activity in the presence of bleach as compared to solutions without bleach. The M202L variant had increased activity in the presence of bleach as compared to results obtained with the wild-type *Bacillus* sp. no. 707 α-amylase. *See* FIG. 10. In the absence of bleach, the M202L variant was less active than the composition comprising the wild-type form.

All references cited above are herein incorporated by reference in their entirety for all purposes.

The following numbered paragraphs (paras.) contain statements of broad combinations of the inventive technical features herein disclosed :-

1. A manual or automatic dishwashing composition comprising a *Bacillus* sp. no. 707 α-amylase or variant thereof, and one or more of a surfactant, detergent builder, a complexing agent, a polymer, a bleach, $CaCl_2$, a bleaching system, a stabilizer, a foam booster, a suds suppressor, an anti-corrosion agent, a soil-suspending agent, an anti-soil redeposition agent, a dye, a bactericide, a hydrotope, a tarnish inhibitor, and a perfume.

2. A method of cleaning dishes comprising administering the manual or automatic dishwashing composition of para. 1.

3. A laundry detergent additive comprising a *Bacillus* sp. no. 707 α-amylase or variant thereof.

4. A laundry detergent comprising the laundry additive of para. 3 and further comprising one or more of the following and one or more of a surfactant, detergent builder, a complexing agent, a polymer, a bleaching system, a bleach, $CaCl_2$, a stabilizer, a foam booster, a suds suppressor, an anti-corrosion agent, a soil-suspending agent, an anti-soil redeposition agent, a dye, a bactericide, a hydrotope, an optical brightener, a fabric conditioner, and a perfume.

5. The laundry detergent of para. 4, wherein the $CaCl_2$ is present in the detergent in an amount of about 0.1 mM to about 20 mM.

6. An isolated nucleic acid encoding a *Bacillus* sp. no. 707 α-amylase or a variant thereof, wherein the variant has a residue substitution or deletion selected from the group consisting of substitutions of M202, M208, S255, R172, and/or M261 of SEQ ID NO: 3.

7. The isolated nucleic acid of para. 6 , wherein the M202 variant is a substitution variant selected from the group consisting of: M202L, M202V, M202S, M202T, M202I, M202Q, and M202W; wherein the S255 substitution is S255N; and wherein the R172 substitution is R172Q.

8. An α-amylase comprising SEQ ID NO: 3 or a variant thereof, wherein said variant comprises a residue substitution or deletion at residue M202, M208, and/or M261 of SEQ ID NO: 3.

9. The α-amylase of para. 8, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

10. A vector comprising the nucleic acid of para. 6.

11. An isolated host cell comprising the nucleic acid of para. 6.

12. An isolated host cell comprising the vector of para. 10.

13. The isolated host cell of any of paras. 11 or 12, wherein the cell is a microorganism.

14. The isolated host cell of para. 13, wherein the microorganism is a bacterium or a fungus.

15. The isolated host cell of para. 14, wherein the bacterium is a Gram positive bacterium selected from the group consisting of *Bacillus subtilis*, *B. licheniformis*, *B. lentus*, *B. brevis*, *B. stearothermophilus*, *B. alkalophilus*, *B. amyloliquefaciens*, *B. coagulans*, *B. circulans*, *B. lautus*, *B. thuringiensis*, *Streptomyces lividans*, or *S. murinus*; or a Gram negative bacterium, wherein said Gram negative bacterium is *Escherichia coli* or a *Pseudomonas* species.

16. Use of a polypeptide of para. 8 for laundry washing and/or dishwashing.

17. Use of an α-amylase or variant thereof of para. 8 for laundry washing and/or dishwashing.

18. A detergent additive comprising an α-amylase or variant thereof of para. 8, optionally in the form of a non-dusting granulate, microgranulate, stabilized liquid, or protected enzyme.

19. The detergent additive of para. 18, wherein the detergent additive further comprises an enzyme selected from the group consisting of: a cellulase, a protease, an aminopeptidase, an amylase, a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

20. The detergent additive of para. 19, wherein the amylase is another α-amylase, a β-amylase, an isoamylase, or a glucoamylase.

21. A detergent composition comprising an α-amylase or variant thereof of para. 8.

22. A detergent composition comprising the detergent additive of para. 18.

23. The detergent composition of para. 21, further comprising an enzyme from the group consisting of: a cellulase, a protease, an aminopeptidase, an amylase, a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, an α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

24. A manual or automatic dishwashing detergent composition comprising a polypeptide of an α-amylase or variant thereof of para. 8.

25. The manual or automatic dishwashing detergent composition of para. 24 further comprising an enzyme selected from the group consisting of: a cellulase, a protease, an aminopeptidase, an amylase, a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, an α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

26. The manual or automatic dishwashing detergent composition of any of paras. 24 or 25 further comprising one or more of a surfactant, detergent builder, a complexing agent, a polymer, a bleach, $CaCl_2$, a bleaching system, a stabilizer, a foam booster, a suds suppressor, an anti-corrosion agent, a soil-suspending agent, an anti-soil redeposition agent, a dye, a bactericide, a hydrotope, a tarnish inhibitor, and a perfume.

27. The manual or automatic dishwashing detergent composition of para. 26, wherein the $CaCl_2$ is present in the detergent in an amount of about 0.1 mM to about 20 mM.

28. A detergent additive comprising the α-amylase or variant thereof of para. 8.

29. A manual or automatic laundry washing composition comprising the detergent additive of para. 28.

30. The manual or automatic laundry washing composition of para. 29, further comprising an enzyme selected from the group consisting of: a cellulase, a protease, an aminopeptidase, an amylase, a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, an α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

31. The manual or automatic laundry washing composition of any of paras. 29 or 30, further comprising one or more of the following and one or more of a surfactant, detergent builder, a complexing agent, a polymer, a bleaching system, a bleach, $CaCl_2$, a stabilizer, a foam booster, a suds suppressor, an anti-corrosion agent, a soil-suspending agent, an anti-soil redeposition agent, a dye, a bactericide, a hydrotope, an optical brightener, a fabric conditioner, and a perfume.

32. The manual or automatic laundry washing composition of para. 31, wherein CaCl2 is present in said composition in an amount of about 0.1 mM to about 20 mM.

33. A manual or automatic laundry washing composition comprising the α-amylase or variant thereof of para. 8.

34. The manual or automatic laundry washing composition of para. 33, further comprising an enzyme selected from the group consisting of: a cellulase, a protease, an aminopeptidase, an amylase, a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, an α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

35. The manual or automatic laundry washing composition of any of paras. 33 and 34, further comprising one or more of the following and one or more of a surfactant, detergent builder, a complexing agent, a polymer, a bleaching system, a bleach, $CaCl_2$, a stabilizer, a foam booster, a suds suppressor, an anti-corrosion agent, a soil-suspending agent, an anti-soil redeposition agent, a dye, a bactericide, a hydrotope, an optical brightener, a fabric conditioner, and a perfume.

36. Use of an α-amylase or variant thereof of para. 8 for textile desizing.

37. A textile desizing composition comprising a *Bacillus* sp. no. 707 α-amylase or variant thereof of para. 8 in an aqueous solution, and optionally comprising an enzyme.

38. The textile desizing composition of para. 37, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

39. A method of desizing a textile comprising administering the desizing composition of any of paras. 37 or 38 for a time sufficient to desize said textile.

40. A starch processing composition comprising a *Bacillus* sp. no. 707 α-amylase or variant thereof of para. 8 in an aqueous solution.

41. The starch processing composition of para. 40, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

42. The starch processing composition of para. 40, further comprising a glucoamylase, an isoamylase, a pullulanase, phytase or a combination thereof.

43. A method of processing a starch comprising administering the composition of any of paras. 40-42 for a time sufficient to process said starch.

44. A biofilm hydrolyzing composition comprising a *Bacillus* sp. no. 707 α-amylase or variant thereof of para. 8 in a solution or gel, and optionally further comprising a cellulase, a hemicellulase, a xylanase, a lipase, a protease, a pectinase, an antimicrobial agent, or any combination thereof.

45. The biofilm hydrolyzing composition of para. 44, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

46. A method of hydrolyzing a biofilm comprising administering the composition of any of paras. 44 or 45 for a period sufficient to process said biofilm.

47. A composition for saccharifying starch comprising a *Bacillus* sp. no. 707 α-amylase or variant thereof of para. 8 in a solution.

48. The composition of para. 47, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

49. A method of saccharifying starch comprising administering the composition of any of paras. 47 or 48 for a period sufficient to saccharify said starch.

50. A composition for liquefying starch comprising a *Bacillus* sp. no. 707 α-amylase or variant thereof of para. 8 in a solution.

51. The composition of para. 50, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

52. A method of liquefying a starch comprising administering the composition of any of paras. 50 or 51 for a period sufficient to liquefy said starch.

53. A baking composition comprising a *Bacillus* sp. no. 707 α-amylase or variant thereof of para. 8 in a solution or in a gel.

54. The baking composition of para. 53, wherein the variant is:

(a) a M202 variant selected from the group consisting of M202L, M202V, M202S, M202T, M202I, M202Q, and M202W;
(b) a S255 variant of S255N; and/or
(c) a R172 variant of R172Q.

55. A method of baking comprising administering the baking composition of any of paras. 53 or 54.

## Claims

1. An α-amylase which has at least 90% sequence identity to SEQ ID NO:3 and comprises the substitution M202L, M202V, M202S, M202T, M202I, M202Q or M202W.

2. The α-amylase of claim 1, further comprising the substitution S255 and/or R172Q.

3. An isolated nucleic acid encoding an α-amylase according to claim 1 or claim 2.

4. A vector comprising the nucleic acid of claim 3.

5. An isolated host cell comprising the nucleic acid of claim 3 or the vector of claim 4, wherein optionally the cell is a microorganism, such as a bacterium or a fungus, e.g. a Gram positive bacterium selected from the group consisting of *Bacillus subtilis*, *B. licheniformis*, *B. lentus*, *B. brevis*, *B. stearothermophilus*, *B. alkalophilus*, *B. amyloliquefaciens*, *B. coagulans*, *B. circulans*, *B. lautus*, *B. thuringiensis*, *Streptomyces lividans*, or *S. murinus*; or a Gram negative bacterium, wherein said Gram negative bacterium is *Escherichia coli* or a *Pseudomonas* species.

6. A detergent additive, detergent composition, textile desizing composition, starch processing composition, biofilm hydrolysing composition or baking composition comprising an α-amylase according to claim 1 or claim 2.

7. The detergent additive or detergent composition of claim 6, wherein the detergent additive or detergent composition further comprises an enzyme selected from the group consisting of: a cellulose, a protease, an aminopeptidase, an amylase (e.g. another α-amylase), a carbohydrase, a carboxypeptidase, a catalase, a chitinase, a cutinase, a cyclodextrin glycotransferase, a deoxyribonuclease, an esterase, an α-galactosidase, a β-galactosidase, a glucoamylase, α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

8. A detergent composition according to claim 6 or claim 7 which is a manual or automatic dishwashing detergent composition, optionally further comprising one or more of a surfactant, detergent builder, a complexing agent, a polymer, a bleach, $CaCl_2$, a bleaching system, a stabilizer, a foam booster, a suds suppressor, an anti-corrosion agent, a soil-suspending agent, an anti-soil redeposition agent, a dye, bactericide, a hydrotope, a tarnish inhibitor, and a perfume, e.g. wherein the $CaCl_2$ is present in the detergent in an amount of about 0.1 mM to about 20 mM.

9. A detergent composition according to claim 6 or claim 7 which is a laundry detergent composition, optionally further comprising one or more of a surfactant, detergent builder, a complexing agent, a polymer, a bleach, $CaCl_2$, a bleaching system, a stabilizer, a foam booster, a suds suppressor, an anti-corrosion agent, a soil-suspending agent, an anti-soil redeposition agent, a dye, bactericide, a hydrotope, an optical brightener, a fabric conditioner, and a perfume, e.g. wherein the $CaCl_2$ is present in the detergent in an amount of about 0.1 mM to about 20 mM.

10. A method of desizing a textile comprising administering the α-amylase of claim 1 or the desizing composition of claim 6 for a time sufficient to desize said textile.

11. The starch processing composition of claim 6 which is a starch saccharifying composition or a starch liquefying composition, and optionally further comprises a glucoamylase, an isoamylase, a pullulanase, phytase or a combination thereof.

12. A method of processing, saccharifying or liquefying a starch comprising administering the composition of claim 6 or claim 11 for a time sufficient to process, saccharify or liquefy said starch.

13. A biofilm hydrolyzing composition according to claim 6 in a solution or gel, and optionally further comprising a cellulase, a hemicelluase, a xylanase, a lipase, a protease, a pectinase, an antimicrobial agent, or any combination thereof.

**14.** A method of hydrolyzing a biofilm comprising administering the biofilm hydrolyzing composition of claim 6 or claim 12 for a period sufficient to process said biofilm.

**15.** A method of introducing mutations into an $\alpha$-amylase-encoding DNA sequence by site-directed mutagenesis.

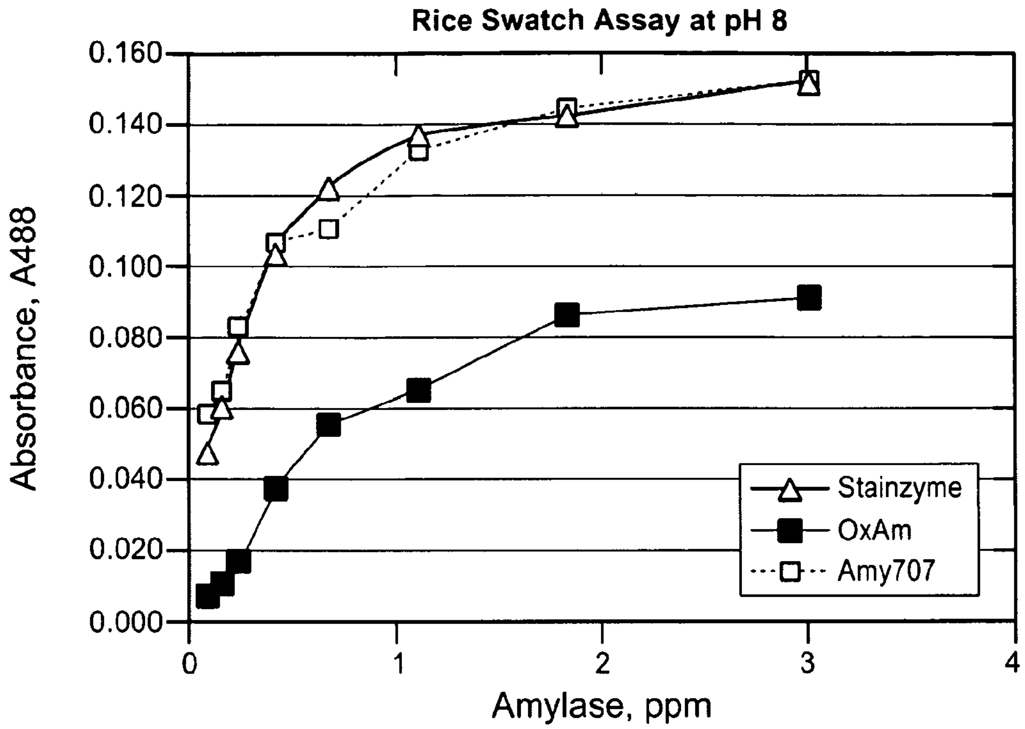
Rice Swatch Assay at pH 8
Absorbance, A488
0.160
0.140
0.120
0.100
0.080
0.060
0.040
0.020
0.000
0
1
2
3
4
Amylase, ppm
Stainzyme
OxAm
Amy707

FIG. 1

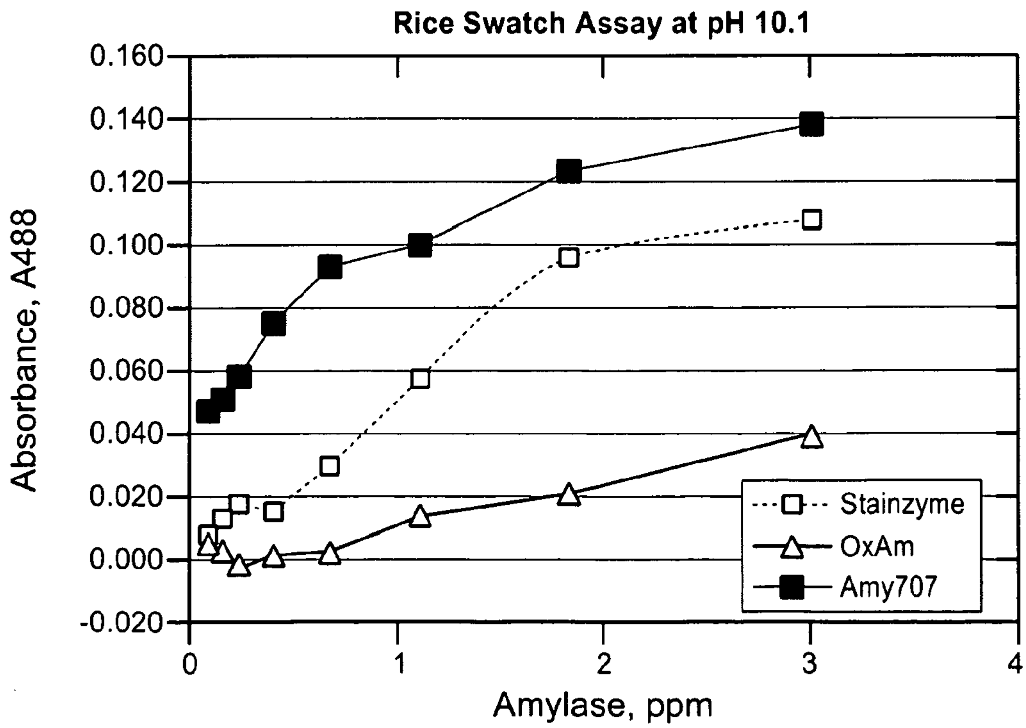
Rice Swatch Assay at pH 10.1
Absorbance, A488
0.160
0.140
0.120
0.100
0.080
0.060
0.040
0.020
0.000
-0.020
0
1
2
3
4
Amylase, ppm
Stainzyme
OxAm
Amy707

FIG. 2

**Pre-LAT Signal Peptide-encoding DNA Sequence** (SEQ ID NO: 1):

```
5'-atgaaacaac aaaaacggct ttacgcccga ttgctgacgc tgttatttgc     50
   gctcatcttc ttgctgcctc attctgcagc ttcagca-3'                87
```

FIG. 3A

**Native *Bacillus* Sp. No. 707 α-amylase Gene** (SEQ ID NO: 2):

```
5'-catcataacg gtacgaacgg gacaatgatg caatactttg aatggtatct     50
   acctaatgac ggaaatcatt ggaatcgatt aaactctgat gcgagtaacc    100
   ttaaaagcaa agggattaca gcggtgtgga ttcctccagc atggaagggc    150
   gcttctcaaa atgacgtagg atacggagcc tatgacctgt atgatctggg    200
   agaatttaat caaaaaggta ccgtccgtac aaaatatgga acacgtagtc    250
   agttacaagc tgcggtaacc tccttaaaaa ataatggaat tcaagtatat    300
   ggtgacgttg ttatgaatca caaaggtggc gcagacgcta ctgaaatggt    350
   aagggccgtt gaagtgaatc ccaataaccg taaccaagaa gtgactggtg    400
   aatataccat tgaagcttgg actagatttg attttccagg gcgaggaaat    450
   actcattcta gctttaaatg gagatggtat cattttgatg gtgtggattg    500
   ggatcagtca cgtagactga acaatcgcat ctataaattt agaggtcatg    550
   gcaaagcttg ggattgggaa gttgatacgg aaaatggtaa ttatgattat    600
   ttaatgtacg ctgatattga tatggatcac ccagaagtag taaatgaatt    650
   aagaaattgg ggtgtttggt acacaaacac attaggactc gatggattta    700
   gaatagatgc ggttaaacat ataaagtata gctttacgcg cgattggatt    750
   aatcacgtta gaagtgcaac aggtaaaaat atgtttgcgg ttgctgagtt    800
   ttggaagaat gatttaggtg caattgaaaa ctatctgcag aaaacaaact    850
   ggaaccattc agtctttgat gtgccgttac attataatct ttataatgca    900
   tcaaaaagcg gagggaacta tgatatgcga aacatattta atggaacggt    950
   tgttcaacga catccaagtc atgctgtaac atttgttgat aatcatgatt   1000
   cgcagcctga agaagcatta gaatcttttg ttgaagaatg gtttaaacca   1050
   ttagcgtatg cgcttacatt aacgcgtgaa caaggatacc cttctgtatt   1100
   ttacggagat tattatggga ttccaacaca tggagtgcca gcaatgagat   1150
   caaaaatcga tccgatttta gaagcacgtc aaaagtatgc atacggaaaa   1200
   caaaatgatt acttagacca tcataatatc attggttgga cgcgtgaagg   1250
   gaatacagca caccccaatt caggtctagc taccatcatg tctgatggag   1300
   cgggtggaag taagtggatg tttgttgggc gtaataaggc tggtcaagta   1350
   tggagtgata ttacaggaaa ccgtacaggt acggttacaa tcaatgcaga   1400
   cggttggggc aatttctctg tgaatggagg gtcagtttct atttgggtca   1450
   acaaa-3'                                                 1455
```

FIG. 3B

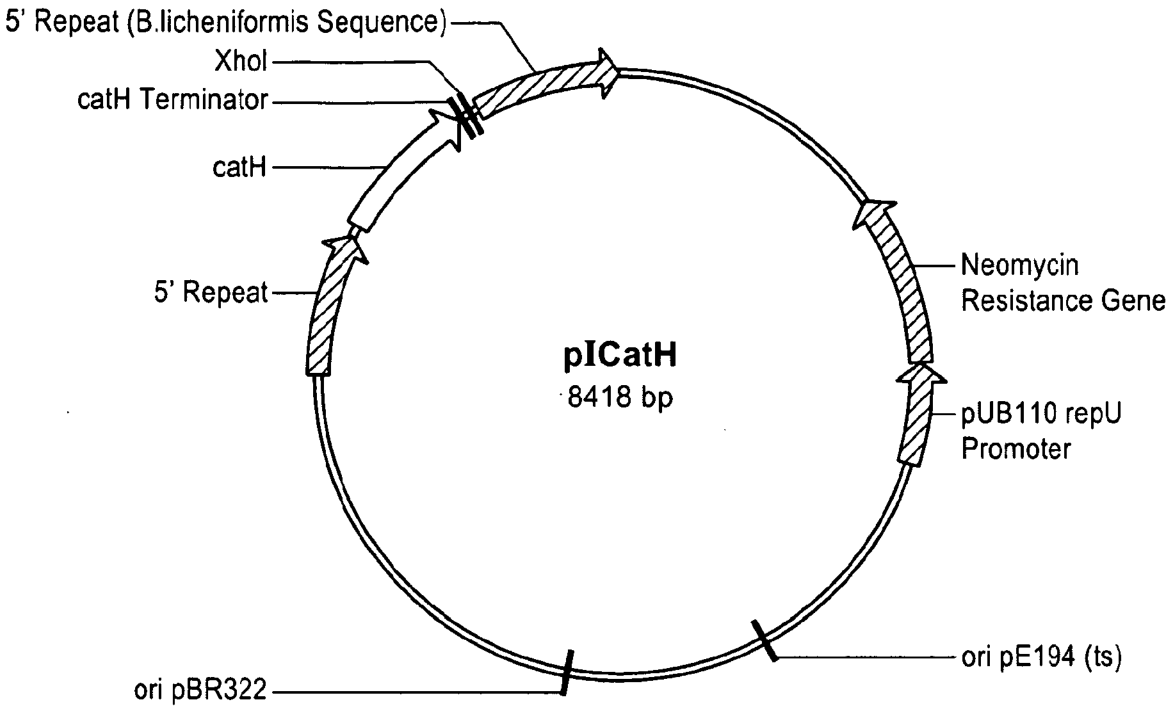
5' Repeat (B.licheniformis Sequence)
XhoI
catH Terminator
catH
5' Repeat
pICatH
8418 bp
Neomycin
Resistance Gene
pUB110 repU
Promoter
ori pE194 (ts)
ori pBR322

*FIG. 4*

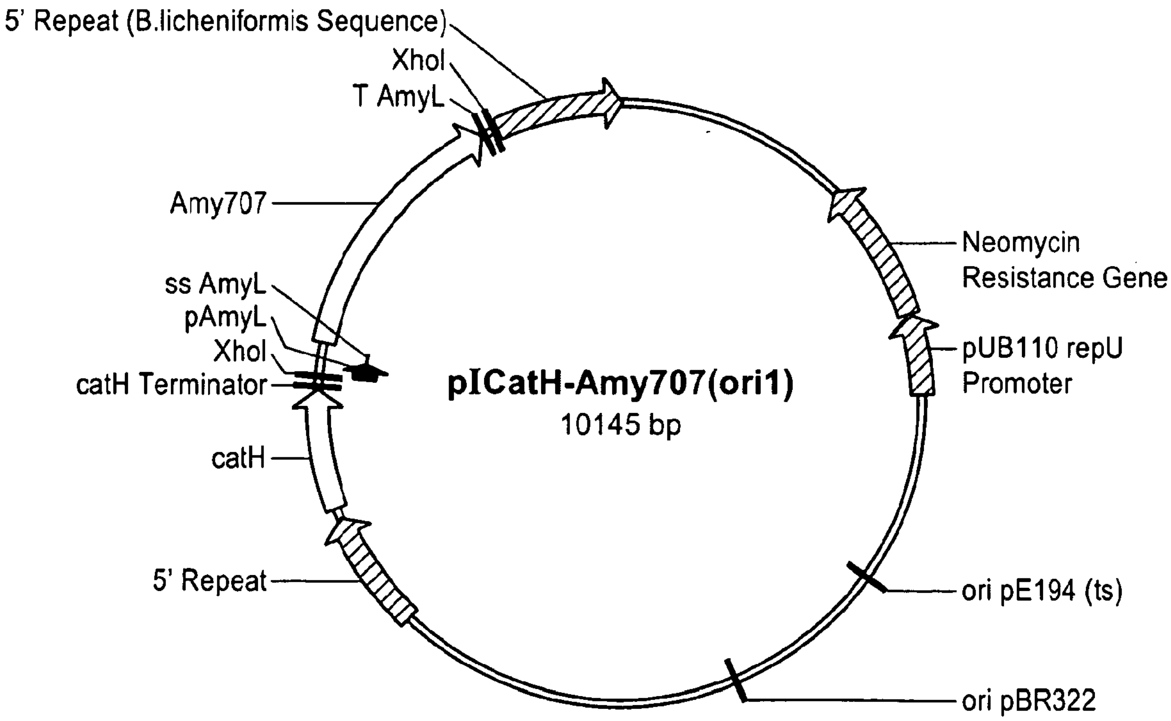
5' Repeat (B.licheniformis Sequence)
XhoI
T AmyL
Amy707
ss AmyL
pAmyL
XhoI
catH Terminator
catH
5' Repeat
pICatH-Amy707(ori1)
10145 bp
Neomycin
Resistance Gene
pUB110 repU
Promoter
ori pE194 (ts)
ori pBR322

*FIG. 5*

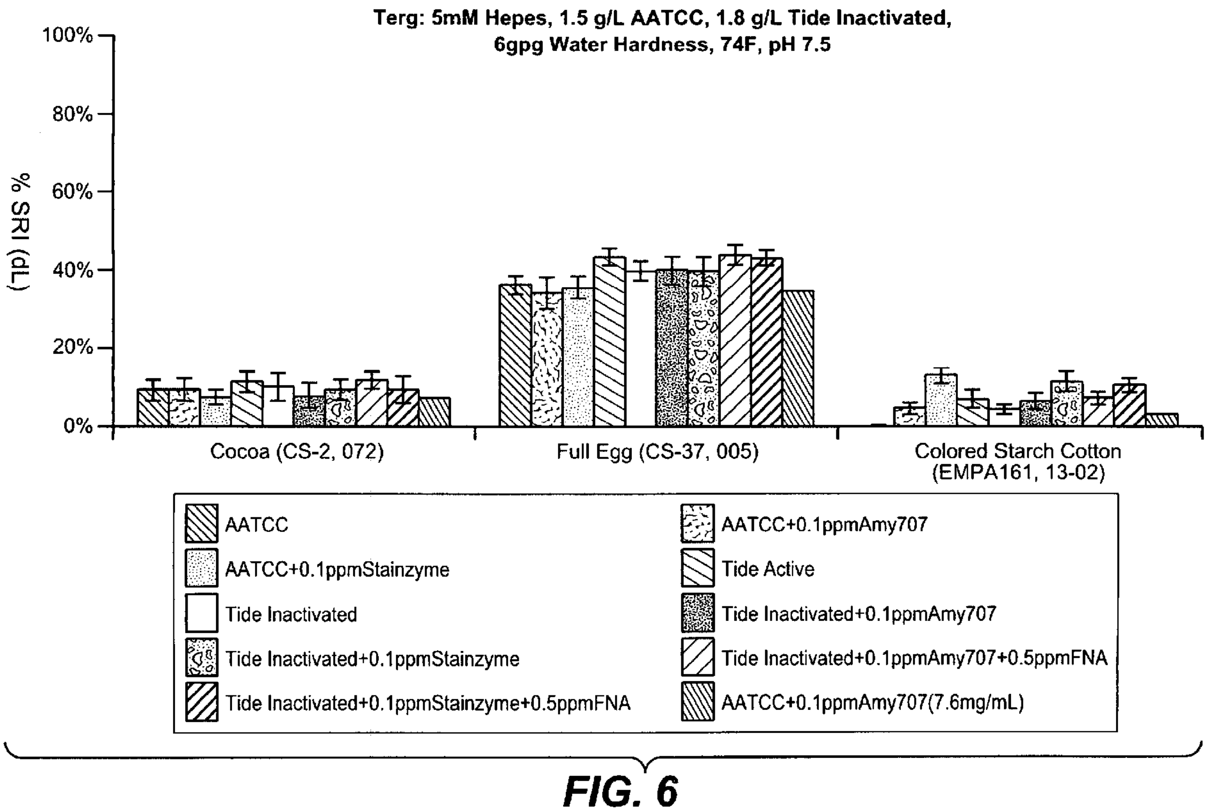
Terg: 5mM Hepes, 1.5 g/L AATCC, 1.8 g/L Tide Inactivated,
6gpg Water Hardness, 74F, pH 7.5
100%
80%
60%
40%
20%
0%
% SRI (dL)
Cocoa (CS-2, 072)
Full Egg (CS-37, 005)
Colored Starch Cotton (EMPA161, 13-02)
AATCC
AATCC+0.1ppmAmy707
AATCC+0.1ppmStainzyme
Tide Active
Tide Inactivated
Tide Inactivated+0.1ppmAmy707
Tide Inactivated+0.1ppmStainzyme
Tide Inactivated+0.1ppmAmy707+0.5ppmFNA
Tide Inactivated+0.1ppmStainzyme+0.5ppmFNA
AATCC+0.1ppmAmy707(7.6mg/mL)

FIG. 6

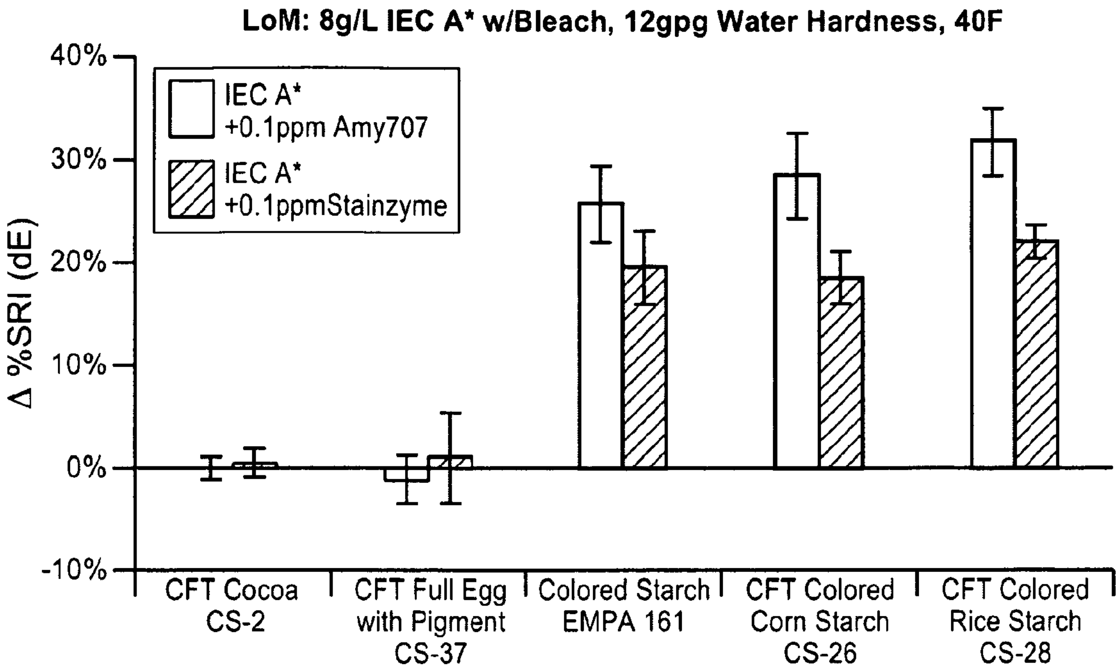
LoM: 8g/L IEC A* w/Bleach, 12gpg Water Hardness, 40F
40%
30%
20%
10%
0%
-10%
Δ %SRI (dE)
IEC A*
+0.1ppm Amy707
IEC A*
+0.1ppmStainzyme
CFT Cocoa
CS-2
CFT Full Egg
with Pigment
CS-37
Colored Starch
EMPA 161
CFT Colored
Corn Starch
CS-26
CFT Colored
Rice Starch
CS-28

*FIG. 7*

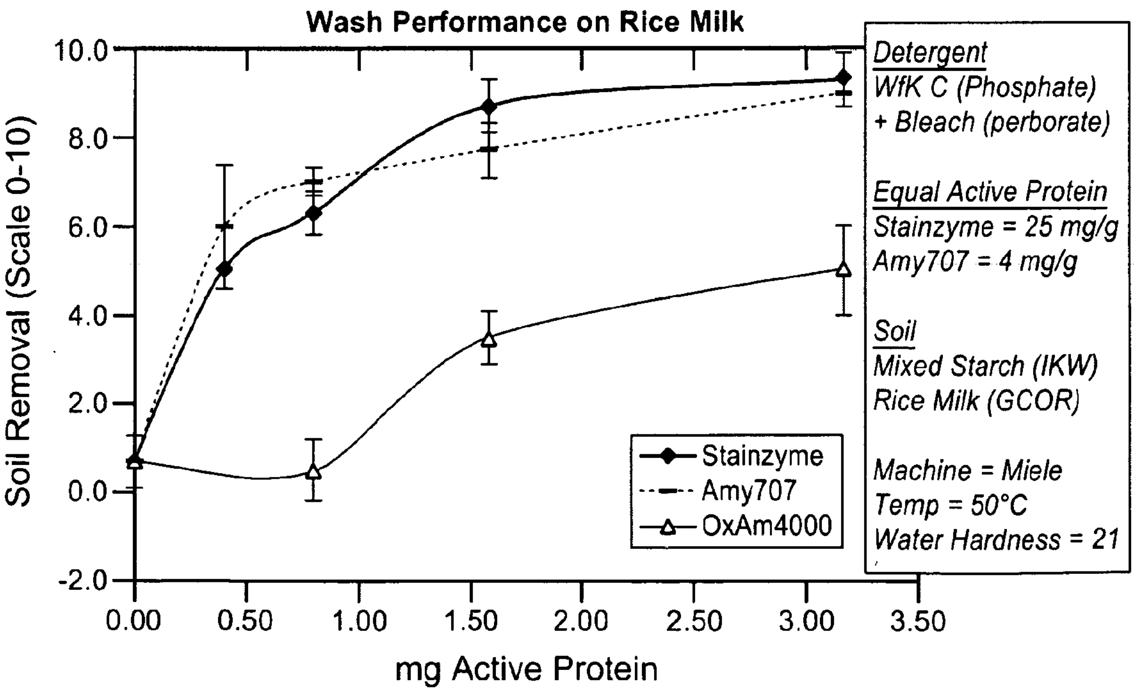
Wash Performance on Rice Milk
10.0
8.0
6.0
4.0
2.0
0.0
-2.0
Soil Removal (Scale 0-10)
0.00
0.50
1.00
1.50
2.00
2.50
3.00
3.50
mg Active Protein
Stainzyme
Amy707
OxAm4000
Detergent
WfK C (Phosphate)
+ Bleach (perborate)
Equal Active Protein
Stainzyme = 25 mg/g
Amy707 = 4 mg/g
Soil
Mixed Starch (IKW)
Rice Milk (GCOR)
Machine = Miele
Temp = 50°C
Water Hardness = 21

*FIG. 8*

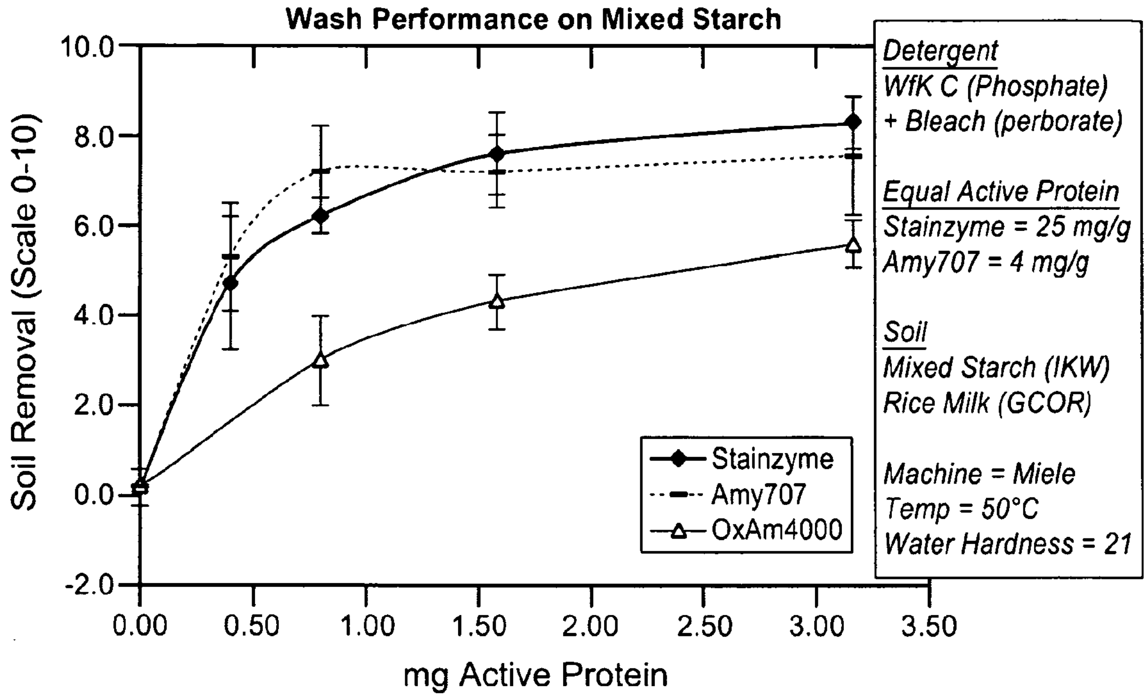
Wash Performance on Mixed Starch
Soil Removal (Scale 0-10)
10.0
8.0
6.0
4.0
2.0
0.0
-2.0
0.00
0.50
1.00
1.50
2.00
2.50
3.00
3.50
mg Active Protein
Stainzyme
Amy707
OxAm4000
Detergent
WfK C (Phosphate)
+ Bleach (perborate)
Equal Active Protein
Stainzyme = 25 mg/g
Amy707 = 4 mg/g
Soil
Mixed Starch (IKW)
Rice Milk (GCOR)
Machine = Miele
Temp = 50°C
Water Hardness = 21

*FIG. 9*

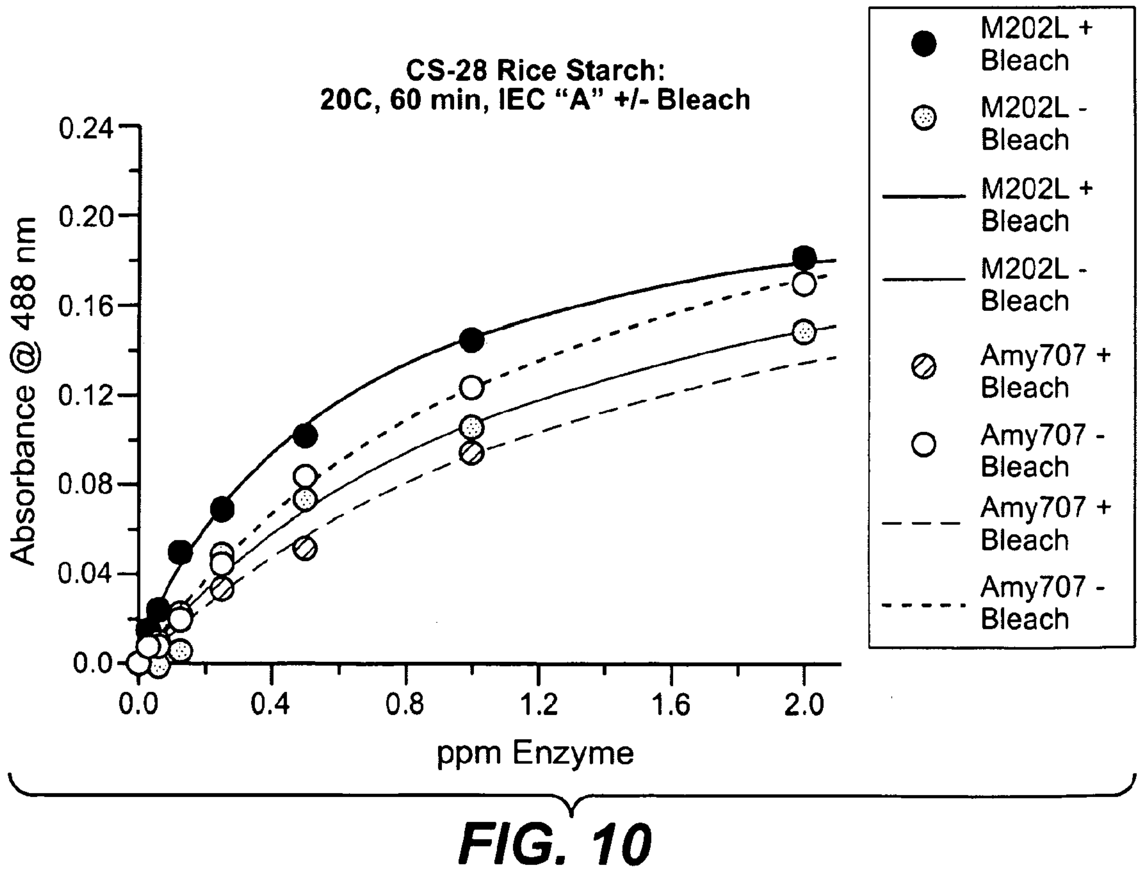
CS-28 Rice Starch:
20C, 60 min, IEC "A" +/- Bleach
Absorbance @ 488 nm
0.24
0.20
0.16
0.12
0.08
0.04
0.0
0.0
0.4
0.8
1.2
1.6
2.0
ppm Enzyme
M202L + Bleach
M202L - Bleach
M202L + Bleach
M202L - Bleach
Amy707 + Bleach
Amy707 - Bleach
Amy707 + Bleach
Amy707 - Bleach

*FIG. 10*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 90581107 P
- US 4683202 A
- US 4760025 A, Morinaga
- WO 9117243 A
- EP 238023 A
- US 6297037 B
- US 785624 A
- US 5281526 A
- CA 2006687
- GB 2200132 A
- GB 2234980 A
- GB 2228945 A
- DE 3741617
- DE 3727911
- DE 4212166
- DE 4137470
- DE 3833047
- DE 4205071
- WO 9325651 A
- WO 9318129 A
- WO 9304153 A
- WO 9206157 A
- WO 9208777 A
- WO 9321299 A
- WO 9317089 A
- WO 9303129 A
- EP 481547 A
- EP 530870 A
- EP 533239 A
- EP 554943 A
- EP 429124 A
- EP 346137 A
- EP 561452 A
- EP 318204 A
- EP 318279 A
- EP 271155 A
- EP 271156 A
- EP 346136 A
- EP 518719 A
- EP 518720 A
- EP 518721 A
- EP 516553 A
- EP 561446 A
- EP 516554 A
- EP 516555 A
- EP 530635 A
- EP 414197 A
- US 5112518 A
- US 5141664 A
- US 5240632 A
- US 4106991 A
- US 4661452 A
- GB 1483591 A
- EP 238216 A
- WO 9206154 A
- WO 2005056783 A
- WO 9219709 A
- WO 9219708 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- WO 9535381 A
- WO 9600292 A
- WO 9530744 A
- WO 9425578 A
- WO 9514783 A
- WO 9522615 A
- WO 9704079 A
- WO 9707202 A
- EP 407225 A
- EP 260105 A
- WO 0134899 A
- WO 0114629 A
- US 4435307 A
- US 5648263 A
- US 5691178 A
- US 5776757 A
- WO 8909259 A
- EP 0495257 A
- EP 0531372 A
- WO 9611262 A

- WO 9629397 A
- WO 9808940 A
- WO 9407998 A
- WO 9812307 A
- WO 9524471 A
- DK 9800299 W
- EP 531315 A
- US 5457046 A
- US 5686593 A
- US 5763254 A
- WO 9324618 A
- WO 9510602 A
- WO 9815257 A
- US 7122334 B
- WO 9742825 A
- DK 971273
- WO 9502044 A
- WO 9706775 A
- WO 9200381 A
- WO 0004136 A
- WO 8402921 A
- WO 9928448 A
- US RE32153 E
- US 4587215 A
- EP 135138 A
- WO 8601831 A
- US 6077316 A
- WO 9500636 A
- WO 9119782 A
- WO 9118977 A
- WO 9201793 A
- WO 9217573 A
- WO 8802775 A
- EP 214761 A
- WO 8901032 A
- US 6093562 A
- US 6187576 A
- US 6197565 A
- US 6204232 A
- US 6297038 A
- US 6309871 A
- US 6486113 A
- US 6528298 A
- US 6623948 A
- US 6673589 A
- US 6867031 A
- US 6887986 A
- US 20040096952 A
- US 20040253676 A
- US 20050059131 A
- US 20050084937 A
- US 20050196853 A
- US 2005025664 A
- US 20060035323 A
- EP 1423513 A
- EP 1538155 A
- WO 0164852 A
- WO 0166712 A
- WO 0188107 A
- WO 0196537 A
- WO 02092797 A
- WO 0210355 A
- JP 2001520006 B
- JP 2001521739 B
- JP 2002530072 B
- JP 2004505606 B

**Non-patent literature cited in the description**


- **A. TSUKAMOTO et al.** Nucleotide sequence of the maltohexaose-producing amylase gene from an alkalophilic Bacillus sp. #707 and structural similarity to liquefying type α-amylase. *Biochem. & Biophys. Res. Comm.,* 1988, vol. 151 (1), 25-31
- **K. KIMURA et al.** Cloning of a gene for maltohexaose producing amylase of an alkalophilic Bacillus and hyper-production of the enzyme in Bacillus subtilis cells. *Appl. Microbiol. Biotechnol.,* 1988, vol. 27, 372-377
- **R. KANAI et al.** Biochemical and crystallographic analyses of maltohexaose-producing amylase from alkalophilic Bacillus sp. 707. *Biochemistry,* 2004, vol. 43, 14047-14056
- CURRENT PROTOCOLS IN MOLECULAR BIOLOGY. 1987, vol. 30
- **PEARSON et al.** *Proc. Natl, Acad. Sci USA,* 1988, vol. 85, 2444-2448
- **ALTSCHUL et al.** BLAST Manual. Natl Cent. Biotechnol. Inf., Natl Lib. Med,
- **ALTSCHUL et al.** *NAR,* 1997, vol. 25, 3389-3402
- **NEEDLEMAN ; WUNSCH.** *J.Mol. Biol.,* 1970, vol. 48, 443-453
- **GABORIAUD et al.** *FEBS LETTERS,* 1987, vol. 224, 149-155
- **HUBER, T ; TORDA, AE.** *PROTEIN SCIENCE,* 1998, vol. 7 (1), 142-149
- **SAMBROOK et al.** Molecular Cloning: A Laboratory Manual. Cold Spring Harbor, 1989
- **SAMBROOK et al.** MOLECULAR CLONING: A LABORATORY MANUAL. Cold Spring Harbor, 1989
- **M. BRADFORD.** *Anal. Biochem.,* 1976, vol. 72, 248
- **J. K. KAUSHIK et al.** Why is trehalose an exceptional protein stabilizer? An analysis of the thermal stability of proteins in the presence of the compatible osmolyte trehalose. *J. Biol. Chem.,* 2003, vol. 278, 26458-65

- **MONICA CONTI et al.** Capillary isoelectric focusing: the problem of protein solubility. *J. Chromatography,* 1997, vol. 757, 237-245
- Efficient manganese catalysts for low-temperature bleaching. *Nature,* 1994, vol. 369, 637-639
- **DARTOIS et al.** *Biochemica et Biophysica Acta,* 1993, vol. 1131, 253-360
- **MORRIS ; PRATO.** *Textile Research Journal,* 1982, vol. 52 (4), 280-286
- **CAYOT ; TAINTURIER.** *Anal. Biochem.,* 1997, vol. 249, 184-200
- **W. M. FOGARTY ; C. T. KELLY.** *PROGRESS IN INDUSTRIAL MICROBIOLOGY,* 1979, vol. 15, 112-115
- **BOEL et al.** *EMBO J.,* 1984, vol. 3 (5), 1097-1102
- *Agric. Biol. Chem.,* 1991, vol. 55 (4), 941-949
- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **C. CHRISTOPHERSEN et al.** *Starch,* 1997, vol. 50 (1), 39-45
- **A. TSUKAMOTO et al.** *Biochem. Biophys. Res. Commun.,* 1988, vol. 151 (1), 25-31